(19) Europäisches Patentamt
European Patent Office
Office européen des brevets

(11) EP 0 749 429 B1

(12) # EUROPÄISCHE PATENTSCHRIFT

(45) Veröffentlichungstag und Bekanntmachung des Hinweises auf die Patenterteilung:
**30.06.1999 Patentblatt 1999/26**

(21) Anmeldenummer: **95912197.1**

(22) Anmeldetag: **03.03.1995**

(51) Int Cl.[6]: **C07D 261/04**, A61K 31/42, C07D 261/08, C07F 9/653, C07D 413/04, C07D 413/12, C07D 261/20

(86) Internationale Anmeldenummer:
**PCT/EP95/00784**

(87) Internationale Veröffentlichungsnummer:
**WO 95/24397 (14.09.1995 Gazette 1995/39)**

(54) **3,5-DISUBSTITUIERTE UND 3,4,5-TRISUBSTITUIERTE 2-ISOXAZOLINE UND ISOXAZOLE, VERFAHREN ZU IHRER HERSTELLUNG UND IHRE VERWENDUNG ALS ARZNEIMITTEL**

3,5-DISUBSTITUTED AND 3,5,6-TRISUBSTITUTED 2-ISOXAZOLINES AND ISOXAZOLES, PROCESS FOR PREPARING THE SAME AND THEIR USE AS MEDICAMENTS

ISOXAZOLINES-2 ET ISOXAZOLES 3,5-DISUBSTITUES ET 3,4,5-TRISUBSTITUES, LEUR PROCEDE DE PREPARATION ET LEUR UTILISATION COMME MEDICAMENTS

(84) Benannte Vertragsstaaten:
**AT BE CH DE DK ES FR GB GR IE IT LI LU NL PT SE**

(30) Priorität: **10.03.1994 DE 4408084**

(43) Veröffentlichungstag der Anmeldung:
**27.12.1996 Patentblatt 1996/52**

(73) Patentinhaber: **HOECHST AKTIENGESELLSCHAFT**
**65929 Frankfurt am Main (DE)**

(72) Erfinder:
- **SCHWAB, Wilfried**
  **D-65207 Wiesbaden (DE)**
- **ANAGNOSTOPULOS, Hiristo**
  **D-65193 Wiesbaden (DE)**
- **BARTLETT, Robert, Ryder**
  **D-64291 Darmstadt (DE)**
- **SCHLEYERBACH, Rudolf**
  **D-65719 Hofheim (DE)**
- **WEITHMANN, Klaus, Ulrich**
  **D-65719 Hofheim (DE)**

(56) Entgegenhaltungen:
**EP-A- 0 245 825** **EP-A- 0 449 223**

- **CHEMICAL AND PHARMACEUTICAL BULLETIN, Bd. 32,Nr. 1, Januar 1984 TOKYO JP, Seiten 152-165, YASUO ISOMURA ET AL 'Synthesis and anti-inflammatory activity of 2,6-di-tert-butylphenols with a heterocyclic group at the 4-position'**
- **CHEMICAL ABSTRACTS, vol. 100, no. 5, 30.Januar 1984 Columbus, Ohio, US; abstract no. 34538, Seite 453; Spalte 1; & JP,A,58 148 858 (YAMANOUCHI PHARMACEUTICAL CO., LTD) 5.September 1983**
- **JOURNAL OF MEDICINAL CHEMISTRY, Bd. 34,Nr. 2, Februar 1991 WASHINGTON US, Seiten 518-525, DANIEL L. FLYNN ET AL 'Styrylpyrazoles,styrylisoxazoles,and styrylisothiazoles.Novel 5-lipoxygenase and cyclooxygenase inhibitors'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 749 429 B1

## Beschreibung

Die vorliegende Erfindung betrifft Arzneimittel zur Prophylaxe und/oder Behandlung von Entzündungen, Asthma, rheumatischen Erkrankungen und Autoimmunerkrankungen.

Die Wirkungsweise von nichtsteroidalen antiinflammatorischen Medikamenten, z.B. Acetylsalicylsäure und Indometacin, ist bereits bekannt. Sie beruht auf der Hemmung des Enzyms Cyclooxygenase, eines in vielen Zellen vorhandenen Enzyms, das für die Bereitstellung von proinflammatorischen Prostaglandinen verantwortlich ist.

Daniel L. Flynn et al. (J. Med. Chem. 1991, 34, 518-525) beschreiben Styrylpyrazol-, Styrylisoxazol- und Styrylisothiazolderivate, die sowohl die 5-Lipoxygenase als auch die Cyclooxygenase in basophilen Leukemiezellen der Ratte hemmen. Die Europäische Patentanmeldung, Publikations-Nr. 0 449 223 A1, beschreibt 3,5-Di-tertiär-butyl-4-hydroxyphenyl-thiozolyl,- Oxazolyl oder -Imidazolyl Methane und Methanon-Oxime, die die 3-Lipoxygenase und Cyclooxygenase hemmen.

Es ist auch bereits bekannt, daß derartige Cyclooxygenase-Inhibitoren in der Rheumatherapie eingesetzt werden können (EP 245 825).

Inzwischen hat sich allerdings herausgestellt, daß Prostaglandine neben ihren unerwünschten Effekten auch erwünschte zellschützende Eigenschaften auszuüben vermögen, z. B. im Thrombozyten, im Gastroduodenum und in der Niere. Alle Cyclooxygenase-Inhibitoren verursachen deshalb unerwünschte Nebenwirkungen, wie Verlängerung der Blutungszeit, Generierung von Magenulcera und Hemmung der Nierenfunktion.

Es kann somit vorteilhaft sein, statt der Prostaglandine die besonders potenten Entzündungsmediatoren aus der Stoffklasse der Leukotrienen und der Zytokine wie Interleukin 1 alpha und Interleukin 1 beta, Sauerstoffradikale und Proteasen durch Blockade ihrer Bildung zu hemmen.

Es wurde nun gefunden, daß die Verbindung der Formel I im wesentlichen keine hemmende Wirkung auf die Cyclooxygenase zeigt, aber die Bildung von Leukotrien, Zytokinen, Sauerstoffradikalen und Proteasen aus weißen Blutzellen hemmt, sowie Wirkung in Modellen zur Testung von Asthma, Autoimmunerkrankungen und rheumatischen Erkrankungen zeigt.

Die Erfindung betrifft eine Verbindung der Formel I

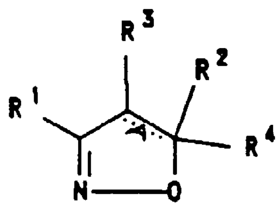

(I)

und/oder ein physiologisch verträgliches Salz der Verbindung der Formel I und/oder eine stereoisomere Form der Verbindung der Formel I; dabei hat ein Rest $R^1$ oder $R^2$ die Bedeutung der Formel II

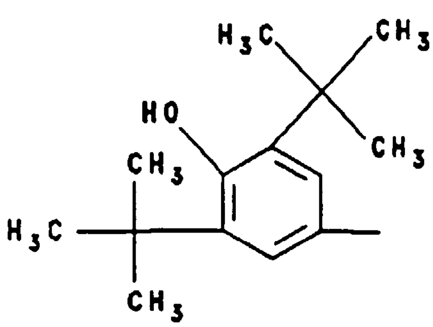

(II)

und der andere Rest $R^1$ oder $R^2$ hat die nachstehende Bedeutung:

a) Pyridyl,
b) Thiophenyl,
c) Thiazolyl,
d) ein Rest der Formel III

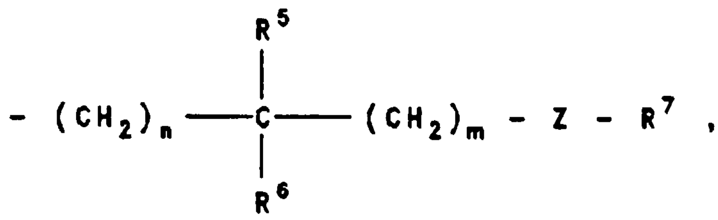

(III)

wobei n für die Zahl Null, 1, 2, 3, 4, 5 oder 6,
m für die Zahl Null, 1, 2, 3 oder 4 steht,

Z ist

1) -O- oder
2) -NH-,

$R^5$ und $R^6$ sind unabhängig voneinander

1) Wasserstoffatom,
2) ($C_1$-$C_4$)-Alkyl,
3) ($C_2$-$C_4$)-Alkenyl,
4) ($C_2$-$C_4$)-Alkinyl,
5) Phenyl oder
6) OH, und

$R^7$ ist

1) Wasserstoffatom,
2) Rest einer Aminosäure, aus der Gruppe Asparagin, Valin, Arginin, Asparaginsäure, Glutamin, Glutaminsäure, Tryptophan, β-Alanin, Lysin, Prolin, Glycin, y-Aminobutyrat, Nε -Acetyllysin, Nδ-Acetylornithin, Ny-Acetyldiaminobutyrat, Nα-Acetyldiaminobutyrat, Histidin, Isoleucin, Leucin, Methionin, Phenylalanin, Serin, Cystein, Threonin, Alanin und Tyrosin,
3) Trifluormethylsulfonyl,
4) -O-P(O)$(OH)_2$,
5) -O-P(O)$(OH)_2$, ein- oder zweifach verestert mit Phenyl oder ($C_1$-$C_6$)-Alkyl, oder
6) ein Rest der Formel IV

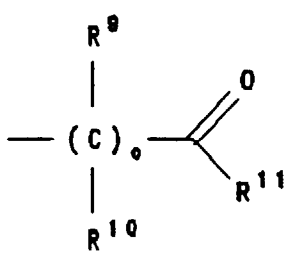

,

(IV)

dabei ist o die Zahl Null oder 1,
dabei haben $R^9$ und $R^{10}$ unabhängig voneinander die nachstehende Bedeutung:

1) Wasserstoffatom oder
2) ($C_1$-$C_4$)-Alkyl, und

$R^{11}$ ist

1) OH,
2) -O-($C_1$-$C_4$)-Alkyl,

3) ($C_1$-$C_{20}$)-Alkyl,
4) ($C_1$-$C_{20}$)-Alkyl, ein oder mehrfach unabhängig voneinander substituiert durch

4.1 -COOH,
4.2 -OH,
4.3 O-Acetyl oder
4.4 =O,

5) -COOH,
6)

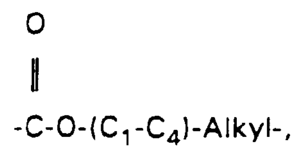

7) N-Glycyl,
8) N-Glycyl-($C_1$-$C_4$)-Alkylester,
9) N-($C_1$-$C_4$)-Alkyl-hydroxylamino,
10) N-(1H-tetrazol-5-yl)-amino,
11) 5-Methyl-isoxazol-4-yl
12) 1-Cyano-2-hydroxy-1-propenyl,
13) Phenyl,
14) Phenyl, ein- oder mehrfach substituiert durch

14.1

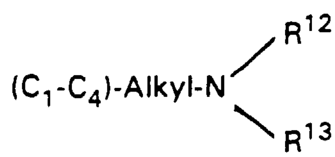

dabei haben $R^{12}$ und $R^{13}$ unabhängig voneinander die nachstehende Bedeutung.

1) Wasserstoffatom,
2) ($C_1$-$C_4$)-Alkyl,
3) Phenyl-($C_1$-$C_2$)-Alkyl,
4) $R^{12}$ und $R^{13}$ bilden zusammen mit dem sie verknüpfenden Stickstoffatom einen fünf- bis siebengliedrigen Heterozyklus, der unsubstituiert oder ein- bis dreifach durch ($C_1$-$C_4$)-Alkyl substituiert ist, wobei ein Kohlenstoffatom durch ein Schwefel-, Sauerstoff- oder Stickstoffatom ersetzt sein kann, oder
5) Rest einer Aminosäure, oder

$R^6$ und $R^7$ sind miteinander verbunden und sind zusammen 1 bis 3 $CH_2$-Reste,

e) ein Rest der Formel V

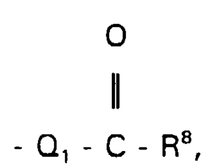 (V)

dabei ist $Q_1$

1) $-(CH_2)_m-$, wobei m die Zahl Null, 1, 2, 3 oder 4 ist, oder
2) -CH=CH-, und

$R^8$ ist

1) Wasserstoffatom,
2) $(C_1-C_4)$-Alkyl,
3) OH,
4) $-O-(C_1-C_4)$-Alkyl,
5) $NH_2$,
6) N-(1 H-Tetrazol-5-yl)-amino oder
7) N-(3,5-Dimethyl-4-hydroxybenzyl)-amino, oder

f) ein Rest der Formel VI

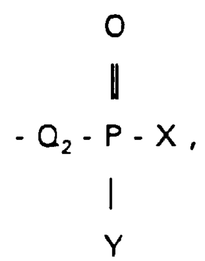

(VI)

dabei ist $Q_2$

1) $-(CH_2)_m$, wobei m die Zahl Null, 1, 2, 3 oder 4 ist, oder
2) -CH=CH-, und

X und Y sind unabhängig voneinander

1) $(C_1-C_4)$-Alkyl,
2) $-O-(C_1-C_4)$-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) $(C_1-C_6)$-Alkyl, oder

$R^2$ und $R^4$ bilden zusammen mit dem Kohlenstoffatom, an das sie gebunden sind, einen aliphatischen Ring der aus 3, 4 oder 5 Kohlenstoffatomen besteht und
$R^3$ ist

1) Wasserstoffatom,
2) $(C_1-C_4)$-Alkyl oder
3) Hydroxy-$(C_1-C_4)$-alkyl.

Die Verbindungen der Formel I können in den Arzneimitteln als optische Isomere, Diastereomere, Racemate oder als Gemische derselben vorliegen. Die bezeichneten Alkyl- oder Alkanoylreste können sowohl geradkettig als auch verzweigt vorliegen.

Fünf- bis siebengliedrige Heterozyklen im Sinne der vorliegenden Erfindung sind beispielsweise Pyrrol, Pyridin, Azepin, Thiazol, Isothiazol, Oxazol, Isoxazol, Pyrazol, Imidazol, Thiazin, 1,2-Oxazin, 1,3-Oxazin, Morpholin, Pyridazin, Pyrimidin, Pyrazin, 1,2-Thiazepin, 1,3-Thiazepin, 1,4-Thiazepin, 1,2-Oxazepin, 1,3-Oxazepin, 1,4-Oxazepin, 1,2-Diazepin, 1,3-Diazepin, 1,4-Diazepin sowie deren teilweise oder ganz gesättigten Varianten. Insbesondere seien

genannt Pyrrolidin, Piperidin, Morpholin, Piperazin, N-Methylpiperazin oder Pyridin. Unter dem Begriff "Aminosäure" werden die stereoisomeren Formen, z.B. D-und L-Formen, folgender Verbindungen verstanden:
Asparagin, Valin, Arginin, Asparaginsäure, Glutamin, Glutaminsäure, Tryptophan, β-Alanin, Lysin, Prolin, Glycin, γ-Aminobutyrat, Nε-Acetyllysin, Nδ-Acetylornithin, Nγ-Acetyldiaminobutyrat, Nα-Acetyldiaminobutyrat, Histidin, Isoleucin, Leucin, Methionin, Phenylalanin, Serin, Cystein, Threonin, Alanin und Tyrosin. L-Aminosäuren sind bevorzugt. Aminosäurereste leiten sich von den entsprechenden Aminosäuren ab. Die Kurzschreibweise der Aminosäuren erfolgt nach der allgemein üblichen Schreibweise. Der Aminosäurerest ist durch eine Amidbindung an den Rest -NH- gebunden oder über eine Esterbindung an den Rest -O-.

Folgende Aminosäurereste sind bevorzugt:
Gly, Ala, Phe, Ser, Thr, Val, β-Ala, γ-Aminobutyrat, Asp, Glu, Asn, Gln, Nε-Acetyllysin, NΔ-Acetylornithin, Nγ-Acetyldiaminobutyrat, Nα-Acetyldiaminobutyrat, Lys oder Tyr.

Bevorzugt ist eine Verbindung der Formel I und/oder ein physiologisch verträgliches Salz der Verbindung der Formel I und/oder eine stereoisomere Form der Verbindung der Formel I, wobei ein Rest $R^1$ oder $R^2$ die Bedeutung der Formel II hat und der andere Rest $R^1$ oder $R^2$ hat die nachstehende Bedeutung:

a) 2-Pyridyl,
b) 4-Pyridyl,
c) Thiophen-3-yl,
d) 2-Thiazolyl,
e) ein Rest der Formel III,

dabei ist n die Zahl Null, 1, 2, 3 oder 4,
m ist die Zahl Null oder 1,
Z ist

1) -O- oder
2) -NH-, oder

$R^5$ und $R^6$ sind unabhängig voneinander

1) Wasserstoffatom,
2) ($C_1$-$C_2$)-Alkyl,
3) Phenyl oder
4) OH, oder

$R^6$ und $R^7$ sind miteinander verbunden und bilden zusammen eine Methylengruppe, oder
$R^7$ ist

1) Wasserstoffatom,
2) Trifluormethylsulfonyl,
3) eine Aminosäure aus der Gruppe Gly, Phe, Ala, Lys, Tyr oder Ser, oder
4) ein Rest der Formel IV,

dabei ist o die Zahl Null oder 1,
$R^9$ und $R^{10}$ sind unabhängig voneinander

1) Wasserstoffatom oder
2) Methyl,

$R^{11}$ ist:

1) OH,
2) -O-($C_1$-$C_4$)-Alkyl,
3) ($C_1$-$C_{18}$-)Alkyl,
4) ($C_1$-$C_6$)-Alkyl, ein oder mehrfach unabhängig voneinander substituiert durch

4.1 -COOH,
4.2 OH oder

4.3 O-Acetyl,

5) -COOH,
6)

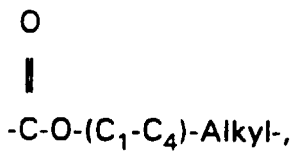

7) N-Glycyl,
8) N-Glycyl-$(C_1-C_4)$-Alkylester,
9) N-Methyl-hydroxylamino,
10) N-(1 H-Tetrazol-5-yl-amino),
11) 5-Methyl-isoxazol-4-yl,
12) 1-Cyano-2-hydroxy-1-propenyl oder
13) Phenyl, einfach substituiert durch

13.1 4-(4-Morpholino)-methyl,

f) ein Rest der Formel V,

dabei ist $Q_1$

1) $-(CH_2)_m-$, wobei m die Zahl Null, 1 oder 2 ist, oder
2) -CH=CH- und

$R^8$ ist

1) Wasserstoffatom,
2) Methyl,
3) OH,
4) $-O-(C_1-C_2)$-Alkyl,
5) Amino,
6) N-(1H-Tetrazol-5-yl)-amino oder
7) N-3,5-Dimethyl-4-hydroxybenzyl, oder

g) ein Rest der Formel VI,

dabei ist $Q_2$

1) $-(CH_2)_m-$, wobei m die Zahl Null, 1 oder 2 ist, oder
2) -CH=CH-,

X und Y sind unabhängig voneinander

1) Methyl,
2) $-O-(C_1-C_2)$-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) $(C_1-C_2)$-Alkyl, oder

$R^2$ und $R^4$ bilden zusammen mit dem Kohlenstoffatom, an das sie gebunden sind, einen aliphatischen Ring mit 3, 4 oder 5 Kohlenstoffatomen,
$R^3$ ist

1) Wasserstoffatom,
2) Methyl oder
3) Hydroxymethyl.

Insbesondere bevorzugt ist eine Verbindung der Formel I und/oder ein physiologisch verträgliches Salz der Verbindung der Formel I und/oder eine stereoisomere Form der Verbindung der Formel I, wobei ein Rest $R^1$ oder $R^2$ die Bedeutung der Formel II hat und der andere Rest $R^1$ oder $R^2$ hat die nachstehende Bedeutung:

a) 2-Pyridyl,
b) 4-Pyridyl,
c) Thiophen-3-yl,
d) 2-Thiazolyl,
e) ein Rest der Formel III,

dabei ist n die Zahl Null, 1, 2, 3 oder 4,
m ist die Zahl Null oder 1,
Z ist

1) -O- oder
2) -NH-, oder

$R^5$ und $R^6$ sind unabhängig voneinander

1) Wasserstoffatom,
2) $(C_1-C_2)$-Alkyl, oder
3) Phenyl, oder

$R^6$ und $R^7$ sind miteinander verbunden und sind zusammen eine Methylengruppe, oder
$R^7$ ist

1) Wasserstoff,
2) Trifluormethylsulfonyl,
3) eine Aminosäure aus der Gruppe Gly, Lys oder Phe oder
4) ein Rest der Formel IV,

dabei ist o die Zahl Null oder 1,
$R^9$ und $R^{10}$ sind unabhängig voneinander

1) Wasserstoffatom oder
2) Methyl,

$R^{11}$ ist

1) OH,
2) O-Methyl oder O-Ethyl,
3) $(C_1-C_{18})$-Alkyl,
4) $(C_1-C_6)$-Alkyl, ein oder mehrfach unabhängig voneinander substituiert durch

4.1 -COOH,
4.2 OH oder
4.3 O-Acetyl,

5) -COOH,
6)

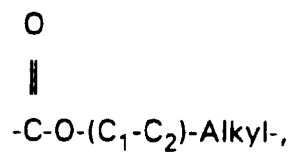

7) N-Glycyl,
8) N-Glycyl-($C_1$-$C_2$)-Alkylester,
9) N-Methyl-hydroxylamino,
10) N-(1H-Tetrazol-5-yl-amino),
11) 5-Methyl-isoxazol-4-yl oder
12) 1-Cyano-2-hydroxy-1-propenyl,

f) ein Rest der Formel V,

dabei ist $Q_1$

1) $-(CH_2)_m-$, wobei m die Zahl O, 1 oder 2 ist, oder
2) -CH=CH- und

$R^8$ ist

1) Wasserstoffatom,
2) Methyl,
3) OH,
4) -O-Methyl,
5) Amino,
6) N-(1H-Tetrazol-5-yl)-amino oder
7) N-3, 5-Dimethyl-4-hydroxybenzyl, oder

g) ein Rest der Formel VI,

dabei ist $Q_2$

1) $-(CH_2)_m-$, wobei m die Zahl Null oder 2 ist, oder
2) -CH = CH-,

X und Y sind unabhängig voneinander

1) Methyl,
2) -O-($C_1$-$C_2$)-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) Methyl, oder

$R^2$ und $R^4$ bilden zusammen mit dem Kohlenstoffatom, an das sie gebunden sind, einen aliphatischen Ring mit 3, 4 oder 5 Kohlenstoffatomen,
$R^3$ ist

1) Wasserstoffatom oder
2) Hydroxymethyl.

Ganz besonders bevorzugt sind die in Tabelle 1 mit Strukturformeln aufgeführten Verbindungen.

Die Erfindung betrifft auch ein Verfahren zur Herstellung von 2-Isoxazolin oder Isoxazol der Formel I und/oder einer stereoisomeren Form der Verbindung der Formel I und/oder eines physiologisch verträglichen Salzes der Verbindung der Formel I, das dadurch gekennzeichnet ist, daß man

a) eine primäre Nitroverbindung mittels Isocyanaten und katalytischen Mengen Triethylamin oder ein durch Chlorierung eines entsprechenden Aldoximes erhaltes Hydroxamsäurechlorid mit einer organischen oder anorganischen Base in das entsprechende Nitriloxid überführt und dieses intermediär erhaltene Nitriloxid ohne Reinigung mit einem entsprechend substituierten Olefin oder Alkin im Sinne einer 1,3-dipolaren Cycloaddition zu einem 2-Isoxazolin oder Isoxazol der Formel I umsetzt und das entstandene Produkt gegebenenfalls durch Kristallisation oder Chromatographie reinigt, oder

b) einen nach a) oder o) hergestellten Carbonsäureester der Formel I oder eine über Verfahren h) oder k) zusätzlich eingeführte Estergruppe zur Carbonsäure verseift, oder

c) einen nach Verfahren a) oder o) hergestellten Carbonsäurealkylester der Formel I mit einem entsprechend substituierten primären oder sekundären Amin in das entsprechende Amid überführt, oder

d) einen nach a) oder o) hergestellten Phosphinsäuremonoalkyl- oder Phosphonsäuredialkylester der Formel I zum Phosphonsäurehalbester, zur Phosphonsäure oder zur Phosphinsäure verseift, oder

e) eine nach b) erhaltene Carbonsäure zunächst in ein aktiviertes Säurederivat überführt, dieses anschließend mit Alkoholen verestert oder mit primären und sekundären Aminen in das entsprechende Amid oder mit N-alkyliertem Hyroxylamin in das entsprechende Hydroxylamid überführt, oder

f) in einer nach den Verfahren a), b), h), e), o) oder g) intermediär erhaltene oder mitgeführte N- oder O-Schutzgruppe abspaltet, oder eingeführte oder mitgeführte Carbonsäure-, Phosphin-, Phosphon-, oder Phosphorsäureester entsprechend verseift und eine Verbindung der Formel I erhält, oder

g) eine nach Verfahren f) oder o) erhaltene Verbindung mit einer freien Aminogruppe durch Umsetzung mit Isocyanat in das entsprechende Harnstoffderivat, oder durch Umsetzung mit einem aktivierten Carbonsäurederivat oder einer N-geschützten Aminosäure in das entsprechende Amid oder durch Umsetzung mit einem Sulfonsäurechlorid in das entsprechende Sulfonamid überführt, oder

h) eine nach Verfahren a), o) oder f) erhaltene Verbindung mit einer freien Alkoholgruppe durch Umsetzung mit Isocyanat in das entsprechende Urethan oder durch Umsetzung mit einem an der Carboxygruppe aktivierten, gegebenenfalls weitere funktionelle Gruppen tragenden Carbonsäurederivat oder einem entsprechenden N-geschützten Aminosäurederivat in den entsprechenden Ester oder mit Diketen in das entsprechende 3-Oxo-butyrat oder durch Umsetzung mit einer Halogenverbindung wie $\alpha$-Halogen-Carbonsäure in den entsprechenden Ether überführt, oder

i) eine nach Verfahren a), o) oder f) erhaltene Verbindung mit einer primären oder sekundären Alkoholgruppe zu dem entsprechenden Aldehyd oder Keton oxidiert, oder

k) eine nach Verfahren i) hergestellte Carbonylverbindung durch Umsetzung mit einer Base und Dialkylphosphonoessigsäureester in das entsprechende Crotonsäurederivat oder durch Umsetzung mit Tetraalkylmethylendiphosphonat in das entsprechende trans-2-(Dialkoxyphosphono)-vinyl-Derivat überführt, oder

l) eine während der Cycloaddition eingeführte Epoxid-Gruppe in das entsprechende 1,2-Diol überführt, oder

m) ein nach Verfahren g) hergestelltes Amid, das einen 3-H-Isoxazolring enthält, durch Behandlung mit einer Base im Sinne einer Ringöffnung in das entsprechende substituierte 2-Cyano-3-hydroxy-crotonsäureamid überführt, oder

n) eine nach Verfahren a) - m) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppe in die reinen

Enantiomeren auftrennt, oder

o) ein durch Cycloaddition an chirale oder racemische Olefine erhaltenes 2-Isoxazolin-Diastereomerengemisch der Formel I durch Säulenchromatographie an Kieselgel in die reinen Diastereomeren auftrennt, oder

p) die nach Verfahren a) - o) hergestellte Verbindung der Formel 1 entweder in freier Form isoliert oder im Falle des Vorliegens von sauren oder basischen Gruppen gegebenenfalls in physiologisch verträgliche Salze umwandelt, oder

q) eine nach Verfahren h) hergestellte Verbindung, die eine weitere funktionelle Gruppe wie eine Halogenmethylgruppe trägt, mit einer primären oder sekundären Aminogruppe alkyliert oder mit einem Alkohol verethert.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren, Phosphon- und Phosphinsäuren sowie die Phosphonsäurehalbester bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder auch basischen Aminosäuren, etwa Lysin, Ornithin oder Arginin stabile Alkali-, Erdalkali- oder gegebenenfalls substituierte Ammoniumsalze. Sofern die Verbindungen der Formeln I basische Gruppen im Rest $R^1$ oder $R^2$ aufweisen, lassen sich mit starken Säuren auch stabile Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, oder Trifluoressigsäure in Frage.

Die Herstellung und Umsetzung der als Ausgangsstoffe für 1,3-dipolare Cycloadditionen verwendeten Nitriloxide ist in einer aktuellen Monographie beschrieben (K.P.G. Torsell: Nitrile Oxides, Nitrones and Nitronates in Organic Synthesis, VCH Verlagsgesellschaft, Weinheim, 1988). Die als Vorstufen dienenden Hydroxamoylhalogenide sind nach literaturbekannten Verfahren durch Halogenierung entsprechender Aldoxime oder im Falle von Chloroxyiminoessigsäureestern über eine Diazotierungsreaktion ausgehend von Glycinalkylestern (G. S. Skinner, J. Am Chem. Soc. 64 (1924), 731) erhältlich. Bei Verwendung von tert.-Butylhypochlorit zur Chlorierung der Aldoxime kann auf eine Isolierung der entsprechenden Hydroxamoylchloride verzichtet werden. Die ebenfalls verwendeten Nitroverbindungen sind teilweise literaturbekannt oder lassen sich nach im Prinzip literaturbekannten Methoden herstellen, so zum Beispiel die 4-Nitrobuttersäureester durch Fluorid- oder Basen-katalysierte Addition von Nitromethan an Acrylsäurederivate (D. W. Chasar, Synthesis 1982, 841 - 42; N. Ono, Synthesis 1984, 226 - 227). Durch Verwendung von 1,8-Diazabicylo [5.4.0]undec-7-en (DBU) als Base und eine spezielle Reaktionsführung lassen sich die literaturbekannten Ausbeuten wesentlich verbessern sowie das Entstehen der normalerweise als schwer abtrennbaren Nebenprodukte auftretenden Bis-Addukte weitestgehend verhindern.

Die weiterhin als Reaktionspartner dienenden, gegebenenfalls substituierten Alken- oder Alkinderivate sind als Grundkörper zumeist literaturbekannt oder sogar käuflich. Entsprechende Hinweise sind bei den Beispielen aufgeführt. Die Erzeugung der leicht zur Oligomerisierung neigenden Nitriloxide geschieht vorteilhaft "in situ" in Gegenwart der olefinischen oder acetylenischen Reaktionspartner ohne zwischenzeitliche Isolierung. Im Falle der Erzeugung aus Nitroverbindungen nach Mukaiyama werden zur Dehydratisierung bevorzugt aromatische Isocyanate, zum Beispiel Phenylisocyanat oder Diisocyanatobenzol eingesetzt. Dabei empfielt es sich, in einem gegenüber den Reaktionspartnern inerten aprotischen Lösungs- oder Verteilungsmittel zu arbeiten wie Ethylacetat, Dimethylformamid, Dimethylacetamid, Dialkylether, Tetrahydrofuran, halogenierten Kohlenwasserstoffen, z.B. Dichlormethan, Chloroform oder Dichlorethan, Kohlenwasserstoffen wie Hexan, Cyclohexan, Benzol, Toluol oder anderen substituierten Aromaten, wobei auch Mischungen der vorgenannten Lösungsmittel in Frage kommen. Zur Erzeugung der Nitriloxide aus Hydroxamoylhalogeniden werden organische oder anorganische Basen wie tertiäre Amine, Alkalicarbonate oder -Hydroxide verwendet. Dabei arbeitet man unter Verwendung organischer Basen bevorzugt in den vorgenannten, gegebenenfalls chlorierten aliphatischen oder aromatischen Kohlenwasserstoffen oder aliphatischen, auch cyclischen Ethern, während bei Verwendung anorganischer Basen zusätzlich auch in zweiphasigen Lösungsmittelgemischen, zum Beispiel Ethylacetat/Wasser oder Dichlormethan/Wasser gearbeitet werden kann. Die Herstellung der Nitriloxide und die Cycloaddition erfolgen in der Regel bei Temperaturen von - 20° C bis + 80° C, vorzugsweise jedoch von 0° bis + 40° C.

Die Verseifung der bei der Cycloaddition mitgeführten oder über weitere Reaktionen später eingeführten Alkylester zu den entsprechenden Carbonsäuren gelingt in der Regel mit wässrigem Alkali, das äquimolar oder im Überschuß eingesetzt werden kann, in wässriger Lösung oder, falls das Edukt schlecht wasserlöslich ist, in wässrig-organischen Lösungsmittelgemischen, wobei sich Wasser-Alkohol Gemische besonders bewährt haben.

Die Spaltung der in den Substituenten $R^1$ oder $R^2$ der allgemeinen Formel I mitgeführten Amin- Alkohol- oder Carbonsäure-Schutzgruppen erfolgt meist nach aus der Peptidchemie bekannten Methoden, wobei im Falle der vorzugsweise verwendeten tert.-Butoxycarbonylgruppe eine saure Abspaltung, zum Beispiel mit alkoholischer Salzsäure

oder Trifluoressigsäure vorzuziehen ist, während niedere Alkylester bevorzugt alkalisch gespalten werden. Die Umwandlung der Phosphonsäure- und Phosphinsäureester in die entsprechenden freien Säuren gelingt unter sauren Bedingungen, bevorzugt in wasserfreiem Medium, zum Beispiel durch Verwendung von Bromwasserstoffsäure in organischen Säuren wie Essigsäure, oder durch Verwendung von Trimethylbromsilan oder Trimethyljodsilan in aprotischen Lösungsmitteln, wobei bevorzugt halogenierte Kohlenwasserstoffe eingesetzt werden. Dabei wird als Reaktionstemperatur zur schonenden Spaltung bevorzugt ein Bereich von 0° bis 50° C gewählt. Zur Herstellung der Phosphonsäurehalbester werden die Phosphonsäurediester in der Regel einer alkalischen Hydrolyse unterworfen.

Verbindungen der Formel I mit einer freien Carbonsäurefunktion in $R^1$ oder $R^2$ können nach entsprechender Aktivierung der Carboxygruppe mit primären und sekundären Aminen sowie auch mit N-alkylierten Hydroxylaminen oder 5-Aminotetrazol in an sich bekannter Weise in die entsprechenden Amide überführt werden.

Verbindungen der Formel I mit einer freien Alkohol- oder Aminfunktion in $R^1$ oder $R^2$ können mit aktivierten Carbonsäurederivaten wie Säurehalogeniden oder -Anhydriden in die entsprechenden Ester- oder Amid-Derivate überführt werden. Auch können, ausgehend von N-geschützten Aminosäuren entsprechende Ester oder Amide erhalten werden. Zur Aktivierung der Aminosäurekomponente benutzt man in diesem Fall vorteilhaft aus der Peptidchemie bekannte Methoden, zum Beispiel die Hydroxybenzotriazol/Dicyclohexylcarbodiimid-Methode (W. König, R. Geiger, Chem. Ber. 103 (1970), 2034-2040) oder die Aktivierung mittels Propanphosphonsäreanhydrid (PPA), wobei als Lösungsmittel neben aliphatischen und cyclischen Ethern auch chlorierte Kohlenwasserstoffe sowie Dimethylformamid Verwendung finden können. Als Hilfsbasen bevorzugt sind tertiäre Amine wie Triethylamin, N-Ethylmorpholin oder Pyridin. Es wird im Temperaturbereich von -10° C bis +50° C, vorzugsweise bei 0° C bis +20° C gearbeitet.

Sofern die in geschützter Form vorliegenden Acylderivate in der Form freier Amino- oder Carbonsäurefunktionen gewünscht werden, lassen sich die entsprechenden Schutzgruppen einzeln oder auch gemeinsam auf den oben bereits beschriebenen Wegen entfernen.

Weiterhin lassen sich Verbindungen der Formel I mit einer freien Alkohol- oder Aminfunktion in $R^1$ oder $R^2$ durch Addition an entsprechend substituierte Isocyanate in Urethan- oder Harnstoff-Derivate überführen. Die gegebenenfalls bei der Isocyanat-Addition mitgeführten oder als zusätzliche Funktion eingeführten Estergruppen können, wie oben beschrieben, in die entsprechenden Carbonsäuren überführt werden.

Verbindungen mit einer freien primären oder sekundären Alkoholfunktion können in einfacher Weise nach der als "Swern-Oxidation" bekannten Methode mittels Dimethylsulfoxid und Oxalylchlorid zu der entsprechenden Aldehyden oder Ketonen oxidiert werden. Dabei können durch entsprechnde Reaktionsführung bei tiefer Temperatur Nebenreaktionen wie Oxidation des Phenolringes oder oxidativer Abbau der 2-Isoxazolinringes vermieden werden. Die so erhaltenen Carbonylverbindungen können mittels Basen und CH-aciden Verbindungen wie Dialkylphosphonoessigsäurealkylestern oder Tetraalkyl-methylendiphosphonat im Sinne einer Kondensation in die entsprechend substituierten trans-Olefine überführt werden.

Sofern die Verbindungen der allgemeinen Formel I in diastereoisomeren oder enantiomeren Formen auftreten und bei der gewählten Synthese als deren Gemische anfallen, gelingt die Trennung in die reinen Stereoisomeren entweder durch Chromatographie an einem gegebenenfalls chiralen Trägermaterial, oder, sofern die racemischen Verbindungen der Formel I zur Salzbildung befähigt sind, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren der in der Regel racemisch anfallenden 2-Isoxazoline mit asymmetrischem C-Atom in 5-Position eignen sich beispielsweise modifizierte Kieselgelträger (sogenannte Pirkle-Phasen) sowie hochmolekulare Kohlenhydrate, zum Beispiel Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren, Phosphonsäuren, und Phosphinsäuren werden mit einer optisch aktiven, in der Regel kommerziell erhältlichen Base, zum Beispiel (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen.

Auf prinzipiell gleiche Weise kann man die racemischen Verbindungen der Formel I, die eine basische Gruppe wie eine Aminogruppe enthalten, mit optisch aktiven Säuren wie (+)-Campher-10-sulfonsäure, D- und L- Weinsäure, D-und L-Milchsäure oder (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Die Erfindung betrifft auch Arzneimittel, gekennzeichnet durch einen wirksamen Gehalt an mindestens einer Verbindung der Formel I und/oder eine stereoisomere Form der Verbindung der Formel I und/oder ein physiologisch verträgliches Salz der Verbindung der Formel I, neben pharmazeutisch geeigneten und physiologisch verträglichen Trägerstoffen, Zusatzstoffen und/oder Wirk- und Hilfsstoffen. Die erfindungsgemäßen Arzneimittel können intravenös, parenteral, topisch, rektal oder oral verabreicht werden.

Die erfindungsgemäßen Arzneimittel eigenen sich vorzugsweise zur Prophylaxe und/oder Therapie von asthmatischen Erkrankungen, Entzündungen und Autoimmunerkrankungen.

Dazu gehören beispielsweise rheumatische Erkrankungen, akute und chronische Entzündungen von Muskeln, Gelenken oder des Magen-Darm-Traktes, allergische Atemwegserkrankungen, Psoriasis oder Autoimmunerkrankungen, z.B. systemischer Lupus erythematodes (SLE), Typ-II Diabetes, Myasthenia gravis, Sjögren-Syndrom, Dermatomyositis, Sklerodermie oder Multiple Sklerose (MS).

Die Erfindung betrifft auch ein Verfahren zur Herstellung eines erfindungsgemäßen Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykole und Lösungsmittel, wie etwa steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindung der Formel I - bei Mensch und Tier Tagesdosen von etwa 50 bis 3000 mg Wirkstoff, vorzugsweise etwa 150 bis 1000 mg, bei oraler Verabreichung und von etwa 50 bis 1000 mg, bevorzugt etwa 100 bis 300 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen. Schließlich können die Verbindungen der Formel I und/oder gegebenenfalls ihre physiologisch verträglichen Salze bei der Herstellung der vorgenannten galenischen Darreichungsformen auch zusammen mit anderen geeigneten Wirkstoffen, z.B. durchblutungsfördernden Substanzen, Plättchenaggregationshemmern, Thrombozytenaggregationshemmern, Calciumantagonisten, Antithrombotika, Antihyperlipidämika, Neuroprotektiva, Analgetika, Sedativa, Antidepressiva, Antiinflammatorika, antianginösen Mitteln, Cardiotonika, Antiarrhytmika, Diuretika, Antihypertensiva einschließlich β-Rezeptor- und Calcium-Blockern, Plasmaexpandern und anderen Vasotherapeutika, formuliert werden.

Die Strukturformeln der nachfolgend beschriebenen Herstellungsbeispiele sind zusammen mit den Schmelzpunkten und den $^1$H-NMR-Daten in der Tabelle 1 zusammengefaßt. Beim Vorliegen stereoisomerer Formen ist in den Strukturformeln die relative Konfiguration angegeben. Die delta-Werte der nachfolgend aufgeführten NMR-Spektren sind in ppm angegeben.

Beispiel 1:

3-(4-Hydroxy-3,5-di-tert.-butyl-phenyl)-5-hydroxymethyl-2-isoxazolin

a) 3-(4-Hydroxy-3,5-di-tert.-butyl)-benzaldoxim
Ausgehend von 3-(4-Hydroxy-3,5-di-tert.-butyl)-benzaldehyd wurde nach literaturbekannten Verfahren (Houben-Weyl: Meth. d. Org. Chem., Bd X/4, S. 55 f)) das Oxim hergesteHt.
b) 1,3-dipolare Cycloaddition mit Nitriloxid
24,9 g (0,1 mol) des unter a) hergestellten Aldoxims wurden in 250 ml Dichlormethan gelöst, unter Kühlung wurden 12,0 g (0,11 mol) tert.-Butylhypochlorit zugetropft und nach Beendigung des Zutropfens wurde das Gemisch noch

45 min bei Raumtemperatur nachgerührt. Nach Zugabe von 11,6 g (0,2 mol) Allylalkohol wurden langsam (während 6 Stunden) 16,7 ml, (0,12 mol) Triethylamin, gelöst in 150 ml Dichlormethan, zugetropft. Man rührte über Nacht, wusch mit Wasser, verdünnter wässriger Zitronensäure und NaCl-Lösung, trocknete über Natriumsulfat und engte unter vermindertem Druck ein. Das Produkt wurde durch Kristallisation aus tert.-Butylmethylether/Petrolether in kristalliner Form erhalten. Ausbeute: 13,1 g

Die in der nachfolgenden Tabelle 1 beschriebenen Beispiele:
2, 5, 8, 9, 10, 11, 12, 13, 14, 15, 16, 17, 18, 19, 20, 21, 22, 23, 24, 25, 26, 27, 28, 29, 30, 31, 32, 35, 44, 48, 66, 67, 68, 69, 78, 79 und 80 wurden analog zu Beispiel 1 durch Cycloaddition der in situ hergestellten Nitriloxide an die entsprechenden Olefine oder Alkine hergestellt.

Bei zunächst ölig anfallenden Rohprodukten hat sich außerdem die Chromato-graphie an Kieselgel mit tert.-Butylmethylether-Petrolether oder Ethylacetat-Petrolether-Gemischen und nachfolgende Kristallisation bewährt.

Besonderheiten zur Synthese der in Tabelle 1 aufgeführten Beispiele:

Zu Beisp. 2):
Die Synthese des Olefinbausteins ist in EP 0220573 beschrieben.
Zu Beisp. 8):
3-Vinyl-thiophen: CAS-Nr. 13679-64-6
Zu Beisp. 11):
Cycloaddition an Acrylsäuremethylester. Überführung ins Amid erfolgte mittels gesättigter methanolischer Ammoniaklösung im Überschuß bei Raumtemperatur.
Zu Beisp. 14):
Cycloaddition an N-(3,5-Dimethyl-4-hydroxybenzyl)-methacrylamid (Synthese beschrieben in DE 3820699).
Zu Beisp. 15):
Aufgrund der nicht stereoselektiven Cycloaddition fällt das Produkt als Diastereomerengemisch an.
Zu Beisp. 16 u. 17):
Das bei der Cyclcaddition an Butadienmonoxid anfallende racemische erythro/ threo-Diastereomerengemisch wurde durch Chromatografie an Kieselgel mittels Petrolether/Ethylacetat 9:1 und anschließende Kristallisation in die Diastereomeren aufgetrennt (Beispiel 16: rac. erythro-, Beispiel 17: rac. threo-isomer).
Zu Beisp. 18-20):
Das durch Cycloaddition an 1-Buten-3-ol anfallende racemische erythro/threo-Diastereomerengemisch (Beisp. 18) wurde durch Chromatographie an Kieselgel mittels Petrolether/tert.-Butylmethylether 5:1 in die Diastereomeren aufgetrennt (Beisp. 19: rac. erythro-, Beisp. 20: rac. threo-Isomer).
Zu Beisp. 21-22):
Das durch Cycloaddition an 1-Buten-3-ol-acetat anfallende racemische erythro/threo-Diastereomerengemisch wurde durch Chromatographie an Kieselgel mittels Petrolether/Ethylacetat 19:1 in die Diastereomeren aufgetrennt (Beisp. 21: rac. erythro-, Beip. 22: rac. threo-Isomer).
Zu Beisp. 23):
Die Cycloaddition wurde mit Acrylsäure-tert.-butylester durchgeführt. Die Abspaltung der tert.-Butylestergruppe erfolgte mittels Trifluoressigsäure in Dichlormethan bei Raumtemperatur. Das Produkt konnte nach Einengen aus tert.-Butylmethylether/Petrolether kristallisiert werden.
Zu Beisp. 35):
Die Synthese des Olefinbausteins ist in EP 0220573 beschrieben.
Zu Beisp. 48):
Die Cycloaddition wurde mittels N-tert.-Butoxycarbonyl-propargylamin durchgeführt. Die Abspaltung der N-Schutzgruppe erfolgte anschließend mit Trifluoressigsäure. Das Produkt konnte als Trifluoracetat kristallin aus Methanol/tert.-Butylmethylether erhalten werden. Die freie Base erhielt man durch Extraktion aus wässriger verdünnter NaOH mittels Dichlormethan, Trocknen und Einengen der organischen Phase.
Zu Beisp. 78):
Die Cycloaddition wurde mit 6-Hepten-2,4-diol durchgeführt. Durch Chromatographie an Kieselgel mit tert.-Butylmethylether-Petrolether und anschließender mehrfachen Kristallisation aus den obigen Lösungsmitteln wurde ein nach NMR-Spektroskopie isomerenreines Produkt erhalten.
Zu Beispielen 79) und 80):
Die Cycloaddition wurde mit einem Überschuß an 1,6-Heptadien-4-ol in Analogie zu Beispiel 1) durchgeführt. Die Trennung in die Diastereoisomeren erfolgte durch Chromatographie an Kieselgel mittels Petrolether/Ethylacetat-Gemischen (Gradient 6:1 - 2:1). Die Zuordnung der threo/erythro-Isomeren war nach NMR-Spektroskopie nicht möglich.

Beispiel 3:

2-(3-(4-Hydroxy-3,5-di-tert.-butyl-phenyl)-2-isoxazolin-5-ylmethoxy)-methyl-propionsäure

4,0 g (0,0095 mol) des Produktes aus Beispiel 2 wurden in 80 ml Methanol gelöst, 25 ml wässriger 1 N NaOH wurde zugesetzt und nach Beendigung der Verseifung (DC-Kontrolle) mit 25 ml 1 N HCl angesäuert. Das ausgefallene Produkt wurde filtriert, mit Wasser gewaschen und getrocknet. Ausbeute: 3,0 g

Beispiel 4:

N-(3-(4-Hydroxy-3,5-di-tert.-butyl-phenyl)-2-isoxazolin-5-ylmethoxycarbonyl)-glycin

a) Umsetzung mit Isocyanat
5,5 g ( 0,018 mol) des Produktes aus Beispiel 1 wurden in 5 ml Dichlormethan gelöst, 3,2 g (0,025 mol) Ethylisocyanoacetat und etwa 4 Tropfen Triethylamin wurden zugesetzt und unter Rühren auf 50-60 °C erwärmt. Nach Beendigung der Reaktion (DC-Kontrolle) wurde eingeengt und an Kieselgel mittels tert.-Butylmethylether/Petrolether chromatographiert. Ausbeute: 7,2 g öliges Produkt

b) Verseifung des Ethylesters
7,2 g des Produktes von a) wurden, wie in Beispiel 3) beschrieben, mit einem Überschuß an 1 N NaOH verseift und nach Ansäuern in kristalliner Form isoliert. Ausbeute: 4,56 g.

Beispiel 6:

3-(4-Hydroxy-3,5-di-tert.-butyl-phenyl)-2-isoxazolin-5-yl-phosphonsäure

5,0 g (0,013 mol) des Produktes aus Beispiel 5 wurden bei Raumtemperatur mit etwa 100 ml einer 33%-igen Lösung von HBr in Eisessig bis zur Vollständigkeit der Reaktion (DC-Kontrolle) behandelt. Der nach Einengen verbliebene Rückstand wurde mit tert.-Butylmethylether ausgerührt. Ausbeute: 3,15 g.

Beispiel 7:

3-(4-Hydroxy-3,5-di-tert.-butyl-phenyl)-2-isoxazolin-5-yl-phosphonsäure-monomethylester

5,0 g (0,013 mol) des Produktes aus Beispiel 5 wurden bei Raumtemperatur in 100 ml Methanol gelöst, 30 ml 1 N NaOH wurden zugesetzt und 15 Stunden gerührt (DC-Kontrolle). Nach Ansäuern (1 N HCl) wurde mit Dichlormethan extrahiert, mit Wasser und gesättigter NaCl-Lösung gewaschen, getrocknet und eingeengt. Der Rückstand wurde aus tert.-Butylmethylether/Petrolether kristallisiert. Ausbeute: 3,4 g.

Beispiele 21 und 22 (alternative Synthese):

Essigsäure-1-(3-(4-Hydroxy-3,5-di-tert.-butyl-phenyl)-2-isoxazolin-5-yl)-ethylester (erythro- bzw. threo-Isomer)

2,5 g (0,008 mol) des Produktes aus den Beispielen 19 oder 20 wurden in 100 ml Dichlormethan und 6 ml Pyridin gelöst. Nach Zugabe von 125 mg 4-Dimethylaminopyridin wurde unter Eiskühlung 1,35 ml (0,024 mol) Acetylchlorid, gelöst in 10 ml Dichlormethan, zugetropft, nach Beendigung der Reaktion (DC-Kontrolle) wurde mit Wasser, wässriger Kaliumhydrogensulfatlösung und Wasser gewaschen, getrocknet und eingeengt. Das verbliebene Öl konnte aus tert.-Butylmethylether/Petrolether kristallisiert werden.

Beispiel 33:

(1-(3-(4-Hydroxy-3,5-di-tert.-butyl-phenyl)-isoxazol-5-yl)-1-metyl-ethoxy)-essigsäureethylester

10,0 g (0,03 mol) des Produktes aus Beispiel 29 wurden, gelöst in 40 ml absoluten Dimethylformamid, unter Schutzgas und Eiskühlung zu einer Suspension von 1,06 g (0,044 mol) Natriumhydrid in 30 ml absoluten Dimethylformamid getropft. Nach weiteren 30 min ohne Kühlung wurden 10,8 g (0,044 mol) Bromessigsäureethylester, gelöst in 30 ml absoluten Dimethylformamid, zugetropft und weitere 4 Stunden nachgerührt. Der Reaktionsansatz wurde auf

Eis gegossen, mit Ethylacetat extrahiert, mit Wasser gewaschen, getrocknet und eingeengt. Der ölige Rückstand wurde aus Petrolether kristallisiert. Ausbeute: 6,8 g.

Beispiel 34:

(1-(3-(4-Hydroxy-3,5-di-tert.-butyl-phenyl)-isoxazol-5-yl)-1-metyl-ethoxy)-essigsäure

3,7 g (0,009 mol) des Produktes aus Beispiel 33 wurden in Analogie zu Beispiel 3 verseift. Ausbeute: 2,9 g.

Beispiel 36:

2-(3-(4-Hydroxy-3,5-di-tert.-butyl-phenyl)-isoxazol-5-ylmethoxy)-2-methylpropionsäure

9,5 g (0,023 mol) des Produktes aus Beispiel 35 wurden in Analogie zu Beispiel 3 verseift. Ausbeute: 6,6 g.

Beispiel 37:

N-Methyl-hydroxamoyl-2-((3-(4-Hydroxy-3,5-di-tert.-butyl-phenyl)-isoxazol-5-ylmethoxy)-2-methylpropionat

4,0 g (0,01 mol) des Produktes aus Beispiel 36 wurden in Analogie zu einer Literaturvorschrift (EP 0 199 151) mittels Oxalsäurechlorid und N-Methylhydroxylamin derivatisiert. Das Produkt wurde aus tert.-Butylmethylether kristallisiert. Ausbeute: 2,7 g.

Beispiel 38:

2-(3-(4-Hydroxy-3,5-di-tert.-butyl-phenyl)-isoxazol-5-ylmethoxy)-N-(1H-tetrazol-5-yl)-isobutyramid

6,0 g (0,015 mol) des Produktes aus Beispiel 36 wurden in Analogie zu einer Literaturvorschrift (J Org Chem, 34 (1969), 2766-2767) mit 1,7 ml (0,015 mol) Siliciumtetrachlorid und 1,25 g (0,015 mol) 5-Amino-tetrazol in absoluten Pyridin derivatisiert. Das Produkt wurde aus tert.-Butylmethylether/Petrolether kristallisiert. Ausbeute: 3,9 g.

Beispiel 39:

N-(3-(4-Hydroxy-3,5-di-tert.-butyl-phenyl)-isoxazol-5-ylmethoxycarbonyl)-glycinethylester

14,0 g (0,046 mol) des Produktes aus Beispiel 24 wurden in Analogie zu Beispiel 4a) umgesetzt. Das verbleibende Öl wurde durch Chromatographie an Kieselgel mit tert.-Butylmethylether/Petrolether 1:4 gereinigt. Ausbeute: 16,4 g.

Beispiel 40:

N-(3-(4-Hydroxy-3,5-di-tert.-butyl-phenyl)-isoxazol-5-ylmethoxycarbonyl)-glycin

7,1 g (0,06 mol) des Produktes aus Beispiel 39 wurden in Analogie zu Beispiel 3 verseift. Ausbeute: 4,1 g.

Beispiel 41:

N-(3-(4-Hydroxy-3,5-di-tert.-butyl-phenyl)-isoxazol-5-ylpropoxycarbonyl)-glycinethylester

8,0 g (0,024 mol) des Produktes aus Beispiel 26 wurden in Analogie zu Beispiel 4a) umgesetzt. Das verbleibende Öl wurde durch Kristallisation aus tert.-Butylmethylether/Petrolether gereinigt. Ausbeute: 6,1 g.

Beispiel 42:

N-(3-(4-Hydroxy-3,5-di-tert.-butyl-phenyl)-isoxazol-5-ylpropoxycarbonyl)-glycin

4,0 g (0,0089 mol) des Produktes aus Beispiel 41 wurden in Analogie zu Beispiel 3 verseift. Ausbeute: 2,8 g.

Beispiel 43:

N-(2-(4-Hydroxy-3,5-di-tert.-butyl-phenyl)-isoxazol-5-ylethoxycarbonyl)-glycin

6,25 g des Produktes aus Beispiel 25 wurden in Analogie zu Beispiel 4a) umgesetzt. Das verbleibende Produkt wurde in Analogie zu Beispiel 3 verseift, durch Chromatographie an Kieselgel mittels tert.-Butylmethylether und schließlich durch Kristallisation aus tert.-Butylmethylether/Petrolether gereinigt. Ausbeute: 3,8 g.

Beispiel 45:

3-(4-Hydroxy-3,5-di-tert.-butyl-phenyl)-isoxazol-5-ylpropionsäure

4,3 g (0,011 mol) des Produktes aus Beispiel 44 wurden in Analogie zu Beispiel 3 verseift. Ausbeute: 3,0 g.

Beispiel 46:

3-(3-(4-Hydroxy-3,5-di-tert.-butyl-phenyl)-isoxazol-5-yl)-N-(1H-tetrazol-5-yl)-propionsäureamid

7,0 g (0,02 mol) des Produktes aus Beispiel 45 wurden in Analogie zu Beispiel 38 mittels Siliciumtetrachlorid und 5-Amino-tetrazol in absoluten Pyridin derivatisiert. Das Produkt wurde aus tert.-Butylmethylether/Petrolether kristallisiert. Ausbeute: 6,5 g.

Beispiel 47:

3-(4-Hydroxy-3,5-di-tert.-butyl-phenyl)-isoxazol-5-yl-acrylsäure

a) Swern-Oxidation zum Aldehyd
25,4 g (0,2 mol) Oxalylchlorid wurden in 250 ml absolutem Dichlormethan unter Schutzgas bei -60°C vorgelegt und anschließend wurden 31,2 ml (0,44 mol) Dimethylsulfoxid, gelöst in 20 ml absolutem Dichlormethan, zugetropft. Nach 30 min wurden 30,3 g (0,1 mol) des Produktes aus Beispiel 24, gelöst in 200 ml absoluten Dichlormethan/20 ml Dimethylformamid, bei -50°C in 45 min zugetropft und 20 min nachgerührt. Nach Zugabe von 55,6 ml (0,4 mol) Triethylamin wurde die Kühlung entfernt, 1 Stunde bei Raumtemperatur nachgerührt, mit Wasser, verdünnter wässriger Zitronensäure und NaCl-Lösung gewaschen, getrocknet und eingeengt. Das verbleibende Öl wurde durch Chromatographie an Kieselgel mit tert.-Butylmethylether/Petrolether 1:9 gereinigt und aus Petrolether kristallisiert. Ausbeute: 16,4 g.
b) Olefinierung
0,77 g (0,032 mol) Natriumhydrid wurden in 40 ml absoluten Tetrahydrofuran unter Schutzgas bei 0°C vorgelegt, 5,05 g (0,024 mol) Diethylphosphonoessigsäuremethylester, gelöst in 50 ml absoluten Tetrahydrofuran, wurden zugetropft, die entstandene Suspension wurde bei 0°C 1 Stunde nachgerührt, dann wurden 4,8 g (0,016 mol) des Produktes von Beispiel 47a), gelöst in 50 ml absolutem Tetrahydrofuran zugetropft, weitere 2 Stunden nachgerührt, in Eiswasser gegossen, mehrfach mit Dichlormethan extrahiert, mit Wasser gewaschen getrocknet und eingeengt. Das Produkt wurde aus tert.-Butylmethylether/Petrolether kristallisiert. Ausbeute: 4,2 g.
c) Verseifung
3,7 g (0,01 mol) des Produktes aus Beispiel 47b) wurden in Analogie zu Beispiel 3 verseift. Ausbeute: 3,3 g.

Beispiel 49:

N-(3-(4-Hydroxy-3,5-di-tert.-butyl-phenyl)-isoxazol-5-ylmethyl)-trifluormethansulfonamid

3,6 g (0,012 mol) der Basenform des Produktes aus Beispiel 48 wurden, gelöst in 50 ml absolutem Dichlormethan unter Eiskühlung vorgelegt, 3,95 g (0,014 mol) Trifluormethansulfonsäureanhydrid und anschließend 2,2 ml (0,017 mol) N-Ethylmorpholin wurden zugesetzt, über Nacht wurde nachgerührt, mit Wasser, verdünnter wässriger Zitronensäure und NaCl-Lösung gewaschen, getrocknet und eingeengt. Das verbleibende Öl wurde durch Chromatographie an Kieselgel mit tert.-Butylmethylether/Petrolether 3:1 gereinigt und aus tert.-Butylmethylether/Petrolether kristallisiert. Ausbeute: 2,1 g.

Beispiel 50:

N-(3-(4-Hydroxy-3,5-di-tert.-butyl-phenyl)-isoxazol-5-ylmethyl)-oxalamsäureethylester

9,1 g (0,03 mol) der Basenform des Produktes aus Beispiel 48 wurden in absolutem Tetrahydrofuran unter Eiskühlung mit 3,9 ml (0,035 mol) Chlorformylameisensäureethylester und 9,7 ml (0,07 mol) Triethylamin in Analogie zu einer Literaturvorschrift (J Med Chem, 34 (1991), 600) acyliert. Nach Zugabe von Eiswasser, und Extraktion mit Ethylacetat wurde mit Wasser, verdünnter wässriger Zitronensäure und NaCl-Lösung gewaschen, getrocknet und eingeengt. Das verbleibende Öl wurde aus tert.-Butylmethylether/Petrolether kristallisiert. Ausbeute: 9,1 g.

Beispiel 51:

N-(3-(4-Hydroxy-3,5-di-tert.-butyl-phenyl)-isoxazol-5-ylmethyl)-oxalamsäure

5,0 g (0,012 mol) des Produktes aus Beispiel 50 wurden in Analogie zu Beispiel 4b) verseift. Das ölig anfallende Produkt wurde durch Extraktion mit Dichlormethan, Waschen mit Wasser und NaCl-Lösung, Trocknen und Einengen isoliert. Das verbleibende amorphe Produkt wurde aus tert.-Butylmethylether/Petrolether kristallisiert. Ausbeute: 3,4 g.

Beispiel 52:

5-Methyl-isoxazol-4-carbonsäure-(3-(4-hydroxy-3,5-di-tert.-butyl-phenyl)-isoxazol-5-ylmethyl)-amid

5,0 g (0,0165 mol) der Basenform des Produktes aus Beispiel 48 wurden in 80 ml absolutem Tetrahydrofuran unter Eiskühlung zusammen mit 2,2 ml (0,017 mol) N-Ethylmorpholin vorgelegt, 2,5 g (0,017 mol) 5-Methyl-isoxazol-3-yl-carbonsäurechlorid, gelöst in 10 ml Tetrahydrofuran, wurden zugetropft und es wurde 5 Stunden bei Raumtemperatur nachgerührt. Nach Zugabe von Eiswasser und Extraktion mit Dichlormethan wurde mit Wasser, verdünnter wässriger Zitronensäure und NaCl-Lösung gewaschen, getrocknet und eingeengt. Das erhaltene Öl wurde durch Chromatographie an Kieselgel mit tert.-Butylmethylether/Petrolether 1:1 gereinigt und aus Petrolether kristallisiert. Ausbeute: 4,9 g.

Beispiel 53:

2-Cyano-3-hydroxy-but-2-en-carbonsäure-(3-(4-hydroxy-3,5-di-tert.-butylphenyl)-isoxazol-5-ylmethyl)-amid

4,0 g (0,01 mol) des Produktes aus Beispiel 52 wurden in 50 ml absolutem Tetrahydrofuran gelöst, unter Eiskühlung wurden 20 ml 1 N NaOH zugesetzt, nach Beendigung der Reaktion (DC-Kontrolle) wurde mit 22 ml 1N HCl angesäuert, mit Dichlormethan extrahiert, gewaschen, getrocknet und eingeengt. Der verbleibende Rückstand wurde aus tert.-Butylmethylether/Petrolether kristallisiert. Ausbeute: 3,3 g.

Beispiel 54:

(3-(3-(4-Hydroxy-3,5-di-tert.-butyl-phenyl)-isoxazol-5-ylmethyl)-ureido)-essigsäureethylester

14,2 g ( 0,047 mol) der Basenform des Produktes aus Beispiel 48 wurden in Substanz mit 6,5 g (0,05 mol) Ethylisocyanoacetat unter Rühren auf 50-60°C erwärmt. Nach Beendigung der Reaktion (DC-Kontrolle) wurde eingeengt und aus tert.-Butylmethylether/Petrolether kristallisiert. Ausbeute: 11,3 g.

Beispiel 55:

L-Phenylalanyl-N-(3-(4-hydroxy-3,5-di-tert.-butyl-phenyl)-isoxazol-5-ylmethyl)-amid-Hydrochlorid

a) Acylierung mit N-tert.-Butoxycarbonyl-phenylalanin
5,3 g (0,02 mol) N-tert.-Butoxycarbonyl-phenylalanin wurden in 100 ml wasserfreiem Tetrahydrofuran gelöst, 4,5 g (0,022 mol) Dicyclohexylcarbodiimid und 3,1 g (0,02 mol) 1-Hydroxybenzotriazol-Hydrat wurden zugesetzt und etwa 45 min ge-rührt. Der ausgefallene Harnstoff wurde abfiltriert. Nach Zugabe von 2,3 ml (0,018 mmol) N-Ethylmorpholin wurden 6,25 g (0,015 mol) des Produktes aus Beispiel 48, gelöst in 50 ml Tetrahydrofuran, zugetropft. Nach weiteren 4 h wurde durch Zugabe von Ethylacetat, Waschen mit 0,1 N HCl und gesättigter NaCl-Lösung, Trocknen und Einengen aufgearbeitet. Das Rohprodukt wurde durch Chromatographie an Kieselgel (tert.-Butylmethylether) weiter gereinigt. Ausbeute: 8,9 g des N-tert,-Butoxycarbonyl-geschützten Produktes als Öl.

b) Abspaltung der Schutzgruppe

Die Abspaltung der Schutzgruppe und Überführung in das Hydrochlorid erfolgte durch Behandeln mit Trifluoressigsäure (25 ml) in Dichlormethan (150 ml), Einengen und mehrfaches Eindampfen nach Zugabe von etherischer Salzsäure, danach Ausrühren mit Petrolether. Ausbeute: 6,3 g an amorphem Hydrochlorid.

Beispiel 56:

(3-(3-(4-Hydroxy-3,5-di-tert.-butyl-phenyl)-isoxazol-5-ylmethyl)-ureido)-essigsäure

6,5 g (0,015 mol) des Produktes aus Beispiel 54 wurden in Analogie zu Beispiel 3 verseift. Ausbeute: 5,7 g

Beispiel 57:

2-(3-(4-Hydroxy-3,5-di-tert.-butyl-phenyl)-isoxazol-5-yl)-vinylphosphonsäurediethylester

Die Reaktion wurde, ausgehend von 6,4 g (0,021 mol) des Produktes aus Beispiel 47a), 6,25 ml (0,025 mol) Tetraethylmethylendiphosphonat und 9,6 ml (0,024 mol) einer 2,5 m Butyllithiumlösung in Anlehnung an eine Literaturvorschrift (J Med Chem, 32 (1989), 2171) in absolutem Tetrahydrofuran durchgeführt. Das Reaktionsgemisch wurde nach Beendigung der Reaktion in Eiswasser gegossen, mehrfach mit Dichlormethan extrahiert, mit Wasser gewaschen, getrocknet und eingeengt. Das Produkt wurde aus tert.-Butylmethylether/Petrolether kristallisiert. Ausbeute: 7,9 g.

Beispiel 58:

2-(3-(4-Hydroxy-3,5-di-tert.-butyl-phenyl)-isoxazol-5-yl)-vinyl-phosphonsäure

4,0 g (0,009 mol) des Produktes aus Beispiel 57 wurden in 300 ml absolutem Dichlormethan unter Schutzgas bei Raumtemperatur mit 3,9 ml (0,03 mol) Trimethylbromsilan bis zur Vollständigkeit der Reaktion gerührt (DC-Kontrolle). Nach Zugabe von 0,5 ml Wasser wurde eingeengt, mehrfach in Methanol aufgenommen und unter vermindertem Druck eingeengt. Das Öl wurde mit tert.-Butylmethylether gerührt, bis ein kristalliner Rückstand verblieb. Ausbeute: 3,0 g.

Beispiel 59:

3-(4-Hydroxy-3,5-di-tert.-butyl-phenyl)-5-acetyl-isoxazol

5,0 g (0,016 mol) des Produktes aus Beispiel 28 wurden in Analogie zu Beispiel 47a) einer Swern-Oxidation unterzogen. Ausbeute: 3,88 g.

Beispiel 60:

3-(4-Pyridyl)-5-(4-hydroxy-3,5-di-tert.-butyl-phenyl)-2-isoxazolin

a) 2,6-di-tert.-butyl-4-vinylphenol

Die Synthese des Olefins ist bekannt: (P. Grosso, O. Vogl: J. Macromol. Sci.-Chem., A23 (1986), 1041-1056).

b) Hydroxamoylchlorid als Nitriloxid-Vorstufe

4-Pyridylhydroxamoylchlorid-hydrochlorid wurde in Analogie zu einer Literaturvorschrift (Bull Soc Chim France (1962), 2215) durch Chlorierung von Pyridin-4-carbaldoxim hergestellt.

c) Cycloaddition

11,6 g (0,05 mol) des Produktes von a) und 9,7 g (0,05 mol) des Produktes von Beispiel 60b) wurden in 500 ml Dichlormethan vorgelegt, dann wurde in 6 Stunden eine Lösung von 20,9 ml (0,15 mol) Triethylamin in 200 ml Dichlormethan zugetropft. Die Aufarbeitung erfolgte wie in Beispiel 1. Ausbeute: 13,1 g.

Beispiel 61:

3-(2-Thiazolyl)-5-(4-hydroxy-3,5-di-tert.-butyl-phenyl)-2-isoxazolin

5 g (0,039 mol) 2-Thiazolyl-carbaldoxim (Herstellung: A. Dondoni, Synthesis (1987), 998-1001) wurden wie in Beispiel 1 beschrieben, mittels tert.-Butylhypochlorit in Dichlormethan/Dimethylformamid (1:1) chloriert, dann wurden 16,3 g (0,07 mol) des Olefines aus Beispiel 60a) zugegeben und gemäß Beispiel 1 cycloaddiert. Das verbleibende Öl wurde durch Chromatographie an Kieselgel mit tert.-Butylmethylether/Petrolether 1:2 gereinigt und aus Methanol/wenig Wasser kristallisiert. Ausbeute: 6,4 g.

Beispiel 62:

3-Carboxy-5-(4-hydroxy-3,5-di-tert.-butyl-phenyl)-2-isoxazolin

a) Hydroxamoylchlorid als Nitriloxid-Vorstufe
Chloroxyiminoessigsäureethylester wurde, aus Glycinethylester nach einer Literaturvorschrift (G S Skinner, J Am Chem Soc, 46 (1924), 731) hergestellt.
b) Cycloaddition mit Ethoxycarbonylnitriloxid
4,55 g (0,03 mol) des Produktes aus Beispiel 62a), gelöst in 80 ml Dichlormethan, wurden in 6 Stunden zu einer Lösung von 4,65 g (0,02 mol) des Olefins aus Beispiel 60a) und 5,6 ml (0,04 mol) Triethylamin in 100 ml Dichlormethan getropft. Die Aufarbeitung erfolgte gemäß Beispiel 1. Ausbeute: 4,8 g.
c) Verseifung des Ethylesters
Die Verseifung und Aufarbeitung erfolgte in Analogie zu Beispiel 3. Es wurde 3,55 g kristalline Carbonsäure aus 6,0 g (0,017 mol) des Produktes aus Beispiel 62b) erhalten.

Für den Cycloadditionsschritt der nachfolgenden Beispiele 63 und 65 wurden Nitrobuttersäurebausteine in einer Variante der Methode von Mukaiyama (J Am Chem Soc, 82 (1960), 5339-5342) mittels Isocyanaten dehydratisiert. Der Syntheseweg, ausgehend von Acrylsäurederivaten und Nitromethan ist stellvertretend für die nicht kommerziell erhältlichen Derivate im folgenden für den Nitrobuttersäure-tert.-butylester beschrieben. Durch Verwendung entsprechend substituierter Vinylphosphonsäure- und Virylphosphinsäureester ist diese Methode auch auf 4-Nitropropylphosphinsäurederivate und -phosphonsäurederivate übertragbar.

Beispiel 63:

5-(4-Hydroxy-3,5-di-tert.-butyl-phenyl)-2-isoxazolin-3-ylpropionsäure

a) Nitrobuttersäure-tert.-butylester
537 ml (3,7 mol) Acrylsäure-tert.-butylester wurden bei einer Badtemperatur von 70 °C zu einer vorgelegten Lösung aus 2,0 l Nitromethan und 7 ml 1,8-Diazabicyclo-[5.4.0]undec-7-en (DBU) getropft, wobei man den durch Spuren an Acrylsäure möglicherweise abfallenden pH-Wert durch Zugabe entsprechender Mengen an DBU konstant hielt. Nach Abklingen der exothermen Reaktion (Temperaturanstieg bis 90 °C) ließ man 60 min abkühlen, wusch mehrmals mit verdünnter wässriger Salzsäure und Wasser, trocknete, und engte unter vermindertem Druck ein, wobei 660 g eines rötlichbraunen Öles verblieben, das durch Destillation weiter gereinigt wurde ($Kp_5$: 90 °C).
Auf gleichem Weg wurden die nachfolgenden Nitroverbindungen hergestellt: 3-Nitropropyl-phosphonsäuredimethylester und -diethylester (ausgehend von Vinylphosphonsäureestern)
3-Nitropropyl-P-Methyl-phosphinsäureethylester (ausgehend von Vinyl-P-methylphosphinsäureethylester).
b) Cycloaddition
7,0 g (0,03 mol) des Olefins aus Beispiel 60a) wurden mit 0,5 ml Triethylamin und 6,4 g (0,04 mol) Phenylendiisocyanat in 80 ml Toluol bei 50 °C vorgelegt. 7,6 g (0,04 mol) des unter a) beschriebenen 4-Nitrobuttersäure-tert.-butylester mit 0,2 ml Triethylamin wurden in 80 ml Toluol gelöst und in 5 Stunden zugetropft. Es wurde über Nacht bei Raumtemperatur gerührt, ausgefallener Harnstoff abgesaugt und mit Dichlormethan gewaschen. Nach dem Einengen verblieb ein öliges Rohprodukt, das durch Chromatographie an Kieselgel (Laufmittel: Petrolether/tert.-Butylmethylether-Gemisch) weiter gereinigt wurde.
c) Abspaltung der Carboxyl-Schutzgruppe

5 g (0,012 mol) des Produktes von b) wurden in 80 ml Dichlormethan bei Raumtemperatur mit 20 ml Trifluoressigsäure bis zur Beendingung der Reaktion (etwa 3 Stunden) behandelt. Nach Einengen wurde mit tert.-Butylmethylether ausgerührt, wobei 3,6 g eines kristallinen Produktes erhalten wurden.

Beispiel 64:

5-(4-Hydroxy-3,5-di-tert.-butyl)-phenyl-2-isoxazolin-3-propionsäure-N-(1H-tetrazol-5-yl)-amid

5,0 g (0,15 mol) des Produktes aus Beispiel 63 wurden gemäß Beispiel 38 mit Siliciumtetrachlorid und 5-Aminotetrazol in absoluten Pyridin derivatisiert. Das Produkt wurde aus tert.-Butylmethylether/Petrolether kristallisiert. Ausbeute: 3,5 g.

Beispiel 65:

(2-(5-(4-Hydroxy-3,5-di-tert.-butyl-phenyl)-2-isoxazolin-3-yl)-ethyl)-methylphosphinsäure

Die Cycloaddition erfolgte ausgehend von 3-Nitropropyl-P-Methylphosphinsäureethylester gemäß Beispiel 63. Die Spaltung der Phosphinestergruppe wurde gemäß Beispiel 7 alkalisch durchgeführt.

Beispiel 70:

L-Phenylalanin-1-(3-(4-hydroxy-3,5-di-tert.-butyl-phenyl)-isoxazol-5-yl)-ethylester-Hydrochlorid

a) Acylierung mit N-tert.-Butoxycarbonyl-phenylalanin
2,65 g (0,01 mol) N-tert.-Butoxycarbonyl-phenylalanin wurden in 50 ml wasserfreiem Tetrahydrofuran gelöst, dann wurden 1,53 g (0,01 mol) 1-Hydroxybenzotriazol-Hydrat und 2,27 g (0,011 mol) Dicyclohexylcarbodiimid zugesetzt und etwa 45 min gerührt. Nach Zugabe von 0,12 g 4-Dimethylaminopyridin wurden 3,17 g (0,01 mol) des Produktes aus Beispiel 28, gelöst in 25 ml Tetrahydrofuran, zugetropft. Nach weiteren 2 h wurde wie folgt aufgearbeitet: Zugabe von Ethylacetat, Waschen mit 0,1 N NaOH, 0,1 N HCl und gesättigter NaCl-Lösung, Trocknen und Einengen. Das Rohprodukt wurde durch Chromato-graphie an Kieselgel (Petrolether/tert.-Butylmethylether 5:1) weiter gereinigt. Ausbeute: 3,15 g des N-tert.-Butoxycarbonyl-geschützten Produktes.

b) Abspaltung der Schutzgruppe
Die Abspaltung der Schutzgruppe und Überführung in das Hydrochlorid erfolgte durch Behandeln mit Trifluoressigsäure (10 ml) in Dichlormethan (50 ml), Einengen und mehrfaches Eindampfen nach Zugabe von etherischer HCl, danach Ausrühren mit tert.-Butylmethylether/Petrolether. Ausbeute: 2,2 g.

Beispiele 71 und 72

L-Phenylalanin-1-(3-(4-hydroxy-3,5-di-tert.-butyl-phenyl)-isoxazol-5-yl)-ethylester-Hydrochlorid

a) Abspaltung der Schutzgruppe
3,0 g des Produktes aus Beispiel 70a) wurden mit Trifluoressigsäre behandelt (analog zu Beispiel 70b). Ausbeute: 2,8 g an Rohprodukt.
b) Trennung der Isomeren
Das Rohprodukt wurde durch mehrfache Chromatographie an Kieselgel mit Dichlormethan/tert.-Butylmethylether 1:10 unter Zusatz von 1% Triethylamin in die Diastereomeren aufgetrennt. Ausbeute: jeweils 0,7 - 0,8 g öliges Produkt, im folgenden mit Isomer A (höherer Rf-Wert) und B (geringerer Rf-Wert) bezeichnet. Die durch HPLC bestimmte Reinheit betrug jeweils mehr als 95 %. Die Überführung ins Hydrochlorid erfolgte für beide Isomere, wie in Beispiel 70b) beschrieben. Die beiden Isomeren konnten jeweils aus Dichlormethan/tert.-Butylmethylether kristallisiert werden.

Beispiel 71 (Isomer A): $[a]^D_{20}$ = +56,1° (c=1 in Ethanol)
Beispiel 72 (Isomer B): $[a]^D_{20}$ = -30,8° (c=1 in Ethanol)

Beispiel 73:

L-Phenylalanin-1-(3-(4-hydroxy-3,5-di-tert.-butyl-phenyl)-2-isoxazolin-5-yl)-ethylester-Hydrochlorid

a) Acylierung mit N-tert.-Butoxycarbonyl-phenylalanin
Die Synthese erfolgte ausgehend von 3,2 g (0,01 mol) des Produktes aus Beispiel 19 (rac. erythro-Isomer) gemäß Beispiel 70a).
Ausbeute: 3,5 g des N-tert.-Butoxycarbonyl-geschützten Produktes.
b) Abspaltung der Schutzgruppe
Die Spaltung der Schutzgruppe wurde analog zu Beispiel 70b) durchgeführt.
Ausbeute: 2,6 g des Isomerengemisches (bedingt durch den Einsatz racemischen Isoxazolins).

Beispiele 74 und 75:

L-Phenylalanin-1-(3-(4-hydroxy-3,5-di-tert.-butyl-phenyl)-isoxazolin-5-yl)-ethylester-Hydrochlorid

a) Trennung der Isomeren
2,5 g des Produktes aus Beispiel 73a) wurden durch mehrfache Chromato-graphie an Kieselgel mit Petrolether/tert.-Butylmethylether 5:1 in die Diastereomeren aufgetrennt. Ausbeute: jeweils 0,6 - 0,7 g öliges Produkt, im folgenden mit Isomer A (höherer Rf-Wert) und B (geringerer Rf-Wert) bezeichnet. Die durch HPLC bestimmte Reinheit betrug jeweils mehr als 95 %.
b) Abspaltung der Schutzgruppe
Die Abspaltung der Schutzgruppe und die Überführung ins Hydrochlorid erfolgte für beide Isomere, wie in Beisp. 70b) beschrieben.

Beispiel 74 (Isomer A): $[a]^{D}_{20}$ = +31,6° (c=1 in Ethanol)
Beispiel 75 (Isomer B): $[a]^{D}_{20}$ = -59,7° (c=1 in Ethanol)

Beispiel 76:

Glycin-1-(3-(4-hydroxy-3,5-di-tert.-butyl-phenyl)-isoxazol-5-yl)-ethylester-Hyd rochlorid

Die Synthese erfolgte ausgehend von 2,1 g (0,012 mol) N-BOC-Glycin und 3,2 g (0,01 mol) des Produktes aus Beispiel 28 gemäß Beispiel 70. Nach Abspaltung der Schutzgruppe und Überführung ins Hydrochlorid wurde aus tert.-Butyl-methylether/Petrolether ein amorphes Festprodukt erhalten.
Ausbeute: 2,6 g

Beispiel 77:

Glycin-1-(3-(4-hydroxy-3,5-di-tert.-butyl-phenyl)-2-isoxazolin-5-yl)-ethylester-Hydrochlorid

Die Synthese erfolgte ausgehend von 2,1 g (0,012 mol) N-BOC-Glycin und 3,2 g (0,01 mol) des Produktes aus Beispiel 19 (rac. erythro-Isomer) gemäß Beispiel 70. Nach Abspaltung der Schutzgruppe und Überführung ins Hydrochlorid wurde aus tert.-Butylmethylether/Petrolether ein amorphes Festprodukt erhalten. Ausbeute: 2,3 g.

Beispiel 81:

Hexadecansäure-1-(3-(4-Hydroxy-3,5-di-tert.-butyl-phenyl)-2-isoxazolin-5-yl)-ethylester (erythro-Isomer)

3,2 g (0,01 mol) des Produktes aus Beispiel 19 wurden in 60 ml Tetrahydrofuran gelöst. Nach Zugabe von 125 mg 4-Dimethylaminopyridin und 3,05 ml (0,024 mol) N-Ethylmorpholin wurde unter Eiskühlung 5,5 g (0,02 mol) Hexadecansäurechlorid, gelöst in 30 ml Tetrahydrofuran zugetropft, und nach Beendigung der Reaktion (DC-Kontrolle) mit Wasser, wässriger Kaliumhydrogensulfatlösung und Wasser gewaschen, getrocknet und eingeengt. Das verbliebene Öl wurde durch Chromatographie an Kieselgel mit Petrolether/tert.-Butylmethylether gereinigt. Ausbeute: 1.9 g

Beispiel 82:

Di-O-acetyl-L-Weinsäure-N-(3-(4-Hydroxy-3,5-di-tert.-butyl-phenyl)-isoxazol-5-yl)-methyl-amid

4,2 g (0,01 mol) des Produktes aus Beispiel 48 wurden zusammen mit 0,12 g 4-Dimethylaminopyridin und 4,5 ml (0,035 mol) N-Ethylmorpholin in 250 ml Tetrahydrofuran vorgelegt und 3,3 g (0,015 mol) (+)-Di-O-acetyl-L-weinsäureanhydrid, gelöst in 60 ml Tetrahydrofuran, wurden in 0,5 Stunden bei Raumtemperatur unter Rühren zugetropft. Nach weiteren 4 h bei Raumtemperatur wurden 50 ml 1N HCl, 500 ml Wasser und 500 ml Ethylacetat zugesetzt, nach Phasentrennung mit Wasser und gesättigter NaCl-Lösung gewaschen, getrocknet und eingeengt. Der Rückstand wurde an Kieselgel mit tert.-Butylmethylether chromatographiert und anschließend aus tert.-Butylmethylether/ Petrolether kristallisiert. Ausbeute: 4,9 g: $[a]^D_{20}$ = -1,4° (c=1 in Ethanol)

Beispiel 83:

L-Weinsäure-N- (3-(4-Hydroxy-3,5-di-tert.-butyl-phenyl)-isoxazol-5-yl)-methyl-amid

1,56 g (0,03 mol) des Produktes aus Beispiel 82 wurden in 200 ml Methanol gelöst und 0,84 g (0,06 mol) fein gemahlenes Kaliumcarbonat zugesetzt. Es wurde über Nacht gerührt, mit wässr. Zitronensäure angesäuert und mit Ethylacetat mehrfach extrahiert. Das nach Trocknen und Eindampfen verbleibende Öl wurde kristallisiert.
Ausbeute: 1,25 g: $[\alpha]^{20}_D$ = +21,5° (c=1 in Ethanol)

Beispiel 84:

3-Oxo-buttersäure-1-(3-(4-hydroxy-3,5-di-tert.-butyl-phenyl)-2-isoxazolin-5-yl)-ethylester

1,6 g des Produktes aus Beispiel 19 wurden zusammen mit ca. 15 mg 4-Dimethylaminopyridin in 80 ml abs. Tetrahydrofuran unter Eiskühlung gelöst und tropfenweise 0,46 ml (0,006 mol) Diketen, gelöst in 15 ml Tetrahydrofuran zugesetzt. Nach 1 h wurde das Eisbad entfernt und über Nacht bei Raumtemperatur gerührt. Nach Einengen wurde der Rückstand mit Wasser/Ethylacetat aufgenommen, die organische Phase getrocknet und eingeengt. Danach wurde aus tert.-Butylmethylether/Petrolether kristallisiert. Ausbeute: 1,05 g hellgelber Kristalle

Beispiel 85:

Phosphorsäure-diphenyl-1-(3-(4-hydroxy-3,5-di-tert.-butyl-phenyl)-2-isoxazolin-5-yl)-ethylester

3,2 g (0,01 mol) des Produktes aus Beisp.19 wurden zusammen mit 0,12 g 4-Dimethylaminopyridin und 1,4 ml (11 mmol) N-Ethylmorpholin in 60 ml abs. Tetrahydrofuran gelöst, und bei Raumtemp. wurden 2,95 g (11 mmol) Phosphorsäurediphenylesterchlorid, gelöst in 10 ml Tethrahydrofuran zugetropft. Nach 4 h wurde mit wässr. Zitronensäure und Ethylacetat versetzt, die org. Phase getrocknet, eingeengt und der Rückstand kristallisiert.
Ausbeute: 4,7 g kristallines Produkt

Beispiel 86 und 87

4-(4-Morpholino).methyl-benzoesäure-1-(3-(4-hydroxy-3,5-di-tert.-butyl-phenyl)-2-isoxazolin-5-yl)-ethylester-Hydrochlorid,

86: erythro und 87: threo-Isomer

a) 4-Chlormethyl-benzoyl-but-3-en-2-yl-ester
7,2 g (0,1 mol) rac. 1-Buten-3-ol werden zusammen mit 20,5 g (0,108 mol) 4-Chlormethyl-benzoylchlorid,14 g (0,11 mol) N-Ethylmorpholin und 1,2 g 4-Dimethylaminopyridin in 200 ml abs.Tetrahydrofuran für 24 h gerührt. Nach Zugabe von Dichlormethan und wässr. Zitronensäure wurden die Phasen getrennt, die org. Phase mit Wasser gewaschen, getrocknet und eingeengt. Es wurde mit Petrolether digeriert und von geringen Anteilen eines ausgefallenen Feststoffes abfiltriert. Das Produkt konnte nach Einengen als Öl isoliert werden.

b) Die Umsetzung erfolgte analog zu Beispiel 1), ausgehend von 7,5 g (0,03 mol) Aldoxim und 7,6 g (0,034 mol) des Produktes aus Beispiel 86a). Das zunächst ölig anfallende Rohprodukt wurde durch Chromatografie an Kieselgel (Petrolether/Ethylacetat 9:1) weiter gereinigt.

Ausbeute:3,7 g an rac. erythro/threo-Isomerengemisch

c) Umsetzung mit Morpholin
3,6 g (7,6 mmol) des aus b) angefallenen Produktes wurden in Aceton gelöst, 1,3 ml (0,015 mol) Morpholin und 0,27 g (1,6 mmol) Kaliumjodid zugesetzt und 2 Tage bei Raumtemp. gerührt. Nach Einengen wurde mit Wasser/ Ethylacetat aufgenommen, die organische Phase mit Wasser gewaschen, getrocknet und eingeengt. Es verblieben 4 g des Produktes als Diastereomerengemisch (Isomerenverhältnis ca. 60:40).

d) Isomerentrennung
Das Rohprodukt aus c) wurde in ca. 200 ml tert.-Butylmethylether gelöst, und rnit 8 ml einer 5-molaren ethanolischen Salzsäure, sowie ca. 30 ml Dichlormethan versetzt. Das ausgefallene Hydrochlorid wurde isoliert, und aus Isopropanol umkristallisiert, wobei das erythro-Diastereoisomer zunächst auskristallisierte und durch weitere Kristallisationen aus Isopropanol bis zu einem Diastereoisornerenverhältnis >95:5 angereichert werden konnte.
Ausbeute: 0,75 g.
Aus den gesammelten Mutterlaugen konnte ebenfalls durch mehrfache Kristallisation das threo-Diastereoisomer isoliert werden.

Beispiel 88:

L-Tyrosyl-1 <3(3,5-di-tert.-butyl-4-hydroxy-phenyl)-2-isoxazolin-5-yl> ethylester rac.5,5'-erythro-Isomerengemisch

a) Acylierung mit N,O-Di-tert.-Butoxycarbonyl-L-tyrosin
7,6 g (0,02 mol) N,O-Di-tert.-Butoxycarbonyl-L-tyrosin wurden in 100 ml wasserfreiem Tetrahydrofuran gelöst, 2,7 g (0,02 mol) 1 -Hydroxybenzotriazol und 4,5 g (0,022 mol) Dicyclohexylcarbodiimid zugesetzt, ca. 45 min nachgerührt, und vom ausgefallenen Harnstoff abfiltriert. Nach Zugabe von 2,3 ml (0,018 mol) N-Ethylmorpholin und 0,24 g (0,002 mol)
Dimethylaminopyridin wurden 4,8 g (0,015 mol) des Produktes aus Beispiel 19, gelöst in 80 ml Tetrahydrofuran, zugetropft. Nach 24 h Rühren bei Raumtemperatur wurde durch Zugabe von Ethylacetat, Waschen mit 0,1 N NaOH und 0,1 N HCl und gesättigter NaCl-Lösung, Trocknen und Einengen aufgearbeitet. Das Rohprodukt wurde durch Chromatografie an Kieselgel (tert.-Butylmethylether/Petrolether) weiter gereinigt.
Ausbeute: 10,5 g des N-tert,-Butoxycarbonyl-geschützten Produktes als Öl.

b) Abspaltung der Schutzgruppe
Die Abspaltung der Schutzgruppe erfolgte analog zu Beispiel 70b). Nach Freisetzuung der Base wurde durch Chromatografie an Kieselgel (tert.-Butylmethylether/Dichlormethan/Methanol 10:9:1) weiter gereinigt. Ausbeute: 1,6 g eines amorphen Feststoffes

Beispiel 89:

3-(3,5-Di-tert.-butyl-4-hydroxy-phenyl)-5-(1,2-dihydroxy-ethyl)-2-isoxazolin rac. erythro-Isomer

2,9 g des Produktes aus Beisp. 16 wurden in 50 ml Dioxan und 10 ml Wasser gelöst, 0,75 ml 70%-ige wässr. Perchlorsäure zugesetzt und 18 h bei Raumtemp. gerührt. Nach Zugabe von 20 ml wässr. 5%-iger Natriumcarbonat. Lösung und 150 ml Wasser wurde mehrfach mit Ethylacetat extrahiert, die organische Phase gewaschen, getrocknet und eingeengt. Das Produkt konnte durch Kristallisation aus tert.-Butylmethylether/Petrolether in reiner Form erhalten werden.
Ausbeute: 1,3 g

Beispiel 90

3-(3, 5-Di-tert.-butyl-4-hydroxy-phenyl )-5-(1,2-dihydroxy-ethyl)-2-isoxazolin rac. threo-Isomer

Die Durchführung erfolgte analog zu Beisp. 89 ausgehend von 2,4 g Produktes aus Beisp. 17.
Ausbeute: 1,25 g an kristallinem Produkt.

Beispiel 91

4-(4-Methyl-piperazin-1-yl-methyl-benzoesäure-1-(3-(4-hydroxy-3,5-di-tert.-butyl-phenyl)-2-isoxazolin-5-yl)-ethylester Dihydrobromid erythro-Isomer

Die Durchführung erfolgte analog zu Beisp. 86. Eine Anreicherung der Diastereoisomeren wurde auf Stufe b) durch fraktionierte Kristallisation durchgeführt. Im Schritt c) wurde N-Methyl-piperazin anstelle von Morpholin verwendet. Nach Fällung der Hydrobromide betrug die Isomerenanreicherung auf Stufe d) > 95 %.
Ausbeute: 1,9 g an eryhtro-Produkt, ausgehend von 4,7 g der Stufe b).

**Tab. 1**

| Beispiel | Struktur | Solvens | $^1$H-NMR | Schmp. (°C) |
|---|---|---|---|---|
| 1 | 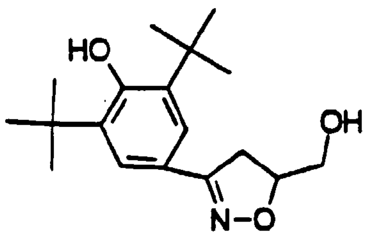 | CDCl3 | 1.45 (s, 18H)<br>2.05 (tb, 1H)<br>3.16-3.45 (m, 2H)<br>3.55-3.95 (m, 2H)<br>4.83 (m, 1H)<br>5.49 (s, 1H)<br>7.5 (s, 2H) | 132-135 |
| 2 | 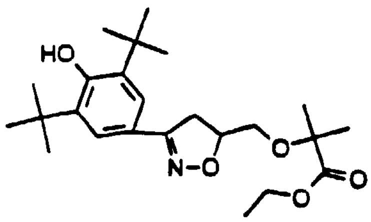 | CDCl3 | 1.26 (t, 3H)<br>1.43-1-46 (sb, 24 H)<br>3.3-3.68 (m, 4H)<br>4.18 (q, 2H)<br>4.86 (m, 1H)<br>5.49 (s, 1H)<br>7.52 (s, 2H) | 86-88 |
| 3 | 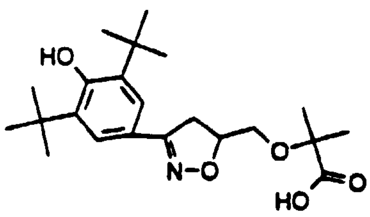 | CDCl3 | 1.46 (s, 18H)<br>1.48 u.1.50 (jew. s, 3H)<br>3.15-3.52 (m, 2H)<br>3.61 (d, 2H)<br>4.89 (m, 1H)<br>5.50 (s, 1H)<br>7.50 (s, 2H) | 183-186 |
| 4 | 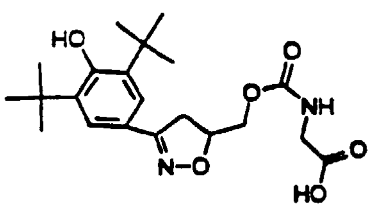 | CDCl3 | 1.39 (s, 18H)<br>3.05-3.70 (m, 4H)<br>3.95-4.19 (m, 2H)<br>4.82 (m, 1H)<br>7.40 (s, 2H)<br>7.58 (tb, 1H) | 124-132 |
| 5 | 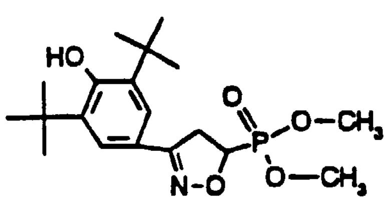 | CDCl3 | 1.45 (s, 18H)<br>3.58-3.78 (m, 2H)<br>3.82-3.95 (2d, je 3H)<br>4.88 (m, 1H)<br>5.52 (sb, 2H)<br>7.50 (s, 2H) | 196 |

| | | | | |
|---|---|---|---|---|
| 6 | 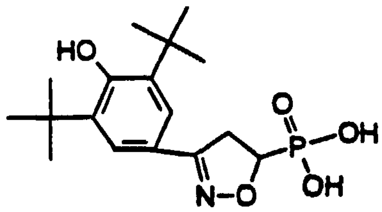 | DMSO-d6 | 1.39 (s, 18H)<br>3.25-3.85 (m, 2H)<br>4.62 (m, 1H)<br>7.39 (s, 2H)<br>11.0 (sb, 2H) | 214<br>(Zers.) |
| 7 | 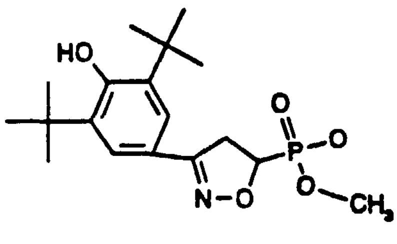 | DMSO-d6 | 1.39 (s, 18H)<br>3.25-3.95 (m, 5H, incl d, 3H bei 3.65)<br>4.76 (m, 1H)<br>7.40 (s, 2H)<br>7.48 (sb, 1H) | 183-186<br>(Zers.) |
| 8 | 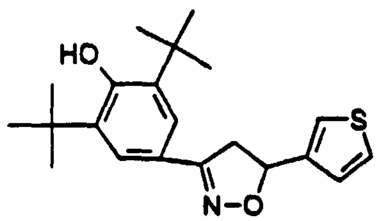 | DMSO-d6 | 1.39 (s, 18H)<br>3.34-3.86 (m, 2H)<br>5.70 (m, 1H)<br>7.13 (m, 1H)<br>7.40-7.58 (m, 4H) | 121-125 |
| 9 | 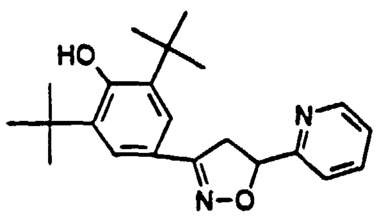 | DMSO-d6 | 1.39 (s, 18H)<br>3.59-3.94 (m, 2H)<br>5.72 (m, 1H)<br>7.30-7.55 (m, 5H)<br>7.82 (m, 1H)<br>8.57 (m, 1H) | 149-150 |
| 10 | 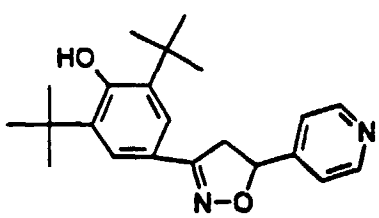 | DMSO-d6 | 1.39 (s, 18H)<br>3.29-3.44 u. 3.82-4.0 (jew. m, 1H)<br>5.73 (m, 1H)<br>7.35-7.47 (m, 5H)<br>8.57 (m, 2H) | 179-180 |
| 11 | 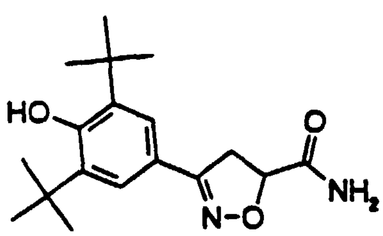 | DMSO-d6 | 1.39 (s, 18H)<br>3.40-3.75 (m, 2H)<br>4.99 (m, 1H)<br>7.41 (s,2H)<br>7.44 (sb, 1H)<br>7.60 (sb, 1H) | 258-262 |

| | | | | |
|---|---|---|---|---|
| 12 | 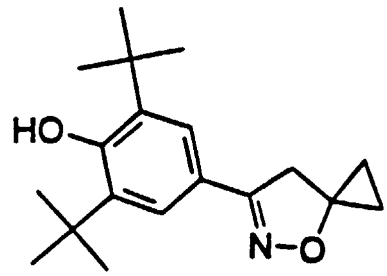 | CDCl3 | 0.81 (m, 2H)<br>1.21 (m, 2H)<br>1.46 (s, 18H)<br>3.41 (s, 2H)<br>5.47 (s, 1H)<br>7.50 (s, 2H) | 149-151 |
| 13 | 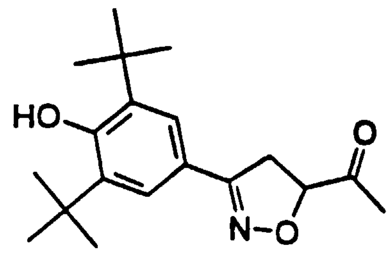 | CDCl3 | 1.40 (s, 18H)<br>2.31 (s, 3H)<br>3.35-3.64 (m, 2H)<br>4.94 (m, 1H)<br>5.47 (s, 1H)<br>7.45 (s, 2H) | 94 |
| 14 | 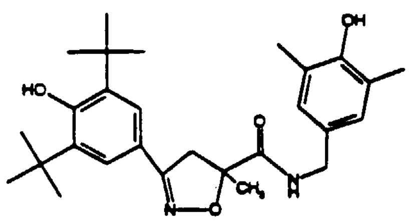 | CDCl3 | 1.44 (s, 18H)<br>1.74 (s, 3H)<br>2.18 (s, 6H)<br>3.24 (d, 1H)<br>3.88 (d, 1H)<br>4.14-4.43 (m, 2H)<br>4.70 (s, 1H)<br>5.52 (s, 1H)<br>6.84 (s, 2H)<br>7.13 (tb, 1H)<br>7.47 (s, 2H) | 194 |
| 15 | 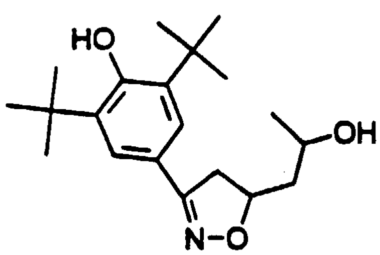 | CDCl3 | 1.34-1.28 (2d, 3H)<br>1.45 (s, 18H)<br>1.58-2.04 (m, 3H)<br>2.93-3.10 u. 3.38-3.58 (jew. m, 1H)<br>4.12 u. 4.90 (jew. m, 1H)<br>5.47 u. 5.48 (jew. s, 0.5H)<br>7.49 u. 7.50 (jew. s, 1H) | 131-133 |

| | | | | |
|---|---|---|---|---|
| 16 | 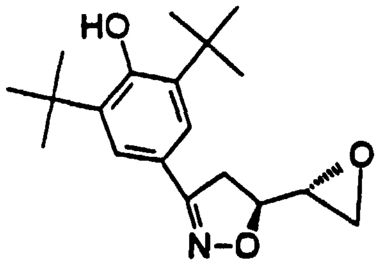 | CDCl3 | 1.46 (s, 18H)<br>2.72, 2.89 u. 3.16 (jew. m, 1H)<br>3.21-3.52 (m, 2H)<br>4.61 (m, 1H)<br>5.50 (s, 1H)<br>7.51 (s, 2H) | 148-153 |
| 17 | 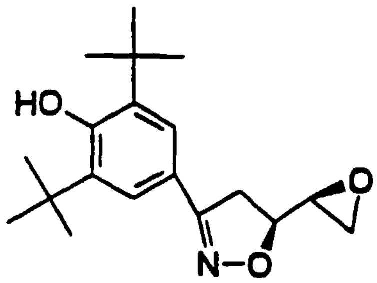 | CDCl3 | 1.45 (s, 18H)<br>2.84 (d, 2H)<br>3.18-3.57 (m, 3H)<br>4.73 (m, 1H)<br>5.48 (s, 1H)<br>7.49 (s, 2H) | 123-126 |
| 18 | 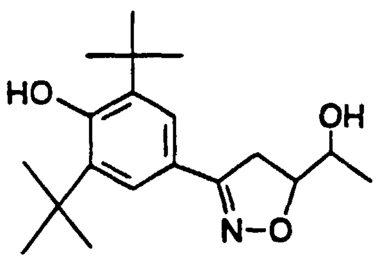 | DMSO-d6 | 1.06-1.11 (2d, 3H)<br>1.40 (s, 18H)<br>3.08-3.43 (m, 2H)<br>3.52-3.80 (m, 1H)<br>4.31-4.55 (m, 1H)<br>4.81-4.96 (2db, 1H, OH)<br>7.36 (sb, 1H)<br>7.40 u. 7.41 (jew. s, 1H) | 126-146 |
| 19 | 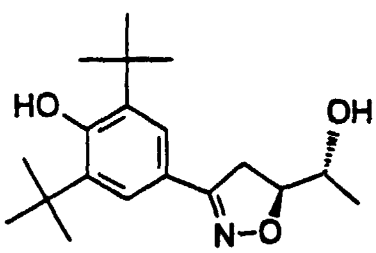 | DMSO-d6 | 1.07 (d, 3H)<br>1.39 (s, 18H)<br>3.15-3.41 (m, 3H)<br>3.62 (m, 1H)<br>4.38 (m, 1H)<br>4.90 (d, 1H)<br>7.38 (s, 2H) | 161 |
| 20 | 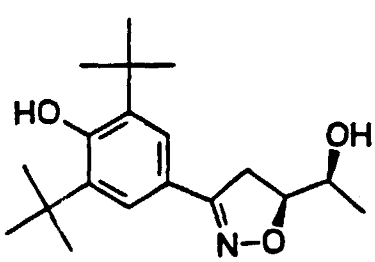 | DMSO-d6 | 1.07 (d, 3H)<br>1.38 (s, 18H)<br>3.05-3.45 (m, 3H)<br>3.66 (m, 1H)<br>4.44 (m, 1H)<br>4.85 (d, 1H)<br>7.38 (s, 2H) | 134-135 |

| | | | | |
|---|---|---|---|---|
| 21 | 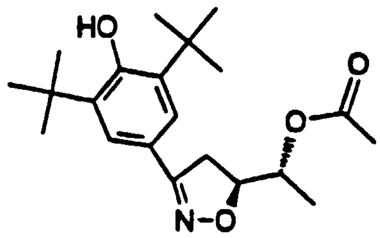 | CDCl3 | 1.30 (d, 3H)<br>1.46 (s, 18H)<br>2.07 (s, 3H)<br>3.05-3.50 (m, 2H)<br>4.72 (m, 1H)<br>5.07 (m, 1H)<br>5.48 (s, 1H)<br>7.49 (s, 2H) | 149-151 |
| 22 | 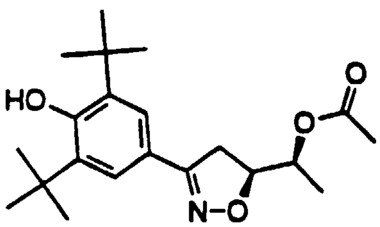 | CDCl3 | 1.32 (d, 3H)<br>1.46 (s, 18H)<br>2.06 (s, 3H)<br>3.02-3.48 (m, 2H)<br>4.73 (m, 1H)<br>5.09 (m, 1H)<br>5.48 (s, 1H)<br>7.49 (s, 2H) | 128-130 |
| 23 | 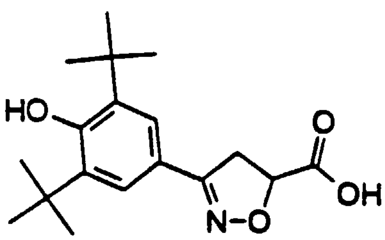 | MeOH-d4 | 1.44 (s, 18H)<br>3.50-3.85 (m, 2H)<br>4.93 (sb, acide H)<br>5.13 (m, 1H)<br>7.50 (s, 2H) | 176-178<br>(Zers.) |
| 24 | 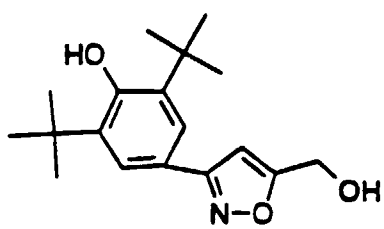 | MeOH-d4 | 1.47 (s, 18H)<br>4.70 (s, 2H)<br>4.88 (sb, acide H)<br>6.67 (s, 1H)<br>7.62 (s, 2H) | 149-155 |
| 25 | 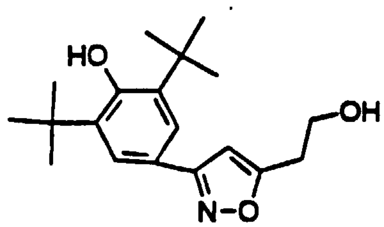 | DMSO-d6 | 1.42 (s, 18H)<br>2.92 (t, 2H)<br>3.75 (m, 2H)<br>4.90 (tb, 1H))<br>6.72 (s, 1H)<br>7.33 (sb, 1H)<br>7.54 (s, 2H) | 125 |

| | | | | |
|---|---|---|---|---|
| 26 | 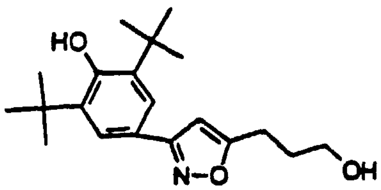 | CDCl3 | 1.48 (s, 18H)<br>2.02 (m, 2H)<br>2.91 (t, 2H)<br>3.75 (t, 2H)<br>5.43 (s, 1H)<br>6.26 (s, 1H)<br>7.59 (s, 2H) | 133-135 |
| 27 | 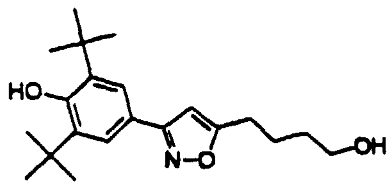 | DMSO-d6 | 1.3-1.59 (m, 20H)<br>1.71 (m, 2H)<br>2.77 (t, 2H)<br>3.41 (m, 2H)<br>4.45 (tb, 1H))<br>6.71 (s, 1H)<br>7.35 (sb, 1H)<br>7.54 (s, 2H) | 82 |
| 28 | 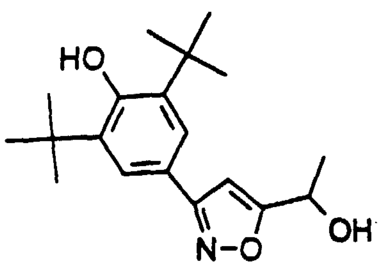 | DMSO-d6 | 1.36-1.53 (s, 18H u. d, 3H)<br>4.89 (m, 1H)<br>5.75 (db, 1H)<br>6.79 (s, 1H)<br>7.38 (sb, 1H)<br>7.56 (s, 2H) | 123-124 |
| 29 | 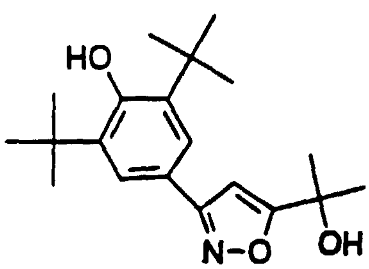 | DMSO-d6 | 1.43 (s, 18H)<br>1.52 (s, 6H)<br>5.62 (sb, 1H)<br>6.74 (s, 1H)<br>7.36 (sb, 1H)<br>7.55 (s, 2H) | 142 |
| 30 | 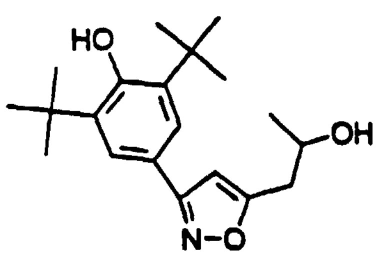 | CDCl3 | 1.32 (d, 3H)<br>1.48 (s, 18H)<br>1.80 (db, 1H)<br>2.95 (d, 2H)<br>4.24 (m, 1H)<br>5.44 (s, 1H)<br>6.36 (s, 1H)<br>7.60 (s, 2H) | 125-132 |

| | | | | |
|---|---|---|---|---|
| 31 | 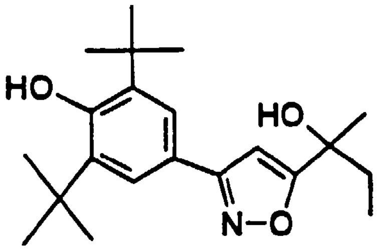 | DMSO-d6 | 0.82 (t, 3H)<br>1.44 (s, 18H)<br>1.48 (s, 3H)<br>1.80 (q, 2H)<br>5.50 (sb, 1H)<br>6.74 (s, 1H)<br>7.35 (sb, 1H)<br>7.57 (s, 2H) | 150-151 |
| 32 | 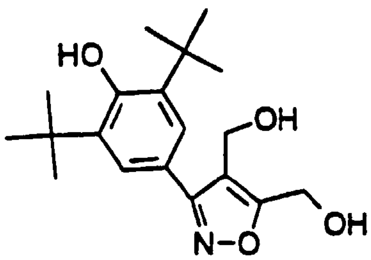 | DMSO-d6 | 1.43 (s, 18H)<br>4.41 u. 4.63 (jew. d, 2H)<br>5.11 u. 5.52 (jew. tb, 1H)<br>7.34 (sb, 1H)<br>7.66 (s, 2H) | 191-195 (Zers.) |
| 33 | 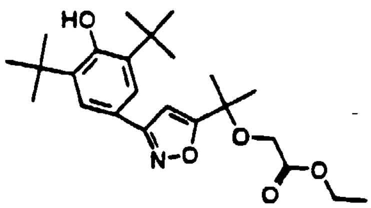 | CDCl3 | 1.33 (t, 3H)<br>1.45 (s, 18H)<br>1.68 (s, 6H)<br>2.19 (sb, 1H)<br>4.32 (q, 2H)<br>4.37 (s, 2H)<br>6.43 (s, 1H)<br>7.67 (s, 2H) | 124 |
| 34 | 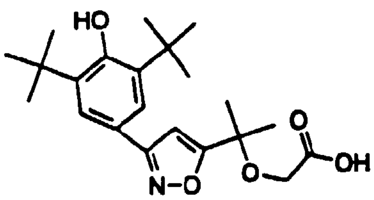 | DMSO-d6 | 1.42 (s, 18H)<br>1.53 (s, 6H)<br>4.30 (s, 2H)<br>5.65 (sb, 1H)<br>6.91 (s, 1H)<br>7.72 (s, 2H)<br>13.1 (sb, 1H) | 209-210 |
| 35 | 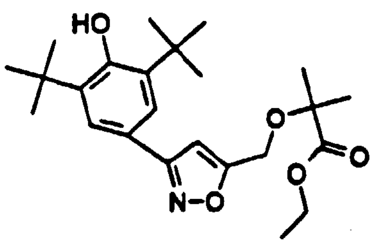 | CDCl3 | 1.30 (t, 3H)<br>1.47 (s, 18H)<br>1.53 (s, 6H)<br>4.23 (q, 2H)<br>4.65 (s, 2H)<br>5.48 (s, 1H)<br>6.54 (s, 1H)<br>7.61 (s, 2H) | 67-68 |

| | | | | |
|---|---|---|---|---|
| 36 | 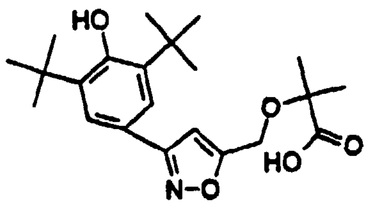 | CDCl3 | 1.48 (s, 18H)<br>1.59 (s, 6H)<br>4.70 (s, 2H)<br>5.45 (sb, 1H)<br>6.54 (s, 1H)<br>7.61 (s, 2H) | 182-185 |
| 37 | 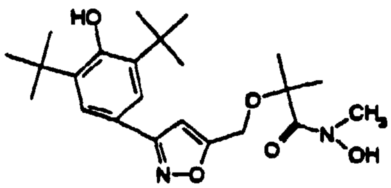 | CDCl3 | 1.48 (s, 18H)<br>1.59 (s, 6H)<br>3.60 (sb, 3H)<br>4.53 (sb, 2H)<br>5.47 (sb, 1H)<br>6.50 (s, 1H)<br>7.60 (s, 2H)<br>8.50 (sb, 1H) | 107-113 |
| 38 | 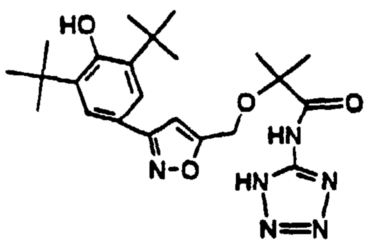 | DMSO-d6 | 1.43 (s, 18H)<br>1.54 (s, 6H)<br>4.69 (sb, 2H)<br>6.99 (s, 1H)<br>7.41 (sb, 1H)<br>7.56 (s, 2H)<br>11.80 (sb, 1H) | 247-249 (Zers.) |
| 39 | 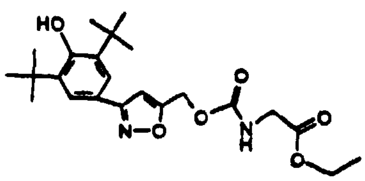 | CDCl3 | 1.28 (t, 3H)<br>1.47 (s, 18H)<br>3.98 (d, 2H)<br>4.21 (q, 2H)<br>5.23 (sb, 2H)<br>5.40 (tb, 1H)<br>5.46 (s, 1H)<br>6.58 (s, 1H)<br>7.60 (s, 2H) | (Öl) |
| 40 | 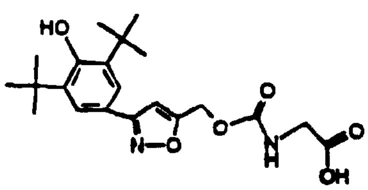 | DMSO-d6 | 1.43 (s, 18H)<br>3.40 (sb, 2H)<br>3.71 (d, 2H)<br>5.20 (sb, 2H)<br>6.99 (s, 1H)<br>7.56 (s, 2H)<br>7.80 (tb, 1H)<br>12.65 (sb, 1H) | 185-188 |

| | | | | |
|---|---|---|---|---|
| 41 | 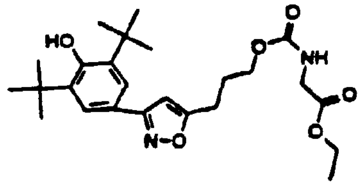 | CDCl3 | 1.29 (t, 3H)<br>1.48 (s, 18H)<br>2.10 (m, 2H)<br>2.88 (t, 2H)<br>3.98 (d, 2H)<br>4.11-4.30 (m, 4H)<br>5.20 (tb, 1H)<br>5.44 (s, 1H)<br>6.27 (s, 1H)<br>7.59 (s, 2H) | 86 |
| 42 | 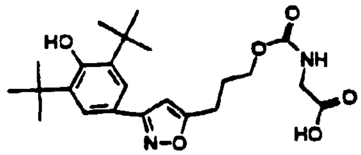 | DMSO-d6 | 1.43 (s, 18H)<br>1.99 (m, 2H)<br>2.82 (tb, 2H)<br>3.67 (d, 2H)<br>4.06 (tb, 2H)<br>6.77 (s, 1H)<br>7.33 (sb, 1H)<br>7.45 (tb, 1H)<br>7.55 (s, 2H)<br>12.55 (sb, 1H) | 201-203 |
| 43 | 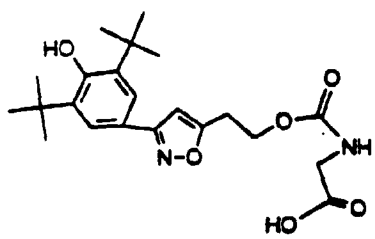 | DMSO-d6 | 1.42 (s, 18H)<br>3.10 (tb, 2H)<br>3.63 (d, 2H)<br>4.29 (tb, 2H)<br>6.80 (s, 1H)<br>7.35 (sb, 1H)<br>7.54 (s, 2H u. tb, 1H)<br>12.55 (sb, 1H) | 152-155 |
| 44 | 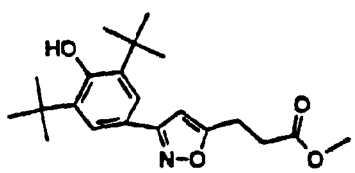 | CDCl3 | 1.48 (s, 18H)<br>2.79 u. 3.12 (jew. t, 2H)<br>3.71 (s, 3H)<br>5.43 (s, 1H))<br>6.28 (s, 1H)<br>7.58 (s, 2H) | 89 |
| 45 | 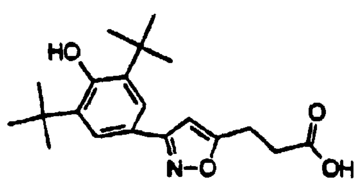 | DMSO-d6 | 1.42 (s, 18H)<br>2.71 u. 2.99 (jew. t, 2H)<br>6.75 (s, 1H)<br>7.35 (sb, 1H)<br>7.54 (s, 2H)<br>12.4 (sb, 1H) | 188 |

| | | | | |
|---|---|---|---|---|
| 46 | 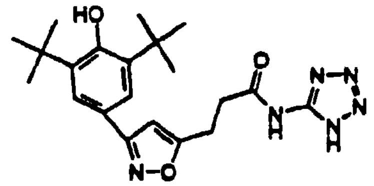 | DMSO-d6 | 1.43 (s, 18H)<br>2.95 u. 3.15 (jew. tb, 2H)<br>6.75 (s, 1H)<br>7.38 (s, 1H)<br>7.54 (s, 2H)<br>12.2 (sb, 1H) | 249 (Zers.) |
| 47 | 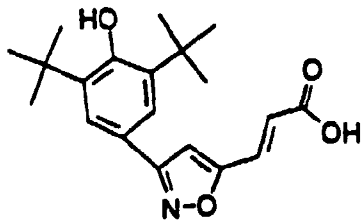 | DMSO-d6 | 1.44 (s, 18H)<br>6.65 u. 7.51 (jew. d, 1H)<br>7.49 (s, 1H)<br>7.60(s, 2H)<br>13.0 (sb, 1H) | 206-208 |
| 48 | 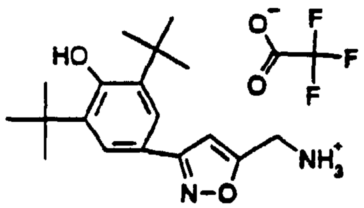 | DMSO-d6 | 1.43 (s, 18H)<br>4.33 (s, 2H)<br>7.06 (s, 1H)<br>7.52 (sb, 1H)<br>7.55 (s, 2H)<br>8.65 (sb, acide H) | 194 |
| 49 | 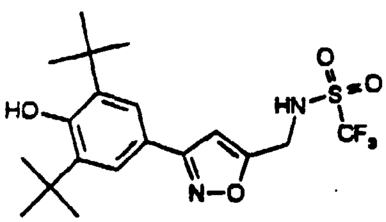 | CDCl3 | 1.48 (s, 18H)<br>4.60 (s, 2H)<br>5.51 (s, 1H)<br>6.55 (s, 1H)<br>7.58 (s, 2H) | 163-165 |
| 50 | 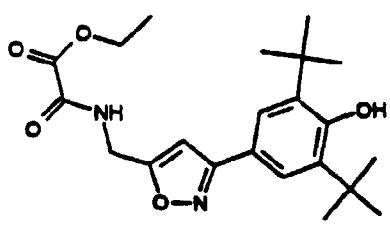 | CDCl3 | 1.35 -1.50 (s, 18H u. t, 3H)<br>4.38 (q, 2H)<br>4.69 (d, 2H)<br>5.46 (s, 1H)<br>6.49 (s, 1H)<br>7.55 (s u. tb, 3H) | 124-126 |
| 51 | 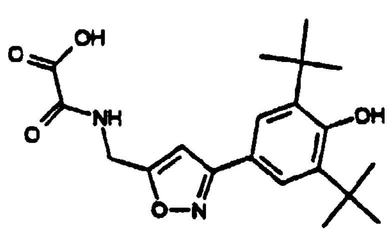 | DMSO-d6 | 1.41 (s, 18H)<br>4.48 (d, 2H)<br>6.81 (s, 1H)<br>7.40 (sb, 1H)<br>7.55 (s, 2H)<br>9.50 (tb, 1H)<br>13.7 (sb, 1H) | 165 (Zers.) |

| | | | | |
|---|---|---|---|---|
| 52 | 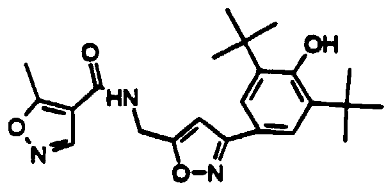 | CDCl3 | 1.46 (s, 18H)<br>2.73 (s, 3H)<br>4.72 (d, 2H)<br>5.47 (s, 1H)<br>6.51 (s, 1H u. tb, 1H)<br>7.57 (s, 2H)<br>8.41 (s, 1H) | 146-148 |
| 53 | 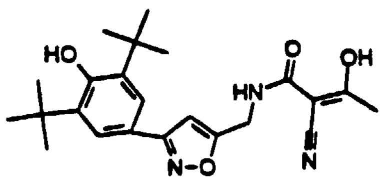 | CDCl3 | 1.47 (s, 18H)<br>2.31 (s, 3H)<br>4.67 (d, 2H)<br>5.46 (s, 1H)<br>6.46 (s, 1H)<br>6.51 (tb, 1H)<br>7.58 (s, 2H)<br>15.3 (sb, 1H) | 178-180 |
| 54 | 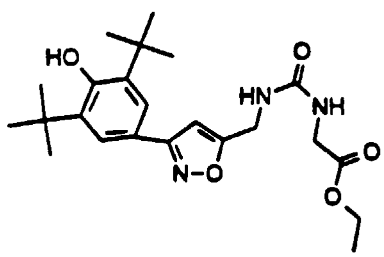 | CDCl3 | 1.25 (t, 3H)<br>1.46 (s, 18H)<br>4.0 (d, 2H)<br>4.18 (q, 2H)<br>4.54 (d, 2H)<br>5.38 (tb, 1H)<br>5.45 (s, 1H)<br>5.54 (tb, 1H)<br>6.47 (s, 1H)<br>7.57 (s, 2H) | 145-150 |
| 55 | 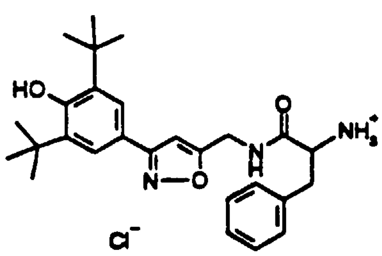 | DMSO-d6 | 1.44 (s, 18H)<br>3.0-3.22 (m, 2H)<br>4.07 (tb, 1H)<br>4.45 (db, 2H)<br>6.75 (s, 1H)<br>7.15-7.35 (m, 5H)<br>7.54 (s, 2H)<br>8.05 (sb, acide H)<br>9.35 (tb, 1H) | 98-138<br>(amorph) |

| | | | | |
|---|---|---|---|---|
| 56 | 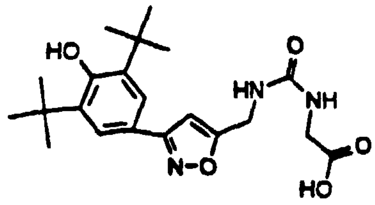 | DMSO-d6 | 1.42 (s, 18H)<br>3.73 (d, 2H)<br>4.37 (d, 2H)<br>6.40 (tb, 1H)<br>6.68 (s, 1H)<br>6.85 (tb, 1H)<br>7.40 (sb, 1H)<br>7.53 (s, 2H) | 159-165 |
| 57 | 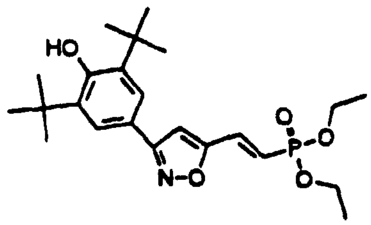 | DMSO-d6 | 1.29 (t, 6H)<br>1.43 (s, 18H)<br>4.0-4.15 (m, 2H)<br>6.75 (t, 1H)<br>7.35 (dd, 1H)<br>7.47 (d, 1H)<br>7.59 (s, 2H) | 119-125 |
| 58 | 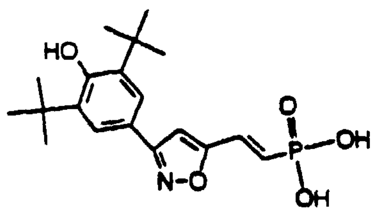 | DMSO-d6 | 1.44 (s, 18H)<br>6.68 (dd, 1H)<br>7.15 (dd, 1H)<br>7.39 (s, 1H)<br>7.60 (s, 2H)<br>9.3-10.0 (sb, acide H) | 247-249<br>(Zers.) |
| 59 | 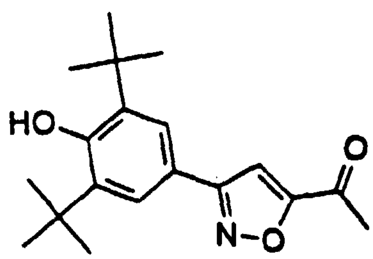 | CDCl3 | 1.49 (s, 18H)<br>2.65 (s, 3H)<br>5.53 (s, 1H)<br>7.17 (s, 1H)<br>7.64 (s, 2H) | 161-162 |
| 60 | 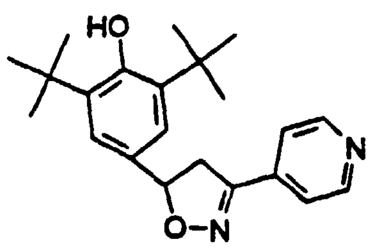 | CDCl3 | 1.44 (s, 18H)<br>3.25-3.41 u. 3.60-3.75 (jew. m, 1H)<br>5.31 (s, 1H)<br>5.71 (m, 1H)<br>7.17 (s, 2H)<br>7.57 u. 8.70 (jew. m, 2H) | 191-193 |

| | | | | |
|---|---|---|---|---|
| 61 | 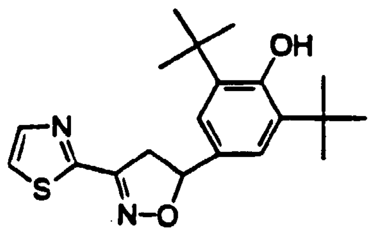 | DMSO-d6 | 1.39 (s, 18H)<br>3.35-3.55 u. 3.81-3.99<br>(jew. m, 1H)<br>5.73 (m, 1H)<br>7.11 (s, 1H)<br>7.16 (s, 2H)<br>7.92 u. 8.02 (jew. d, 2H) | 89-91 |
| 62 | 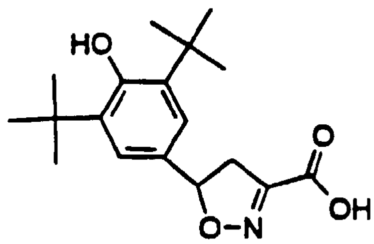 | CDCl3 | 1.44 (s, 18H)<br>3.17-3.33 u. 3.51-3.67<br>(jew. m, 1H)<br>5.33 (sb, 1H)<br>5.78 (m, 1H)<br>7.13 (s, 2H)<br>6.7-7.5 (sb, acide H) | 151-153 |
| 63 | 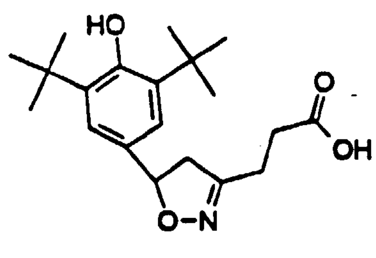 | CDCl3 | 1.43 (s, 18H)<br>2.60-2.82 (m, 4H)<br>2.90-3.05 u. 3.20-3.38<br>(jew. m, 1H)<br>5.25 (sb, 1H)<br>5.49 (m, 1H)<br>7.13 (s, 2H)<br>6.1-7.6 (sb, acide H) | 171-174 |
| 64 | 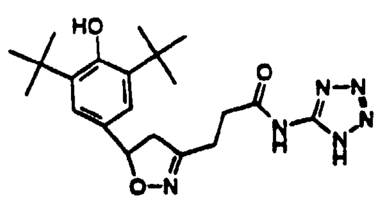 | CDCl3 | 1.20 (s, 18H)<br>2.47-2.88 (m, 5H)<br>3.06-3.25 (m, 1H)<br>4.25 (sb, acide H)<br>5.28 (m, 1H)<br>6.89 (s, 2H) | 220-224 |
| 65 | 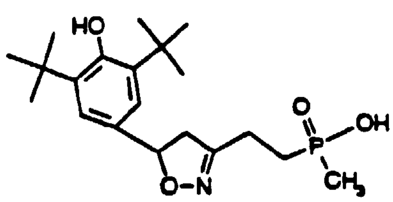 | CDCl3 | 1.43 (s, 18H)<br>1.53 (d, 3H)<br>2.0-2.21 (m, 2H)<br>2.55-2.79 (m, 2H)<br>2.90-3.05 u. 3.20-3.39<br>(jew. m, 1H)<br>5.35 (sb, acide H)<br>5.48 (m, 1H)<br>7.13 (s, 2H) | 182-184<br>(Zers.) |

| | | | | |
|---|---|---|---|---|
| 66 | 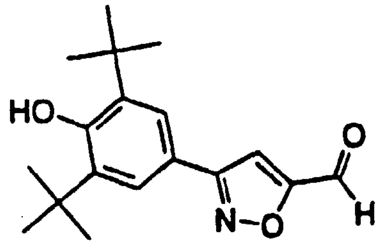 | CDCl3 | 1.49 (s, 18H)<br>5.54 (s, 1H)<br>7.25 (s, 2H)<br>10.02 (s, 1H) | 127 |
| 67 | 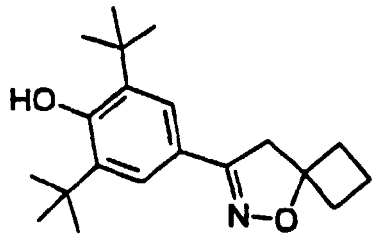 | CDCl3 | 1.35-1-95 (m, 2H u. s, 18H)<br>2.1-2.3 (m, 2H)<br>2.45-2.67 (m, 2H)<br>3.39 (s, 2H)<br>5,46 (s, 1H)<br>7.49 (s, 2H) | 121-122 |
| 68 | 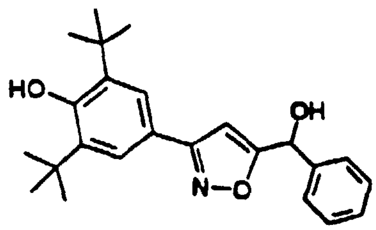 | CDCl3 | 1.47 (s, 18H)<br>2.8 (sb, 1H)<br>5.45 (sb, 1H)<br>5.95 (s, 1H)<br>6.44 (s, 1H)<br>7.3-7.5 (m, 5H)<br>7.59 (s, 2H) | 140 |
| 69 | 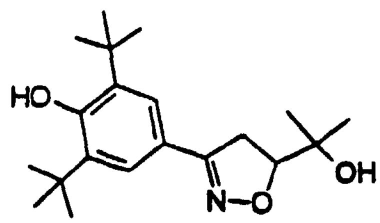 | CDCl3 | 1.23 u. 1.35 (jew. s, 3H)<br>1.45 (s, 18H)<br>2.06 sb, 1H)<br>3.15-3.43 (m, 2H)<br>4.53 (m, 1H)<br>5.48 (sb, 1H)<br>7.50 (s, 2H) | 139 |
| 70 | 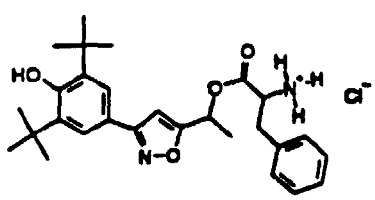 | DMSO-d6 | 1.3-1.65 (2s, 18H u. 2d, 3H)<br>2.95-3.3 (m, 2H)<br>4.42 (tb, 1H)<br>5.9-6.13 (m, 1H)<br>6.98 u. 7.02 (2s, 1H)<br>7.1-7.55 (m, 5H u. 2s, 2H)<br>8.4 (sb, 3H) | 105-125<br>(Zers.) |

| | | | | |
|---|---|---|---|---|
| 71 | 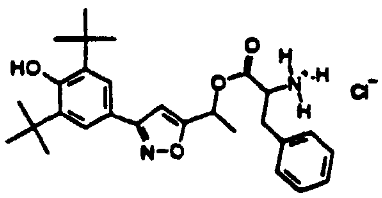 | DMSO-d6 | 1.3-1.45 (s, 18H u. d, 3H)<br>3.04-3.3 (m, 2H)<br>4.38 (tb, 1H)<br>5.93-6.08 (m, 1H)<br>7.11 (s, 1H)<br>7.28-7.41 (m, 5H)<br>7.56 (s, 2H)<br>8.4 (sb, 3H) | 105-115<br>(Zers.) |
| 72 | 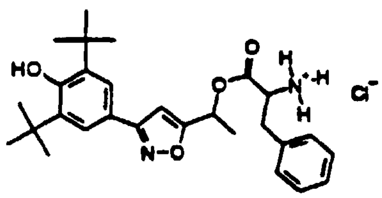 | DMSO-d6 | 1.43 (s, 18H)<br>1.63 (d, 3H)<br>3.0-3.25 (m, 2H)<br>4.41 (tb, 1H)<br>6.05-6.14 (m, 1H)<br>7.0 (s, 1H)<br>7.1-7.28 (m, 5H)<br>7.56 (s, 2H)<br>8.72 (sb, 3H) | 190 |
| 73 | 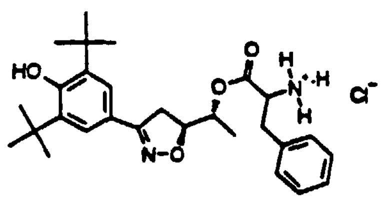 | DMSO-d6 | 1.02 u. 1.23 (jew. d, 1.5H)<br>1.35 u. 1.40 (jew. s, 9H)<br>2.85-3.57 (m, 4H)<br>4.1-4.3 (m, 1H)<br>4.54-4.78 (m, 1H)<br>4.95-5.15 (m, 1H)<br>7.1-7.48 (m, 5H u. 2d, jew. 1H)<br>8.55 (sb, 3H) | 210-215 |
| 74 | 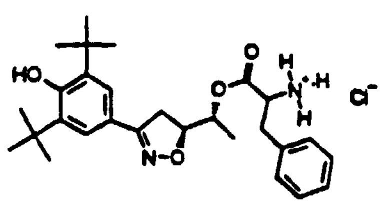 | DMSO-d6 | 1.02 (d, 3H)<br>1.40 (s, 18H)<br>2.90-3.50 (m, 4H)<br>4.1-4.25 (m, 1H)<br>4.58-4.73 (m, 1H)<br>4.90-5.04 (m, 1H)<br>7.1-7.31 (m, 5H)<br>7.37 (s, 2H)<br>8.79 (sb, 3H) | 207 |

| | | | | |
|---|---|---|---|---|
| 75 | 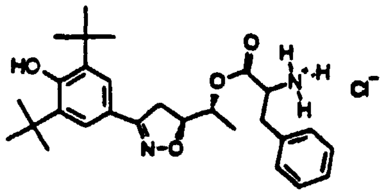 | DMSO-d6 | 1.23 (d, 3H)<br>1.35 (s, 18H)<br>2.95-3.06 (d, 2H)<br>3.20-3.57 (m, 2H)<br>4.25 (t, 1H)<br>4.63-4.78 (m, 1H)<br>5.08-5.15 (m, 1H)<br>7.1-7.3 (m, 5H)<br>7.38 (s, 2H)<br>8.40 (sb, 3H) | 193 |
| 76 | 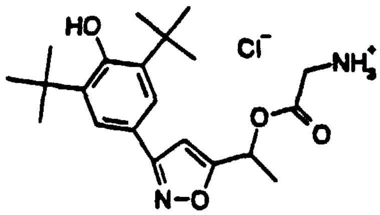 | DMSO-d6 | 1.43 (s, 18H)<br>1.66 (d, 3H)<br>3.93 (sb, 2H)<br>6.13 (q, 1H)<br>7.17 (s, 1H)<br>7.45 (sb, 1H)<br>7.57 (s, 2H)<br>8.60 (sb, 3H) | 70-85<br>(Zers.) |
| 77 | 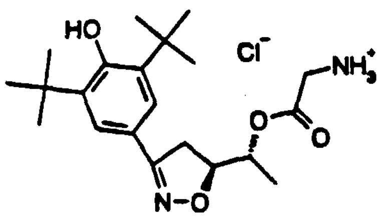 | DMSO-d6 | 1.23 (d, 3H)<br>1.40 (s, 18H)<br>3.22-3.90 (m, 4H)<br>4.65-4.85 (m, 1H)<br>5.0-5.18 (m, 1H)<br>7.40 (s, 2H)<br>7.47 (sb, 1H)<br>8.40 (sb, 3H) | 90-110<br>(Zers.) |
| 78 | 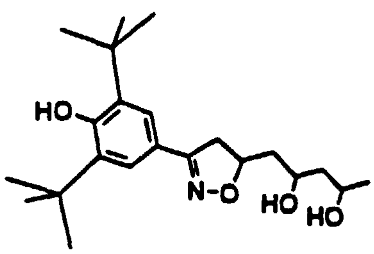 | CDCl3 | 1.22 (d, 3H)<br>1.45 (s, 18H)<br>1.50-2.05 (m, 4H)<br>2.97-3.12 u. 3.40-3.57<br>(jew. m, 1H)<br>3.2-3.8 (sb, 2H)<br>4.0-4.28 (m, 2H)<br>4.82-5.02 (m, 1H)<br>5.50 (s, 1H)<br>7.49 (s, 2H) | 144-145 |

| | | | | |
|---|---|---|---|---|
| 79 | 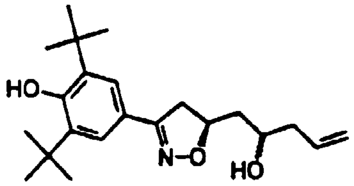 | CDCl3 | 1.45 (s, 18H)<br>1.50-2.0 (m, 2H)<br>2.2-2.35 (m, 2H)<br>2.6 (sb, 1H)<br>3.07-3.16 u. 3.40-3.56 (jew. m, 1H)<br>3.85-4.05 (m, 1H)<br>4.8-5.0 (m, 1H)<br>5.08-5.23 (m, 2H)<br>5.50 (s, 1H)<br>5.7-5-95 (m, 1H)<br>7.49 (s, 2H) | 103-105 |
| 80 | 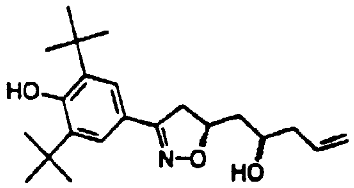 | CDCl3 | 1.45 (s, 18H)<br>1.55-2.45 (m, 5H)<br>2.95-3.10 u. 3.40-3.56 (jew. m, 1H)<br>3.91-4.05 (m, 1H)<br>4.87-5.05 (m, 1H)<br>5.08-5.23 (m, 2H)<br>5.48 (s, 1H)<br>5.7-5-95 (m, 1H)<br>7.49 (s, 2H) | 115-118 |
| 81 | 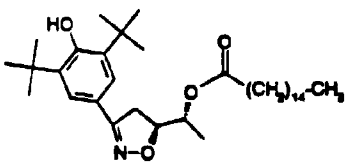 | CDCl3 | 0.88 (t, 3H)<br>1.15-1.7 (m, 27 H u. s, 18 H bei 1.46)<br>2.3 (t, 2H)<br>3.05-3-25 u. 3.3-3.47 (jew. m, 1H)<br>4.6-4-75 (m, 1H)<br>4.98-5.12 (m, 1H)<br>5.48 (s, 1H)<br>7.48 (s, 2H) | Öl |
| 82 | 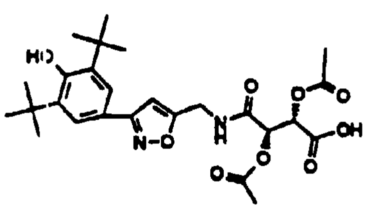 | CdCl3 | 1.47 (s, 18 H)<br>2.06 u. 2.21 (jew. s, 3H)<br>4.45-4.81 (m, 2H)<br>5.48 (sb, 1H)<br>5.64 u. 5.83 (jew. d, 2H)<br>6.49 (s, 1H)<br>6.85 (t, 1H)<br>7.56 (s, 2H) | 88-92<br>(Zers.) |

| Beisp. | Struktur | Solvens | 1-H-NMR | Schmp. |
|---|---|---|---|---|
| 83 | 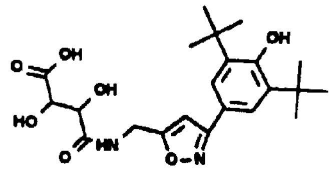 | DMSO-d6 | 1.43 (s, 18H)<br>4.30-4.51 (m, 4H)<br>6.69 (s, 1H)<br>7.39 (sb, 1H)<br>7.52 (s, 2H)<br>8.48 (tb, 1H) | 125-154 |
| 84 | 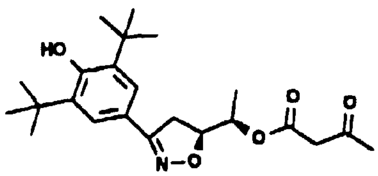 | CDCL3 | 1.34 (d, 3H)<br>1.46 (s, 9H)<br>2.25 (s, 3H)<br>3.13-3.49 (m, 2H )<br>3.45 (s, 2H)<br>4.62-4.77 (m, 1H)<br>5.03-5.18 (m, 1H)<br>5.49 (sb, 1H)<br>7.49 (s, 2H) | 102 |
| 85 | 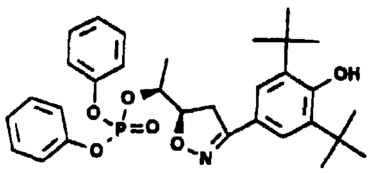 | DMSO-d6 | 1.31 (d, 3H)<br>1.40 (s, 18H)<br>3.18-3.60 (m, 2H)<br>4.69-4.95 (m, 2H)<br>7.13-7.48 (m, 12H) | 118 |
| 86 | 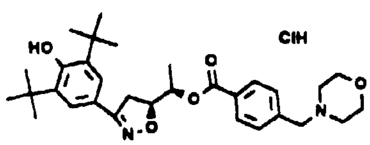 | DMSO-d6 | 1.32 (d, 3H)<br>1.38 (s, 18H)<br>2.95-3.98 (m, 10H)<br>4.38 (sb, 2H)<br>4.76-4.91 (m, 1H)<br>5.19-5.28 (m, 1H)<br>7.40 (s, 2H)<br>7.43 (sb, 1H)<br>7.73 (d, 2H)<br>7.94 (d, 2H)<br>11.79 (sb, 1H) | 241-243<br>(Zers.) |
| 87 | 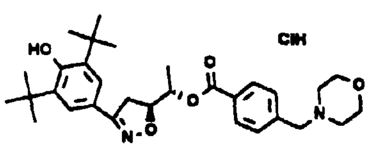 | DMSO-d6 | 1.32-1.45 (d, 3H u. s, 18H)<br>2.95-4.00 (m, 10H)<br>4.40 (db, 2H)<br>4.74-4.91 (m, 1H)<br>5.09-5.23 (m, 1H)<br>7.41 (s, 2H)<br>7.43 (sb, 1H)<br>7.76 (d, 2H)<br>8.00 (d, 2H)<br>11.50 (sb, 1H) | 248-255<br>(Zers.) |
| 88 | 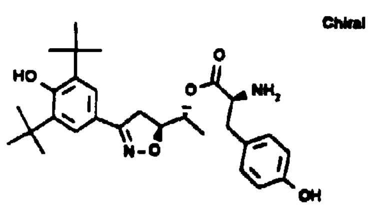 | CDCL3 | 1.25 u. 1.30 (jew. d, 1.5H)<br>1.41 u. 1.44 (jew. s, 9H)<br>2.65-3.71 (m, 5H)<br>4.65-4.82 (m, 1H)<br>5.04-5.22 (m, 1H)<br>5.49 (sb, 1H)<br>6.55-6.68 (m, 2H)<br>6.85-6.97 (m, 2H)<br>7.47 u. 7.49 (jew. s, 1H) | 110-135 |
| 89 | 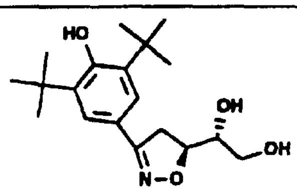 | DMSO-d6 | 1.41 (s, 9H)<br>3.26-3.63 (m, 5H)<br>4.52-4.71 (m, 1H u. sb, 1H)<br>5.02 (d, 1H)<br>7.37 (s, 1H )<br>7.41 ( s, 2H) | 155 |
| | | | | |

| Beisp. | Struktur | Solvens | 1-H-NMR | Schmp. |
|---|---|---|---|---|
| 90 | 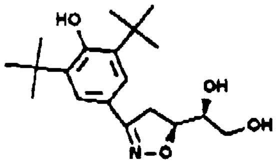 | DMSO-d6 | 1.40 (s, 9H)<br>3.14-3.52 (m, 5H)<br>4.56-4.72 (m, 2H)<br>4.85 (d, 1H)<br>7.36 (s, 1H )<br>7.39 ( s, 2H) | 129 |
| 91 | 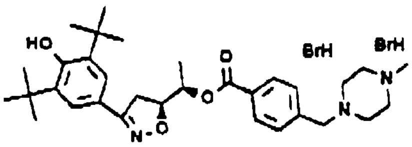 | DMSO-d6 | 1.32 (d, 3H)<br>1.41 (s, 18H)<br>2.87 (sb, 3H)<br>3.10-3.68 (m, 10H)<br>4.27 (sb, 2H)<br>4.79-4.92 (m, 1H)<br>5.21-5.33 (m, 1H)<br>7.45 (s, 2H)<br>7.47 (sb, 1H)<br>7.61 (d, 2H)<br>7.96 (d, 2H)<br>10.0 (sb, 1H) | 195-198 |
| | | | | |

Pharmakologische Prüfungen

Die Verbindungen der Formel I wurden zur Charakterisierung ihrer wertvollen antiphlogistischen, antiasthmatischen und immunmodulierenden Eigenschaften und guten Verträglichkeit in folgenden experimentellen Testanordnungen untersucht, die sich anerkanntermaßen besonders gut für die Beurteilung der Wirkqualität derartiger antirheumatisch aktiver Verbindungen eignen.

Gewinnung von polymorphonuklearen Leukozyten (PMNL) aus peripherem Humanblut zur Durchführung von Test 1 und 2

10 ml Humanblut wurden mit 10 ml HBSS (Serva) verdünnt und mit 15 ml Lymphoprep (Dr. Molter GmbH, Heidelberg) unterschichtet. Es wurde 25 Minuten bei 400xg (1600 Umdrehungen pro Minute (rpm), Minifuge 2, Heraeus, Osterode) zentrifugiert. Dabei bildeten sich drei Phasen: Die Oberste besteht aus Plasma und Thrombozyten, die mittlere Phase ist Lymphoprep. Zwischen diesen beiden Phasen befindet sich ein weißer, aus mononuklearen Zellen bestehender Ring. Die unterste Phase besteht aus Erythrozyten, die mit einer weißen Schicht PMNL bedeckt sind. Die oberste Phase, sowie das Lymphoprep und der weiße Ring wurden mit einer Spritze vorsichtig entfernt. Der verbleibende Niederschlag wurde vorsichtig in 15 ml 3 %igem Dextran in PM16 (MG: 485 000, Sigma, Deisenhofen) resuspendiert. Dann blieb die Suspension eine Stunde bei Raumtemperatur stehen. Dabei sedimentierten die Erythrozyten, während die PMNL im Überstand verblieb. Der Überstand wurde entnommen, mit etwa dem gleichen Volumen PM16 verdünnt und 15 Minuten bei 400xg zentrifugiert. Sollte der PMNL-Niederschlag durch Erythrozyten stark kontaminiert sein, ist eine hypotone Lyse notwendig. Dazu wurde der Niederschlag in 750 µl Wasser resuspendiert. Nach exakt 15 Sekunden wurde mit etwa 7 ml PM16 verdünnt und 10 Minuten bei 400xg zentrifugiert. Diese Lyse wurde, wenn nötig noch einmal wiederholt. Sonst wurde der PMNL-Niederschlag in einem geeigneten Medium resuspendiert.

TEST 1. Freisetzung proteolytischer Aktivität

250 µl PMNL ($5{\cdot}10^6$ Zellen/ml PBS mit 7,5 mM Glucose) wurden mit 1 µl Testsubstanz 20 Minuten bei 37°C inkubiert. Dann wurde 1 µl Cytochalasin B (5 µg/ml Testansatz) zugegeben und weitere 5 Minuten bei 37'C inkubiert. Nach Zugabe von 1 µl Tissue Necrosis Faktor alpha (TNF; 30 ng/ml Testansatz) wurde die Inkubation für 5 Minuten fortgesetzt. Gestartet wurde die Freisetzung mit 1 µl fMLP (10 nMol/L Testansatz). Nach 60 Minuten bei 37'C wurde die Reaktion durch Abkühlen der Proben auf 0°C beendet. Die Proben wurden zentrifugiert, der Überstand entnommen und eingefroren. Cytochalasin B und fMLP wurde in DMSO gelöst und TNF mit Puffer verdünnt. 50 µl des Überstandes wurden in einer 96-well-Mikrotiterplatte mit 175 µl Puffer (100 mM HEPES pH7.5; 500 mM NaCl) gemischt. Nach Zugabe von 25 µl Substrat (Methoxy-succinyl-L-Ala-L-Ala-L-Pro-L-Val-p-nitroanilid) wurde bei 405 nm die Extinktionen jeder Vertiefung nach 0, 5, 10,15, 30 und 60 Minuten gemessen.

TEST 2. Freisetzung von Sauerstoff-Radikalen

Die PMNL wurden in Dulbeccos PBS, komplettiert mit 7,5 mM Glucose, resuspendiert ($5{\cdot}10^6$ Zellen/ml). Es ist darauf zu achten, daß die PMNL frei von Erythrozyten sind. In einer Halbmikroküvette wurden 100 µl PMNL, 700 µl PBS + Glucose und 100 µl Cytochrom C (9,38 mg/ml PBS + Glucose) gemischt. Für die fMLP induzierte Sauerstoffreisetzung wurden 100 µl PMNL, 500 µl PBS + Glucose, 100µl Cytochalasin B ($1{\cdot}10^{-6}$M), 100µl TNF (0,05ng) und 100 µl Cytochrom C (9,38 mg/ml PBS + Glucose) gemischt. Gemessen wurde in einem Zweistrahlphotometer (Perkin-Elmer 552 S) bei einer Wellenlänge von 550 nm. Der Meßbereich beträgt 0-1 Extinktionseinheiten. Die Küvetten wurden auf 37°C thermostatisiert. Die Referenzküvette enthielt zusätzlich 10 µl SOD (6 mg SOD/ml PBS + Glucose). Nach 5 Minuten Vorlauf wird die Reaktion mit 100 µl Inducer gestartet. Die Reaktion wird mit $1{\cdot}10^{-7}$M fMLP gestartet.

TEST 3. $LTB_4$-Bildung weißer Blutzellen

Gewinnung weißer Blutzellen:

40 ml Humanblut wurden mit 8 ml 6%iger Dextranlösung (Dextran MG:480 000 in PM16) gemischt. Nach einer Stunde bei Raumtemperatur wurde der aus weißen Blutzellen bestehende Überstand entnommen, mit dem gleichen Volumen PM16 verdünnt und 15 Minuten bei 300xg (1600 rpm) zentrifugiert. Der Niederschlag wurde zunächst in etwa 5-6 ml PM16 resuspendiert und nach Bestimmen der Zellzahl im Coulter Counter auf $10^7$ Zellen/ml eingestellt.

Der Testansatz bestand aus 0,24 ml Zellsuspension, 0,03 ml 20 mM $CaCl_2$/5 mM $MgCl_2$ und 5 µl Prüfsubstanz. Nach 15 Minuten Vorinkubation bei 37°C wurde die Reaktion mit 0,03 ml A23187/Glutathion (100 µg A23187/ml; 15,4 µg Glutathion/ml) gestartet. Die Inkubation wurde nach 5 Minuten bei 37°C durch sofortiges Kühlen der Proben auf 0°C beendet. Nach 2 minütigem Zentrifugieren in einer Eppendorff-Zentrifuge (0°C) wurde der Überstand entnommen und chromatographisch untersucht.

Hochdruckflüssigchromatographie (HPLC): Die HPLC-Anlage besteht aus einer Kratos Pumpe (Spectro-flow 400), einem gekühlten automatischen Probeninjektor BT 7041 (Biotronik) mit einer 75 µl Probenschleife, einer Nucleosil $C_{18}$ Säule (100*3 mm, 5 µm Partikel von Chrompack), einem UV-Detektor (Kratos) Spectroflow 757 (Wellenlänge: 280 nm, Meßbereich: 0,1 AUFS, Zeitkonstante: 0,5 s) und einem Spectra-Physics Integrator (SP 4270). Das Laufmittel (725 ml Methanol, 275 ml Wasser und 0,1 ml Essigsäure) wurde mit einer Flußrate von 0,7 ml/Minute, bei einem Druck von etwa 100 bar gefördert.
Bemerkungen: Es hat sich als nützlich erwiesen, vor Beginn der eigentlichen Messungen eine zusätzliche Kontrollprobe zu messen. Dabei kann der eingestellte Meßbereich überprüft und gegebenenfalls geändert werden. Zwingend notwendig ist es, die Prüfsubstanzen in der jeweiligen Testkonzentration (z.B.: $10^{-5}$ M) in PM16 ebenfalls vor den Zellinkubaten zu messen, da die Prüfsubstanzen den $LTB_4$-Peak überdecken könnten. Ist dieses der Fall, muß eine niedrigere Konzentration gewählt werden.

Test 4. Stimulierung der Cytokin Freisetzung

Gewinnung mononuklearer Zellen aus Humanblut

10 ml Humanblut, stabilisiert mit 1 ml 3,8%iger Natriumcitrat-lösung, wurde mit 10 ml PM16 (Serva, Heidelberg) verdünnt und mit 15 ml Lymphoprep (Dr.Molter GmbH, Heidelberg) unterschichtet. Die Proben wurden 40 Minuten bei 400xg (1600 rpm, Minifuge 2, Heraeus, Osterode) Raumtemperatur zentrifugiert. Die mononuklearen Zellen sind als weißer Ring an der Grenze Lymphoprep/Plasma sichtbar. Dieser Ring wurde mit einer Spritze vorsichtig entnommen, mit dem gleichen Volumen PM16 verdünnt und 10 Minuten bei 400xg zentrifugiert. Der Niederschlag wird mit etwa 10 ml RPMI1640 (+300 mg/l L-Glutamin, Gibco, Eggenstein) gewaschen (Wallis 1986). Nach Resuspendieren der Zellen in etwa 1 ml RPMI1640 (+ 300 mg/l L-Glutamin + 25 mM HEPES + 100 µg/ml Streptomycin + 100 µg/ml Penicillin) wurde die Zelldichte mit einem Coulter Counter JT (Coulter Diagnostics) bestimmt und auf $5*10^6$/ml eingestellt. Die Zellen bestehen typischerweise aus 90% Lymphozyten und 10% Monozyten.
230 µl mononukleare Zellen wurden mit 10 µl Testsubstanz (10 µM in DMSO/Wasser 1/10) und 10 µl Lipopolysaccharide (LPS; 500 pg gelöst in 1 ml DMSO und vor Testbeginn 1/10 mit Wasser verdünnt, von Salmonella abortus equi, Sigma, Deisenhofen) 20-22 Stunden bei 37°C; 5% $CO_2$ (bei IL-6 5 Stunden) inkubiert. Die Proben wurden in einem Eisbad auf 0°C abgekühlt und in einer Sigma-Zentrifuge zentrifugiert (2 Minuten; 2000rpm). Aliquote des Überstandes werden mit einem Elisa (Biermann, Bad Nauheim) bestimmt. Die Abkürzung IL steht für Interleukin, TNF für Tumor nekrosis faktor alpha.

TEST 5. Cyclooxygenase-abhängige Thrombozyten-Aggregation

Plättchenreiches Plasma (PRP): Humanblut wurde zur Gerinnungshemmung mit $^1/_{10}$ seines Volumens 3,8%iger Natriumcitratlösung versetzt. Dann wird 20 Minuten bei 105xg zentrifugiert und der Überstand (PRP) entnommen. In einem PAP4 Aggregometer (BioData) wurden 200 µl PRP, mit 30 µl Prüfsubstanz 10 Minuten bei 37°C vorinkubiert. Die Prüfsubstanz wurde in organischem Lösemittel gelöst und mit Puffer (50 mM TisHCl, pH 8,0; 90 mM NaCl) 1:30 verdünnt. Die Aggregation wird durch 20 µl Inducer ausgelöst. Während der Aggregation wird mit 800 rpm gerührt. Als Inducer wurde Arachidonsäure 5 mg/ml verwendet.
Die Ergebnisse der Teste 1 bis 3 sind in Tabelle 2 zusammengefasst. Die Werte in der Tabelle zeigen die eingesetzte Konzentration der Prüfsubstanz in pM und daneben die gemessene Restaktivität in Prozent bezogen auf die Kontrolle die gleich 100% gesetzt wurde. In Test 4 wird zusätzlich das Interleukin angegeben.

Tabelle 2

| Beisp. | Test 1 | | Test 2 | | Test 3 | | Test 4 | | Test 5 | |
|---|---|---|---|---|---|---|---|---|---|---|
| 1 | | | | | 10μM | 92 | | | 10μM | 93 |
| 3 | | | | | | | | | 100μM | 98 |
| 5 | | | | | | | | | 100μM | 98 |
| 7 | | | | | | | | | 100μM | 95 |
| 8 | | | | | 10μM<br>1μM | 15<br>93 | | | 100μM | 91 |
| 9 | 100μM | 64 | | | 10μM<br>1μM | 0<br>56 | IL-1<br>10μM<br>IL-6<br>10μM<br>TNFα<br>10μM | 73<br><br><br>93<br><br>63 | 100μM | 92 |
| 10 | | | | | 10μM<br>1μM | 20<br>89 | TNFα<br>10μM | 78 | 100μM | 98 |
| 13 | 100μM<br>10μM | 20<br>77 | 25μM<br>10μM | 22<br>65 | | | | | 10μM | 98 |
| 14 | | | | | | | | | 10μM100 | |
| 18 | | | | | | | IL-1α<br>100 μM<br>10μM | <br>36<br>89 | | |
| 19 | 100μM<br>10μM | 20<br>78 | 10μM | 94 | | | | | 10μM | 100 |
| 20 | | | | | | | | | 10μM | 100 |
| 24 | | | | | | | IL-1<br>10μM | <br>86 | 10μM | 94 |
| 25 | | | | | 10μM<br>1μM | 29<br>85 | IL-1α<br>10μM<br>ILβ | <br>62 | 10μM | 86 |

Tabelle 2 (continued)

| Beisp. | Test 1 | | Test 2 | | Test 3 | | Test 4 | | Test 5 | |
|---|---|---|---|---|---|---|---|---|---|---|
| | | | | | | | 10µM<br>TNFα<br>10µM | 93<br><br>82 | | |
| 26 | | | | | | | TNFα<br>10µM | 88 | 10µM | 94 |
| 27 | | | | | | | | | 100µM | 97 |
| 28 | 10µM<br>1µM | 59<br>97 | 25µM<br>10µM | 8<br>60 | 10µM<br>1µM | 0<br>14 | IL-1α<br>10µM | <br>93 | 10µM | 93 |
| 31 | | | | | | | | | 100µM | 100 |
| 32 | 100µM<br>10µM<br>1µM | 34<br>72<br>84 | | | | | | | | |
| 34 | | | | | | | | | 100µM | 98 |
| 35 | | | | | | | IL-1α<br>10µM | <br>84 | 100µM | 94 |
| 36 | | | | | 10µM | 93 | | | 100µM | 87 |
| 38 | | | | | | | IL-1α<br>1µM<br>IL-1β<br>1µM | <br>89<br><br>85 | 100µM | 90 |
| 39 | | | | | | | | | 100µM | 98 |
| 41 | 100µM | 78 | <br>50µM<br>5µM | <br>36<br>75 | 10µM | 13 | TNFα<br>10µM | <br>89 | 100µM | 95 |
| 43 | | | | | 10µM | 93 | | | 100µM | 99 |
| 44 | | | | | | | | | 10µM | 99 |
| 45 | | | | | | | | | 100µM | 98 |
| 46 | | | | | | | IL-1α | | 100µM | 90 |

Tabelle 2 (continued)

| Beisp. | Test 1 | | Test 2 | | Test 3 | | Test 4 | | Test 5 | |
|---|---|---|---|---|---|---|---|---|---|---|
| | | | | | | | 1µM<br>IL-1β<br>1µM<br>TNFα<br>10µM | 77<br><br>85<br><br>79 | | |
| 47 | 100µM<br>10µM<br>1µM | 17<br>38<br>85 | | | | | IL-1α<br>10µM<br>1µM<br>TNFα<br>10µM | <br>72<br>89<br><br>64 | | |
| 49 | 100µM<br>10µM<br>1µM | 19<br>23<br>88 | 50µM<br>5µM | 12<br>80 | 10µM<br>1µM | 0<br>90 | IL-6<br>1µM<br>TNFa<br>10µM | <br>91<br><br>91 | | |
| 57 | | | | | | | | | 100µM | 99 |
| 58 | | | | | | | | | 100µM | 99 |
| 59 | 100µM<br>10µM<br>1µM | 35<br>61<br>91 | 10µM<br>1µM | 48<br>135 | | | | | | |
| 60 | | | | | 10µM | 88 | | | | |
| 61 | 100µM | 80 | | | 10µM | 3 | IL-1α<br>1µM<br>TNFα<br>10µM | <br>94<br><br>68 | 100µM | 94 |
| 63 | 100µM<br>10µM | 66<br>88 | 50µM | 79 | 10µM | 91 | IL-1α<br>10µM<br>IL-1β<br>10µM | <br>75<br><br>77 | | |
| 64 | 100µM<br>10µM | 72<br>96 | | | | | IL-1α<br>10µM | <br>68 | 100µM | 97 |

Tabelle 2 (continued)

| Beisp. | Test 1 | | Test 2 | | Test 3 | | Test 4 | | Test 5 | |
|---|---|---|---|---|---|---|---|---|---|---|
| | | | | | | | 1µM<br>IL-1<br>10µM | 85<br><br>82 | | |
| 70 | | | 10µM | 22 | | | | | | |
| 71 | 10µM<br>1µM | 42<br>79 | | | | | | | 25µM | 97 |
| 72 | 10µM<br>1µM | 51<br>82 | | | | | | | 25µM | 100 |
| 73 | | | 10µM<br>1µM | 9<br>100 | | | | | 25µM | 100 |
| 74 | 10µM | 64 | | | | | | | 25µM | 95 |
| 75 | 10µM<br>1µM | 49<br>89 | | | | | | | 25µM | 98<br>1 |
| 76 | 100µM<br>10µM | 9<br>68 | | | | | | | 25µM | 99 |
| 77 | 100µM<br>10µM | 30<br>74 | | | | | | | 25µM | 100 |
| 78 | | | | | | | | | 50µM | 94 |
| 79 | | | | | | | | | 100µM | 96 |
| 80 | | | | | | | | | 50µM | 97 |
| 81 | | | | | | | | | 100µM | 100 |
| 82 | | | | | | | | | 100µM | 93 |
| 85 | | | | | | | | | 100µM | 94 |

Interpretation der Tabelle am Beispiel 9):
Nach Zugabe von 100 µl des Hemmstoffes Beisp. 9) werden 64 % der proteolytischen Aktivität (Test 1) freigesetzt. Ohne Hemmstoff würden entsprechend 100 % proteolytische Aktivität freigesetzt.

ERGEBNIS: Die erfindungsgemäßen Verbindungen sind wirksam als Entzündungshemmer, denn sie zeigen deutliche biologische Aktivitäten als Inhibitoren der Freisetzung proteolytischer Aktivität (Test 1) und von aktivem Sauerstoff (Test 2), sowie der Leukotrien B4-Synthese (Test 3) im humanen Leukozyten-System. Die Freisetzung verschiedener Zytokine im humanen System aus mononuklearen Zellen (Test 4) wird ebenfalls gehemmt. Insbesondere haben die Verbindungen keine inhibitorische Wirkung auf das cyclooxygenaseabhängige humane Thrombozyten-System (Test 5). Unerwünschte Nebenwirkungen, (Verlängerung der Blutungszeit,
gastrointestinale Ulcerogenese, Nierentoxizität) ,die auf eine Hemmung der Cyclooxygenase zurückzuführen sind, sind folglich nicht zu erwarten.

Test 6: Prüfung auf antiarthritische Wirkung am Adjuvans-Arthritis Modell

Als Versuchstiere wurden männliche Ratten eines Wistar-Lewis-Stammes (Mollegaard/Dänemark) verwendet. Das Körpergewicht liegt zwischen 160 und 200 g. Subplantare Injektion von 0,1 ml Freund'schem Adjuvans (= 6 mg Mycobacterium-butyricum-Suspension, Difco Lab./Detroit, Mich. USA, pro ml schwerem weißen Paraffinöl, Merck, Darmstadt) in die Schwanzwurzel führte vom 10, bis 14. Versuchstag zu immunpathologischen Prozessen, chronischen Entzündungen, insbesondere in Form arthritischer und periarthritischer Symptome, an anderen Körperstellen (sekundäre Läsionen). Die Tiere wurden ad libitum mit Standardfutter Altromin-R, Firma Altrogge, Lage, und Leitungswasser ernährt. Die Versuchstiere wurden vom 14. Tag bis Versuchsende mit 60 mg/kg Codein analgetisiert. Die Prüfsubstanzen wurden täglich (insgesamt über 17 Tage), beginnend am Tag der Adjuvans-Applikation oral als CMC-Suspension in einem Injektionsvolumen von 1 ml/100 g Körpergewicht verabreicht. Am ersten und 18. Versuchstag wurde das Volumen beider Hinterpfoten und das Körpergewicht bestimmt. Als Wirkungskriterium diente die Herabsetzung der Pfotenvolumenzunahme gegenüber der unbehandelten Kontrollgruppe, wobei beide Hinterpfoten gemittelt wurden. Die Ergebnisse sind in Tabelle 3 zusammengefasst.

Test 7: Prüfung auf antiasthmatische Wirkung

Für die Prüfung auf antiasthmatische Wirkung wurden narkotisierte Meerschweinchen verwendet, die durch Kanulierung der Vena jugularis eine Möglichkeit der Injektion des PAF bieten. Die Prüfpräparate wurden auch intravenös (i.v.) 5 min vor PAF-Verabreichung durch die selbe venöse Kanüle in die Blutbahn oder intraduodenal (i.d.) durch eine operativ angebrachte und kanulierte Öffnung des Zwölffingerdarmes - 15 Minuten vor PAF - appliziert. Registriert wurde die Bronchialweite in einem Over-flow-Pneumonogramm vor und nach Präparategabe. Bewertung: $ED_{50}$ in mg/kg i.d. gegenüber dem PAF-induzierten Asthma-Anfall. Die Ergebnisse sind in Tabelle 3 zusammengefasst.

Test 8: Hemmung der Tumorzellproliferation

Zur Bestimmung der antiproliferativen Eigenschaft von Testsubstanzen wurde die Tumorzellinie 20-10-5S (Hybridomazellinie, bezogen von ATCC (American Type Culture Collection)), die einen Antikörper gegen T-Zellen der Maus produziert) verwendet. Die Zellen wurden unter serumfreien Bedingungen in CG-Medium [Cell-Growth-Medium = Iscove-ATL (+ Albumin + Transferrin + Lipide); Firma Vitromex, Vilshofen] bei 37° C und 5 % $CO_2$ gezüchtet. Zum Testansatz wurden nur Zellen in der logarithmischen Wachstumsphase verwendet. Die Teststubstanzen wurden in verschiedenen Konzentrationen (von 1 bis 50 µM) zusammen mit 4 x $10^3$ 20-10-5S Zellen in Rundbodenmikrotiterplatten pipettiert (Gesamtvolumen mit Medium 200 µl). Nach einer Inkubationszeit von 48 h (37° C und 5 % $CO_2$) wurde in die Platten 25 µl Tritium-Thymidin (= 0,25 µCi/Testansatz, spezifische Aktivität 23 Ci/mM) zugegeben. In den folgenden 16 h wurde das radioaktiv markierte Thymidin in die DNA der wachsenden Zellen eingebaut. Die Ansätze wurden auf Glasfaserfilter abgesaugt und die eingebaute Radio-aktivität wurde mit einem β-Counter (Beta-Plate-System 1205 der Firma Wallac) bestimmt. Die $IC_{50}$ Werte (50 % Hemmungs-konzentration) wurden aufgrund der eingebauten Radioaktivität gegenüber der Positivkontrolle (Testansatz ohne Testsubstanz) berechnet und sind in Tabelle 3 zusammengefasst.

Tabelle 3

| Beispiel | Test 6 | Test 6 | Test 7 | Test 8 |
|---|---|---|---|---|
| | Dosis | Pfotenvolumen | $ED_{50}$ | $IC_{50}$ |
| | (mg/kg) | (% Hemmung) | (mg/kg) | (μM) |
| 1 | 50 | 70 | 10-50i.d. | 5-10 |
| 3 | | | | 10 |
| 4 | | | | 30 |
| 5 | | | <10i.d. | |
| 6 | | | ca.50i.d. | |
| 7 | | | 10-30i.d. | |
| 8 | 50 | 67 | ca.10i.d. | <5 |
| 9 | | | ca.50.i.d. | |
| 11 | | | | <5 |
| 12 | 50 | 82 | | 10 |
| 13 | 50 | 84 | ca.50.i.d. | 6.10 |
| 14 | | | 10-50i-d- | |
| 15 | 50 | 60 | | 8-15 |
| 16 | | | | 30 |
| 17 | | | | 8-15 |
| 18 | | | | 5-10 |
| 19 | 25 | 85 | | <5 |
| 20 | 25 | 67 | ca.50i.d. | <5 |
| 22 | 50 | 74 | | |
| 24 | 50 | 43 | | < 5 |
| 25 | 50 | 44 | 3-10i.d. | <5 |
| 26 | | | | <5 |
| 27 | 50 | 71 | | < 5 |
| 28 | 50 | 74 | | <5 |
| 29 | | | | <5 |
| 30 | 50 | 63 | | 5-10 |
| 33 | | | | <5 |
| 37 | | | | <5 |
| 39 | | | | 30 |
| 40 | | | 1-3i.d. | < 5 |
| 41 | | | | <5 |
| 42 | | | | 5-10 |
| 44 | | | | 5-10 |
| 45 | | | | 5-10 |
| 47 | | | 10-50i.d. | 5-10 |

Tabelle 3 (fortgesetzt)

| Beispiel | Test 6 | Test 6 | Test 7 | Test 8 |
|---|---|---|---|---|
| | Dosis | Pfotenvolumen | $ED_{50}$ | $IC_{50}$ |
| | (mg/kg) | (% Hemmung) | (mg/kg) | (µM) |
| 48 | 50 | 60 | 10-50i.d. | 5-10 |
| 49 | | | ca.50i.d. | 5-10 |
| 52 | | | 3-10i.d. | <5 |
| 53 | 25 | 48 | 10-50i.d. | 8-10 |
| 54 | | | 1-3i.d. | 5-10 |
| 55 | 50 | 47 | | <5 |
| 56 | | | ca.50.i.d. | |
| 57 | | | | <5 |
| 59 | | | 1-3i.d. | 30 |
| 66 | 25 | 59 | | <5 |
| 67 | 50 | 53 | | <5 |
| 69 | 50 | 83 | | 5-10 |
| 70 | 25 | 59 | | 5 |
| 71 | 25 | 66 | | 5-10 |
| 72 | 25 | 60 | | 5-10 |
| 73 | 50 | 73 | | |
| 74 | 25 | 60 | | 5-10 |
| 75 | 25 | 76 | | 5-10 |
| 76 | 50 | 70 | | 20 |
| 77 | 50 | 82 | | 20 |
| 78 | | | | 5-10 |
| 79 | | | | 5-10 |
| 80 | | | | 5-10 |
| 81 | 50 | 67 | | |
| 83 | 50 | 39 | | |
| 84 | 25 | 49 | | |
| 85 | | | | <5 |
| 86 | 20 | 70 | | <5 |
| 87 | 20 | 50 | | <5 |
| 88 | | | | 10 |
| 89 | 50 | 36 | | 20 |
| 90 | 50 | 36 | | 30 |
| < bedeutet kleiner als | | | | |

**Patentansprüche**

**1.** Verbindungen der Formel I

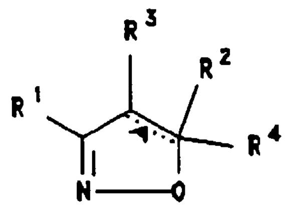

(I)

und/oder ein physiologisch verträgliches Salz der Verbindung der Formel I und/oder eine stereoisomere Form der Verbindung der Formel I; dabei hat ein Rest $R^1$ oder $R^2$ die Bedeutung der Formel II

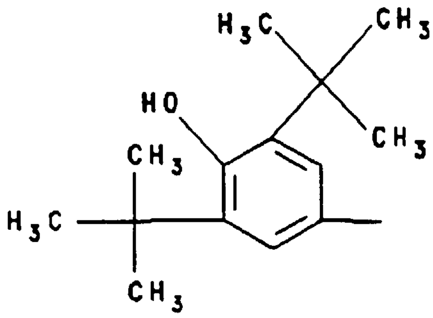

, (II)

und der andere Rest $R^1$ oder $R^2$ hat die nachstehende Bedeutung:

a) Pyridyl,
b) Thiophenyl,
c) Thiazolyl,
d) ein Rest der Formel III

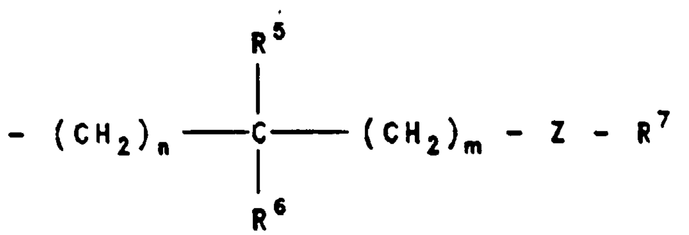

, (III)

wobei n für die Zahl Null, 1, 2, 3, 4, 5 oder 6,
m für die Zahl Null, 1, 2, 3 oder 4 steht,

Z ist

1) -O- oder
2) -NH-,

$R^5$ und $R^6$ sind unabhängig voneinander

1) Wasserstoffatom,
2) $(C_1-C_4)$-Alkyl,
3) $(C_2-C_4)$-Alkenyl,
4) $(C_2-C_4)$-Alkinyl,
5) Phenyl oder
6) OH, und

$R^7$ ist

1) Wasserstoffatom,
2) Rest einer Aminosäure, aus der Gruppe Asparagin, Valin, Arginin, Asparaginsäure, Glutamin, Glutaminsäure, Tryptophan, β-Alanin, Lysin, Prolin, Glycin, y-Aminobutyrat, Nε -Acetyllysin, Nδ-Acetylornithin, Ny-Acetyldiaminobutyrat, Nα-Acetyldiaminobutyrat, Histidin, Isoleucin, Leucin, Methionin, Phenylalanin, Serin, Cystein, Threonin, Alanin und Tyrosin,
3) Trifluormethylsulfonyl,
4) $-O-P(O)(OH)_2$,
5) $-O-P(O)(OH)_2$, ein- oder zweifach verestert mit Phenyl oder $(C_1-C_6)$Alkyl, oder
6) ein Rest der Formel IV

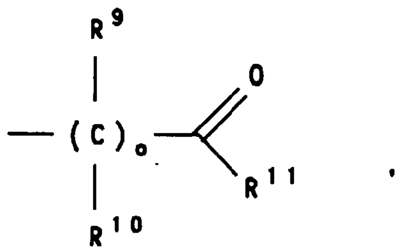

(IV)

dabei ist o die Zahl Null oder 1,
dabei haben $R^9$ und $R^{10}$ unabhängig voneinander die nachstehende Bedeutung:

1) Wasserstoffatom oder
2) $(C_1-C_4)$-Alkyl, und

$R^{11}$ ist

1) OH,
2) $-O-(C_1-C_4)$-Alkyl,
3) $(C_1-C_{20})$-Alkyl,
4) $(C_1-C_{20})$-Alkyl, ein oder mehrfach unabhängig voneinander substituiert durch

4.1 -COOH,
4.2 -OH,
4.3 O-Acetyl, oder
4.4 =O,

5) -COOH,
6)

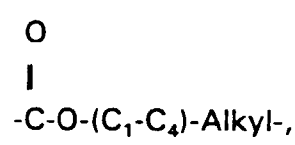

7) N-Glycyl,
8) N-Glycyl-$(C_1-C_4)$-Alkylester,
9) N-$(C_1-C_4)$-Alkyl-hydroxylamino,
10) N-(1H-tetrazol-5-yl)-amino,
11) 5-Methyl-isoxazol-4-yl
12) 1-Cyano-2-hydroxy-1-propenyl,
13) Phenyl,
14) Phenyl, ein- oder mehrfach substituiert durch

14.1

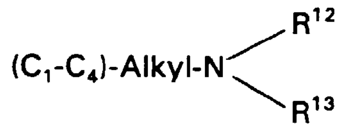

dabei haben $R^{12}$ und $R^{13}$ unabhängig voneinander die nachstehende Bedeutung:

1) Wasserstoffatom,
2) $(C_1-C_4)$-Alkyl,
3) Phenyl-$(C_1-C_2)$-Alkyl,
4) $R^{12}$ und $R^{13}$ bilden, zusammen mit dem sie verknüpfenden Stickstoffatom einen fünf- bis siebengliedrigen Heterozyklus, wobei ein Kohlenstoffatom durch ein Schwefel-, Sauerstoff- oder Stickstoffatom ersetzt sein kann, oder
5) Rest einer Aminosäure aus der Gruppe Asparagin, Valin, Arginin, Asparaginsäure, Glutamin, Glutaminsäure, Tryptophan, β-Alanin, Lysin, Prolin, Glycin, γ-Aminobutyrat, Nε-Acetyllysin, Nδ-Acetylornithin, Nγ-Acetyldiaminobutyrat, Nα-Acetyl-diaminobutyrat, Histidin, Isoleucin, Leucin, Methionin, Phenylalanin, Serin, Cystein, Threonin, Alanin und Tyrosin, oder

$R^6$ und $R^7$ sind miteinander verbunden und sind zusammen 1 bis 3 $CH_2$-Reste,

e) ein Rest der Formel V

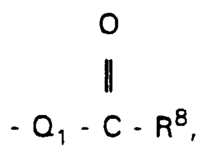 (V)

dabei ist $Q_1$

1) $-(CH_2)_m-$, wobei m die Zahl Null, 1, 2, 3 oder 4 ist, oder
2) -CH=CH-, und

$R^8$ ist

1) Wasserstoffatom,
2) $(C_1-C_4)$-Alkyl,
3) OH,
4) $-O-(C_1-C_4)$-Alkyl,
5) $NH_2$,
6) N-(1 H-Tetrazol-5-yl)-amino oder
7) N-(3,5-Dimethyl-4-hydroxybenzyl)-amino, oder

f) ein Rest der Formel VI

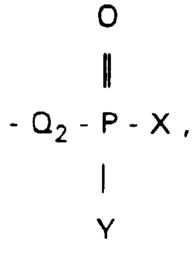 (VI)

dabei ist $Q_2$

1) $-(CH_2)_m$, wobei m die Zahl Null, 1, 2, 3 oder 4 ist, oder
2) -CH=CH-, und

X und Y sind unabhängig voneinander

1) $(C_1-C_4)$-Alkyl,
2) -O-$(C_1-C_4)$-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) $(C_1-C_6)$-Alkyl, oder

$R^2$ und $R^4$ bilden zusammen mit dem Kohlenstoffatom an das sie gebunden sind, einen aliphatischen Ring der aus 3, 4 oder 5 Kohlenstoffatomen besteht und
$R^3$ ist

1) Wasserstoffatom,
2) $(C_1-C_4)$-Alkyl oder
3) Hydroxy-$(C_1-C_4)$-alkyl.

**2.** Verbindung der Formel I nach Anspruch 1, dabei hat ein Rest $R^1$ oder $R^2$ die Bedeutung der Formel II und der andere Rest $R^1$ oder $R^2$ hat die nachstehende Bedeutung:

a) 2-Pyridyl,
b) 4-Pyridyl,
c) Thiophen-3-yl,
d) 2-Thiazolyl,
e) ein Rest der Formel III,

dabei ist n die Zahl Null, 1, 2, 3 oder 4,
m ist die Zahl Null oder 1,
Z ist

1) -O- oder
2) -NH-, oder

$R^5$ und $R^6$ sind unabhängig voneinander

1) Wasserstoffatom,
2) $(C_1-C_2)$-Alkyl,
3) Phenyl oder
4) OH, oder

$R^6$ und $R^7$ sind miteinander verbunden und bilden zusammen eine Methylengruppe, oder
$R^7$ ist

1) Wasserstoffatom,
2) Trifluormethylsulfonyl,
3) eine Aminosäure aus der Gruppe Gly, Phe, Ala, Lys, Tyr oder Ser, oder
4) ein Rest der Formel IV,

dabei ist o die Zahl Null oder 1,

$R^9$ und $R^{10}$ sind unabhängig voneinander

1) Wasserstoffatom oder
2) Methyl,

$R^{11}$ ist

1) OH,
2) -O-($C_1$-$C_4$)-Alkyl,
3) ($C_1$-$C_{18}$-)Alkyl,
4) ($C_1$-$C_6$)-Alkyl, ein oder mehrfach unabhängig voneinander substituiert durch

4.1 -COOH,
4.2 OH oder
4.3 O-Acetyl,

5) -COOH,
6)

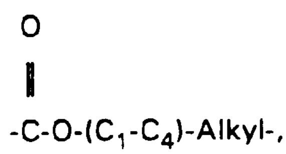

7) N-Glycyl,
8) N-Glycyl-($C_1$-C4)-Alkylester,
9) N-Methyl-hydroxylamino,
10) N-(1H-Tetrazol-5-yl-amino),
11) 5-Methyl-isoxazol-4-yl
12) 1-Cyano-2-hydroxy-1-propenyl oder
13) Phenyl, einfach substituiert durch

13.1) 4-(4-Morpholino)methyl,

f) ein Rest der Formel V,

dabei ist $Q_1$

1) $-(CH_2)_m-$, wobei m die Zahl Null, 1 oder 2 ist, oder
2) -CH=CH- und

$R^8$ ist:

1) Wasserstoffatom,
2) Methyl,
3) OH,
4) -O-($C_1$-$C_2$)-Alkyl,
5) Amino,
6) N-(1H-Tetrazol-5-yl)-amino oder
7) N-3,5-Dimethyl-4-hydroxybenzyl, oder

g) ein Rest der Formel VI,

dabei ist $Q_2$

1) $-(CH_2)_m-$, wobei m die Zahl Null, 1 oder 2 ist, oder

2) -CH = CH-,

X und Y sind unabhängig voneinander

1) Methyl,
2) -O-($C_1$-$C_2$)-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) ($C_1$-$C_2$)-Alkyl, oder

$R^2$ und $R^4$ bilden zusammen mit dem Kohlenstoffatom, an das sie gebunden sind, einen aliphatischen Ring mit 3, 4 oder 5 Kohlenstoffatomen,
$R^3$ ist

1) Wasserstoffatom,
2) Methyl oder
3) Hydroxymethyl.

**3.** Verbindung der Formel I nach Anspruch 1 oder 2, dabei hat ein Rest $R^1$ oder $R^2$ die Bedeutung der Forme II und der andere Rest $R^1$ oder $R^2$ hat die nachstehende Bedeutung:

a) 2-Pyridyl,
b) 4-Pyridyl,
c) Thiophen-3-yl,
d) 2-Thiazolyl,
e) ein Rest der Formel III,

dabei ist n die Zahl Null, 1, 2, 3 oder 4,
m ist die Zahl Null oder 1,
Z ist

1) -O- oder
2) -NH-, oder

$R^5$ und $R^6$ sind unabhängig voneinander

1) Wasserstoffatom,
2) ($C_1$-$C_2$)-Alkyl, oder
3) Phenyl, oder

$R^6$ und $R^7$ sind miteinander verbunden und bilden zusammen eine Methylengruppe, oder
$R^7$ ist

1) Wasserstoff,
2) Trifluormethylsulfonyl,
3) eine Aminosäure aus der Gruppe Gly, Lys oder Phe, oder
4) ein Rest der Formel IV,

dabei ist o die Zahl Null oder 1,
$R^9$ und $R^{10}$ sind unabhängig voneinander

1) Wasserstoffatom oder
2) Methyl,

$R^{11}$ ist

1) OH,
2) O-Methyl oder O-Ethyl,
3) ($C_1$-$C_{18}$)-Alkyl,
4) ($C_1$-$C_6$)-Alkyl, ein oder mehrfach unabhängig voneinander substituiert durch:

4.1 -COOH,
4.2 OH oder
4.3 O-Acetyl,

5) -COOH,
6)

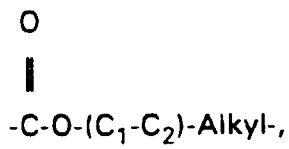

7) N-Glycyl,
8) N-Glycyl-($C_1$-$C_2$)-Alkylester,
9) N-Methyl-hydroxylamino,
10) N-(1 H-Tetrazol-5-yl-amino),
11) 5-Methyl-isoxazol-4-yl oder
12) 1-Cyano-hydroxy-1-propenyl,

f) ein Rest der Formel V,

dabei ist $Q_1$

1) $-(CH_2)_m-$, wobei m die Zahl Null, 1 oder 2 ist, oder
2) -CH=CH- und

$R^8$ ist

1) Wasserstoffatom,
2) Methyl,
3) OH,
4) -O-Methyl,
5) Amino,
6) N-(1 H-Tetrazol-5-yl)-amino oder
7) N-3,5-Dimethyl-4-hydroxybenzyl, oder

g) ein Rest der Formel VI,

dabei ist $Q_2$

1) $-(CH_2)_m-$, wobei m die Zahl Null ist, oder
2) -CH = CH-,

X und Y sind unabhängig voneinander

1) Methyl,
2) -O-($C_1$-$C_2$)-Alkyl oder
3) OH,

...A... ist eine Doppelbindung, die vorhanden ist oder fehlt, mit der Einschränkung, daß die Doppelbindung und der Rest $R^4$ nicht gleichzeitig vorkommen,
$R^4$ ist

1) Wasserstoffatom oder
2) Methyl oder

$R^2$ und $R^4$ bilden zusammen mit dem Kohlenstoffatorn, an das sie gebunden sind, einen aliphatischen Ring mit 3, 4 oder 5 Kohlenstoffatomen,
$R^3$ ist

1) Wasserstoffatom oder
2) Hydroxymethyl.

**4.** Verfahren zur Herstellung von 2-Isoxazolin und Isoxazol der Formel I gemäß Anspruch 1, und/oder einer stereoisomeren Form der Verbindung der Formel I und/oder eines physiologisch verträglichen Salzes der Verbindung der Formel I, dadurch gekennzeichnet, daß man

a) eine primäre Nitroverbindung mittels Isocyanaten und katalytischen Mengen Triethylamin oder ein durch Chlorierung eines entsprechenden Aldoximes erhaltes Hydroxamsäurechlorid mit einer organischen oder anorganischen Base in das entsprechende Nitriloxid überführt und dieses intermediär erhaltene Nitriloxid ohne Reinigung mit einem entsprechend substituierten Olefin oder Alkin im Sinne einer 1,3-dipolaren Cycloaddition zu einem 2-Isoxazolin oder Isoxazol der Formel I umsetzt und das entstandene Produkt gegebenenfalls durch Kristallisation oder Chromatographie reinigt, oder

b) einen nach a) oder o) hergestellten Carbonsäureester der Formel I oder eine über Verfahren h) oder k) zusätzlich eingeführte Estergruppe zur Carbonsäure verseift, oder

c) einen nach Verfahren a) oder o) hergestellten Carbonsäurealkylester der Formel I mit einem entsprechend substituierten primären oder sekundären Amin in das entsprechende Amid überführt, oder

d) einen nach a) oder o) hergestellten Phosphinsäuremonoalkyl- oder Phosphonsäuredialkylester der Formel I zum Phosphonsäurehalbester, zur Phosphonsäure oder zur Phosphinsäure verseift, oder

e) eine nach b) erhaltene Carbonsäure zunächst in ein aktiviertes Säurederivat überführt, dieses anschließend mit Alkoholen verestert oder mit primären und sekundären Aminen in das entsprechende Amid oder mit N-alkyliertem Hyroxylamin in das entsprechende Hydroxylamid überführt, oder

f) in einer nach den Verfahren a), b), h), e), o) oder g) intermediär erhaltene oder mitgeführte N- oder O-Schutzgruppe abspaltet, oder eingeführte oder mitgeführte Carbonsäure-, Phosphin-, Phosphon-, oder Phosphorsäureester entsprechend verseift und eine Verbindung der Formel I erhält, oder

g) eine nach Verfahren f) oder o) erhaltene Verbindung mit einer freien Aminogruppe durch Umsetzung mit Isocyanat in das entsprechende Harnstoffderivat, oder durch Umsetzung mit einem aktivierten Carbonsäurederivat oder einer N-geschützten Aminosäure in das entsprechende Amid oder durch Umsetzung mit einem Sulfonsäurechlorid in das entsprechende Sulfonamid überführt, oder

h) eine nach Verfahren a), o) oder f) erhaltene Verbindung mit einer freien Alkoholgruppe durch Umsetzung mit Isocyanat in das entsprechende Urethan oder durch Umsetzung mit einem an der Carboxygruppe aktivierten, gegebenenfalls weitere funktionelle Gruppen tragenden Carbonsäurederivat oder einem entsprechenden N-geschützten Aminosäurederivat in den entsprechenden Ester oder mit Diketen in das entsprechende 3-Oxo-butyrat oder durch Umsetzung mit einer Halogenverbindung wie $\alpha$-Halogen-Carbonsäure in den entsprechenden Ether überführt, oder

i) eine nach Verfahren a), o) oder f) erhaltene Verbindung mit einer primären oder sekundären Alkoholgruppe zu dem entsprechenden Aldehyd oder Keton oxidiert, oder

k) eine nach Verfahren i) hergestellte Carbonylverbindung durch Umsetzung mit einer Base und Dialkylphosphonoessigsäureester in das entsprechende Crotonsäurederivat oder durch Umsetzung mit Tetraalkylmethylendiphosphonat in das entsprechende trans-2-(Dialkoxyphosphono)-vinyl-Derivat überführt, oder

l) eine während der Cycloaddition eingeführte Epoxid-Gruppe in das entsprechende 1,2-Diol überführt, oder

m) ein nach Verfahren g) hergestelltes Amid, das einen 3-H-Isoxazolring enthält, durch Behandlung mit einer Base im Sinne einer Ringöffnung in das entsprechende substituierte 2-Cyano-3-hydroxy-crotonsäureamid überführt, oder

n) eine nach Verfahren a) - m) hergestellte Verbindung der Formel I, die aufgrund ihrer chemischen Struktur in enantiomenren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppe in die reinen Enantiomeren auftrennt, oder

o) ein durch Cycloaddition an chirale oder racemische Olefine erhaltenes 2-Isoxazolin-Diastereomerengemisch der Formel I durch Säulenchromatographie an Kieselgel in die reinen Diastereomeren auftrennt, oder

p) die nach Verfahren a) - o) hergestellte Verbindung der Formel I entweder in freier Form isoliert oder im Falle des Vorliegens von sauren oder basischen Gruppen gegebenenfalls in physiologisch verträgliche kristalline Salze umwandelt, oder

q) eine nach Verfahren h) hergestellte Verbindung, die eine weitere funktionelle Gruppe wie eine Halogenmethylgruppe trägt, mit einer primären oder sekundären Aminogruppe alkyliert oder mit einem Alkohol verethert.

**5.** Arzneimittel, enthaltend eine wirksame Menge von mindestens einer Verbindung der Formel I nach den Ansprüchen 1, 2 oder 3 oder wie erhalten nach Anspruch 4 und/oder ein physiologisch verträgliches Salz der Verbindung der Formel I und/oder eine stereoisomere Form der Verbindung der Formel I, neben physiologisch annehmbaren Hilfs- und Trägerstoffen, gegebenenfalls weiteren Zusatzstoffen und/oder anderen Wirkstoffen.

**6.** Verwendung von mindestens einer Verbindung der Formel I nach den Ansprüchen 1, 2 oder 3 zur Herstellung von Arzneimitteln zur Prophylaxe und Therapie von asthmatischen Erkrankungen, Entzündungen und/oder Autoimmunerkrankungen.

**7.** Verfahren zur Herstellung eines Arzneimittels nach Anspruch 5, dadurch gekennzeichnet, daß man mindestens eine Verbindung der Formel I nach den Ansprüchen 1, 2 oder 3 und/oder ein physiologisch verträgliches Salz der Verbindung der Formel I und/oder eine stereoisomere Form der Verbindung der Formel I und/oder eine nach dem Verfahren nach Anspruch 4 erhaltene Verbindung der Formel I mit physiologisch annehmbaren Hilfs- und Trägerstoffen und gegebenenfalls weiteren Zusatzstoffen und/oder anderen Wirkstoffen in eine geeignete Darreichungsform bringt.

## Claims

**1.** A compound of the formula I

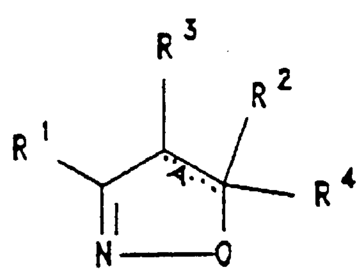

( I )

and/or a physiologically tolerated salt of the compound of the formula I

and/or a stereoisomeric form of the compound of the formula I; in this context, one radical $R^1$ or $R^2$ has the meaning of the formula II

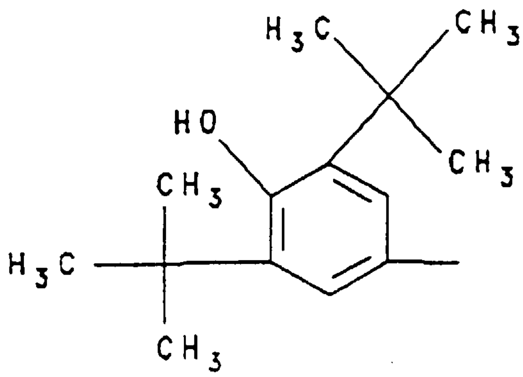

,

(II)

and the other radical $R^1$ or $R^2$ has the following meaning:

a) pyridyl,
b) thiophenyl,
c) thiazolyl,
d) a radical of the formula III

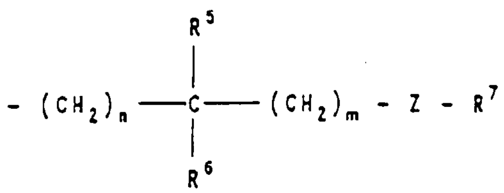

,

(III)

where

n is the number zero, 1, 2, 3, 4, 5 or 6,
m is the number zero, 1, 2, 3 or 4,
Z is

1) -O-, or
2) -NH-,

$R^5$ and $R^6$ are, independently of each other,

1) a hydrogen atom,
2) $(C_1-C_4)$-alkyl,
3) $(C_2-C_4)$-alkenyl,
4) $(C_2-C_4)$-alkynyl,
5) phenyl, or
6) OH, and

$R^7$ is

1) a hydrogen atom,
2) residue of an amino acid from the group asparagine, valine, arginine, aspartic acid, glutamine, glutamic acid, tryptophan, β-alanine, lysine, proline, glycine, γ-aminobutyrate, Nε-acetyllysine, Nδ-acetylornithine, Nγ-acetyldiaminobutyrate, Nα-acetyldiaminobutyrate, histidine, isoleucine, leucine, methionine, phenylalanine, serine, cysteine, threonine, alanine and tyrosine,
3) trifluoromethylsulfonyl,

4) -O-P(O)(OH)$_2$,
5) -O-P(O)(OH)$_2$, mono- or diesterified with phenyl or ($C_1$-$C_6$)-alkyl, or
6) a radical of the formula IV

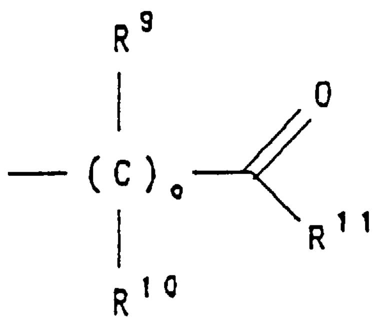

,

(IV)

in which o is the number zero or I, and in which $R^9$ and $R^{10}$, independently of each other, have the following meaning:

1) a hydrogen atom, or
2) ($C_1$-$C_4$)-alkyl, and

$R^{11}$ is

1) OH,
2) -O-($C_1$-$C_4$)-alkyl,
3) ($C_1$-$C_{20}$)-alkyl,
4) ($C_1$-$C_{20}$)-alkyl, substituted once or more than once, independently of each other, by

4.1 -COOH,
4.2 -OH,
4.3 O-acetyl, or
4.4 =O

5) -COOH,
6)

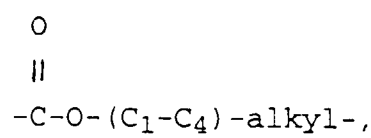

7) N-glycyl,
8) N-glycyl-($C_1$-$C_4$)-alkyl ester,
9) N-($C_1$-$C_4$)-alkylhydroxylamino,
10) N-(1H-tetrazol-5-yl)amino,
11) 5-methylisoxazol-4-yl,
12) 1-cyano-2-hydroxy-1-propenyl,
13) phenyl,
14) phenyl, substituted once or more than once by

14.1

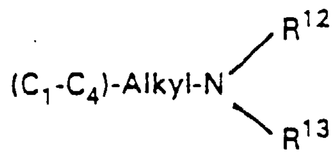

where $R^{12}$ and $R^{13}$ have, independently of each other, the following meaning:

1) hydrogen atom,
2) ($C_1$-$C_4$)-alkyl,
3) phenyl-($C_1$-$C_2$)-alkyl,
4) $R^{12}$ and $R^{13}$ form, together with the nitrogen atom linking them, a five-membered to seven-membered heterocycle, with it being possible for a carbon atom to be replaced by a sulfur, oxygen or nitrogen atom, or
5) residue of an amino acid selected from the group asparagine, valine, arginine, aspartic acid, glutamine, glutamic acid, tryptophan, β-alanine, lysine, proline, glycine, γ-aminobutyrate, Nε-acetyllysine, Nδ-acetylornithine, Ny-acetyldiaminobutyrate, Nα-acetyldiaminobutyrate, histidine, isoleucine, leucine, methionine, phenylalanine, serine, cysteine, threonine, alanine and tyrosine, or

$R^6$ and $R^7$ are bonded to each other and are, together, from 1 to 3 $CH_2$ radicals,

e) a radical of the formula V

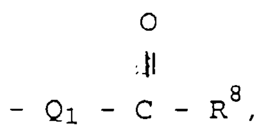 (V)

in which $Q_1$ is

1) -$(CH_2)_m$-, where m is the number zero, 1, 2, 3 or 4, or
2) -CH=CH-, and

$R^8$ is

1) a hydrogen atom,
2) ($C_1$-$C_4$)-alkyl,
3) OH,
4) -O-($C_1$-$C_4$)-alkyl,
5) $NH_2$,
6) N-(1H-tetrazol-5-yl)amino, or
7) N-(3,5-dimethyl-4-hydroxybenzyl)amino, or

f) a radical of the formula VI

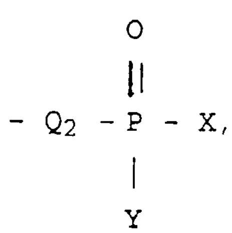 (VI)

in which $Q_2$ is

1) $-(CH_2)_m$, where m is the number zero, 1, 2, 3 or 4, or
2) -CH=CH-, and

X and Y are, independently of each other,

1) $(C_1-C_4)$-alkyl,
2) $-O-(C_1-C_4)$-alkyl, or
3) OH,

...A... is a double bond which is present or absent, with the restriction that the double bond and the radical $R^4$ are not present simultaneously,
$R^4$ is

1) a hydrogen atom, or
2) $(C_1-C_6)$-alkyl, or

$R^2$ and $R^4$ form, together with the carbon atom to which they are bonded, an aliphatic ring which is composed of 3, 4 or 5 carbon atoms, and
$R^3$ is

1) a hydrogen atom,
2) $(C_1-C_4)$-alkyl, or
3) hydroxy-$(C_1-C_4)$-alkyl.

**2.** A compound of the formula I as claimed in claim 1, where one radical $R^1$ or $R^2$ has the meaning of the formula II and the other radical $R^1$ or $R^2$ has the following meaning:

a) 2-pyridyl,
b) 4-pyridyl,
c) thiophen-3-yl,
d) 2-thiazolyl,
e) a radical of the formula III,

in which n is the number zero, 1, 2, 3 or 4, and
m is the number zero or 1,
Z is

1) -O-, or
2) -NH-, or

$R^5$ and $R^6$ are, independently of each other,

1) a hydrogen atom,
2) $(C_1-C_2)$-alkyl,
3) phenyl, or
4) OH, or

$R^6$ and $R^7$ are bonded to each other and together form a methylene group, or
$R^7$ is

1) a hydrogen atom,
2) trifluoromethylsulfonyl,
3) an amino acid from the group Gly, Phe, Ala, Lys, Tyr or Ser, or
4) a radical of the formula IV,

in which o is the number zero or 1,
$R^9$ and $R^{10}$ are, independently of each other,

1) a hydrogen atom, or
2) methyl,

$R^{11}$ is

1) OH,
2) -O-($C_1$-$C_4$)-alkyl,
3) ($C_1$-$C_{18}$)-alkyl,
4) ($C_1$-$C_6$)-alkyl, substituted once or more than once, independently of each other, by

4.1 -COOH,
4.2 OH, or
4.3 O-acetyl,

5) -COOH,
6)

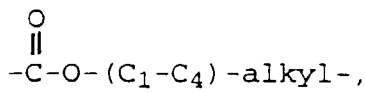

7) N-glycyl,
8) N-glycyl-($C_1$-$C_4$)-alkyl ester,
9) N-methylhydroxylamino,
10) N-(1H-tetrazol-5-ylamino),
11) 5-methylisoxazol-4-yl,
12) 1-cyano-2-hydroxy-1-propenyl, or
13) phenyl, substituted once by

13.1)4-(4-morpholino)methyl,

f) a radical of the formula V,

in which $Q_1$ is

1) -$(CH_2)_m$-, where m is the number zero, 1 or 2, or
2) -CH=CH-, and

$R^8$ is:

1) a hydrogen atom,
2) methyl,
3) OH,
4) -O-($C_1$-$C_2$)-alkyl,
5) amino,
6) N-(1H-tetrazol-5-yl)amino, or
7) N-3,5-dimethyl-4-hydroxybenzyl, or

g) a radical of the formula VI,

in which $Q_2$ is

1) -$(CH_2)_m$-, where m is the number zero, 1 or 2, or
2) -CH=CH-,

X and Y are, independently of each other,

1) methyl,
2) -O-($C_1$-$C_2$)-alkyl, or
3) OH,

...A... is a double bond which is present or absent, with the restriction that the double bond and the radical $R^4$ are not present simultaneously,
$R^4$ is

1) a hydrogen atom, or
2) ($C_1$-$C_2$)-alkyl, or

$R^2$ and $R^4$ form, together with the carbon atom to which they are bonded, an aliphatic ring having 3, 4 or 5 carbon atoms,
$R^3$ is

1) a hydrogen atom,
2) methyl, or
3) hydroxymethyl.

**3.** A compound of the formula I as claimed in claim 1 or 2, where one radical $R^1$ or $R^2$ has the meaning of the formula II and the other radical $R^1$ or $R^2$ has the following meaning:

a) 2-pyridyl,
b) 4-pyridyl,
c) thiophen-3-yl,
d) 2-thiazolyl,
e) a radical of the formula III,

in which n is the number zero, 1, 2, 3 or 4, and
m is the number zero or 1,
Z is

1) -O-, or
2) -NH-, or

$R^5$ and $R^6$ are, independently of each other,

1) a hydrogen atom,
2) ($C_1$-$C_2$)-alkyl, or
3) phenyl, or

$R^6$ and $R^7$ are bonded to each other and together form a methylene group, or
$R^7$ is

1) hydrogen,
2) trifluoromethylsulfonyl,
3) an amino acid from the group Gly, Lys or Phe, or
4) a radical of the formula IV,

in which o is the number zero or 1,
$R^9$ and $R^{10}$ are, independently of each other,

1) a hydrogen atom, or
2) methyl,

$R^{11}$ is

1) OH,

2) O-methyl or O-ethyl,
3) ($C_1$-$C_{18}$)-alkyl,
4) ($C_1$-$C_6$)-alkyl, substituted once or more than once, independently of each other, by:

4.1 -COOH,
4.2 OH, or
4.3 O-acetyl,

5) -COOH,
6)

O
2
-C-O-($C_1$-$C_2$)-alkyl-,

7) N-glycyl,
8) N-glycyl-($C_1$-$C_2$)-alkyl ester,
9) N-methylhydroxylamino,
10) N-(1H-tetrazol-5-ylamino),
11) 5-methylisoxazol-4-yl, or
12) 1-cyano-2-hydroxy-1-propenyl,

f) a radical of the formula V,

in which $Q_1$ is

1) -$(CH_2)_m$-, where m is the number zero, 1 or 2, or
2) -CH=CH-, and

$R^8$ is

1) a hydrogen atom,
2) methyl,
3) OH,
4) -O-methyl,
5) amino,
6) N-(1H-tetrazol-5-yl)amino, or
7) N-3,5-dimethyl-4-hydroxybenzyl, or

g) a radical of the formula VI,

in which $Q_2$ is

1) -$(CH_2)_m$-, where m is the number zero, or
2) -CH=CH-,

X and Y are, independently of each other,

1) methyl,
2) -O-($C_1$-$C_2$)-alkyl, or
3) OH,

...A... is a double bond which is present or absent, with the restriction that the double bond and the radical $R^4$ are not present simultaneously,
$R^4$ is

1) a hydrogen atom, or
2) methyl, or

$R^2$ and $R^4$ form, together with the carbon atom to which they are bonded, an aliphatic ring having 3, 4 or 5 carbon atoms,
$R^3$ is

1) a hydrogen atom, or
2) hydroxymethyl.

**4.** A process for the preparation of 2-isoxazoline and isoxazole of the formula I as claimed in claim 1, and/or a stereoisomeric form of the compound of the formula I, and/or a physiologically tolerated salt of the compound of the formula I, wherein

a) a primary nitro compound is converted into the corresponding nitrile oxide using isocyanates and catalytic quantities of triethylamine, or a hydroxamoyl chloride, obtained by chlorinating a corresponding aldoxime, is converted into the corresponding nitrile oxide using an organic or inorganic base, and this nitrile oxide, obtained as an intermediate, is reacted, without purification, with an appropriately substituted olefin or alkyne, in the sense of a 1,3-dipolar cycloaddition, to form a 2-isoxazoline or isoxazole of the formula I, and the resulting product is, where appropriate, purified by crystallization or chromatography, or

b) a carboxylic ester of the formula I, prepared in accordance with a) or o), or an ester group, additionally introduced via process h) or k), is hydrolyzed to the carboxylic acid, or

c) an alkyl carboxylate of the formula I, prepared in accordance with process a) or o), is converted, using an appropriately substituted primary or secondary amine, into the corresponding amide, or

d) a monoalkyl phosphinate or dialkyl phosphonate of the formula I, prepared in accordance with a) or o), is hydrolyzed to the phosphonic semi-ester, to the phosphonic acid or to the phosphinic acid, or

e) a carboxylic acid, obtained in accordance with b), is initially converted into an activated acid derivative and this derivative is subsequently esterified with alcohols or converted, using primary and secondary amines, into the corresponding amide, or converted, using N-alkylated hydroxylamine, into the corresponding hydroxylamide, or

f) a N-protective group or O-protective group which is obtained as an intermediate or entrained in a in accordance with the processes a), b), h), e), o) or g) is eliminated, or an introduced or entrained carboxylic ester, phosphinic ester, phosphonic ester or phosphoric ester is correspondingly hydrolyzed, and a compound of the formula I is obtained, or

g) a compound obtained in accordance with process f) or o), and having a free amino group, is converted into the corresponding urea derivative by reaction with isocyanate, or converted into the corresponding amide by reaction with an activated carboxylic acid derivative or an N-protected amino acid, or converted into the corresponding sulfonamide by reaction with a sulfonyl chloride, or

h) a compound obtained in accordance with process a), o) or f), and having a free alcohol group, is converted into the corresponding urethane by reaction with isocyanate, or converted into the corresponding ester by reaction with a carboxylic acid derivative which is activated at the carboxy group and which, where appropriate, carries further functional groups, or with a corresponding N-protected amino acid derivative, or converted into the corresponding 3-oxobutyrate with diketene, or converted into the corresponding ether by reaction with a halogen compound such as $\alpha$-halogenocarboxylic acid, or

i) a compound obtained in accordance with process a), o) or f), and having a primary or secondary alcohol group, is oxidized to the corresponding aldehyde or ketone, or

k) a carbonyl compound prepared in accordance with process i) is converted into the corresponding crotonic acid derivative by reaction with a base and dialkylphosphonoacetic acid ester, or converted into the corresponding trans-2-(dialkoxyphosphono)vinyl derivative by reaction with tetraalkylmethylene diphosphonate, or

l) an epoxide group introduced during the cycloaddition is converted into the corresponding 1,2-diol, or

m) an amide prepared in accordance with process g), and containing a 3-H-isoxazole ring, is converted into the corresponding substituted 2-cyano-3-hydroxycrotonamide by treatment with a base in the sense of a ring opening, or

n) a compound of the formula I prepared in accordance with processes a) - m), which compound, owing to its chemical structure, occurs in enantiomeric forms, is resolved into the pure enantiomers by salt formation with enantiomerically pure acids or bases, chromatography on chiral stationary phases, or derivatization using chiral enantiomerically pure compounds such as amino acids, separation of the diastereomers thus obtained, and elimination of the chiral auxiliary group, or

o) a mixture of 2-isoxazoline diastereomers of the formula I, obtained by cycloaddition to chiral or racemic olefins, is resolved into the pure diastereomers by column chromatography on silica gel, or

p) the compound of the formula I prepared in accordance with processes a) - o) is either isolated in free form or, where acid or basic groups are present, is, where appropriate, converted into physiologically tolerated crystalline salts, or

q) a compound which has been prepared in accordance with process h), and which carries a further functional group such as a halogenomethyl group, is alkylated with a primary or secondary amino group or etherified with an alcohol.

5. A pharmaceutical, containing an effective quantity of at least one compound of the formula I as claimed in claims 1, 2 or 3, or as obtained as claimed in claim 4, and/or a physiologically tolerated salt of the compound of the formula I and/or a stereoisomeric form of the compound of the formula I, in addition to physiologically acceptable auxiliary substances and carrier substances, and, where appropriate, further additives and/or other active compounds.

6. The use of at least one compound of the formula I as claimed in claims 1, 2 or 3 for preparing pharmaceuticals for the prophylaxis and therapy of asthmatic diseases, inflammations and/or autoimmune diseases.

7. A process for preparing a pharmaceutical as claimed in claim 5, wherein at least one compound of the formula I as claimed in claims 1, 2 or 3 and/or a physiologically tolerated salt of the compound of the formula I and/or a stereoisomeric form of the compound of the formula I and/or a compound of the formula I obtained by the process as claimed in claim 4 is/are brought into a suitable form for administration together with physiologically acceptable auxiliary substances and carrier substances and, where appropriate, further additives and/or other active compounds.

## Revendications

1. Composés de formule I

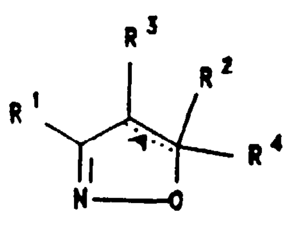

(I)

et/ou sel physiologiquement acceptable d'un composé de formule I et/ou forme stéréoisomère d'un composé de formule I,
formule dans laquelle un radical $R^1$ ou $R^2$ correspond à la formule II

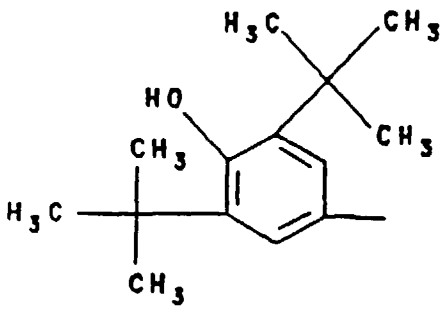

(II)

.

et l'autre radical $R^1$ ou $R^2$ a la signification suivante:

a) le groupe pyridyle,
b) le groupe thiophényle,
c) le groupe thiazolyle,
d) un radical de formule III

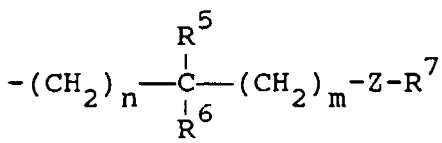

(III)

où

n représente le nombre zéro, 1, 2, 3, 4, 5 ou 6,
m représente le nombre zéro, 1, 2, 3 ou 4,
Z est

1) -O- ou
2) -NH-,

$R^5$ et $R^6$ représentent, indépendamment l'un de l'autre,

1) un atome d'hydrogène,
2) un groupe alkyle en $C_1$-$C_4$,
3) un groupe alcényle en $C_2$-$C_4$,
4) un groupe alcynyle en $C_2$-$C_4$,
5) le groupe phényle ou
6) OH, et

$R^7$ est

1) un atome d'hydrogène,
2) le reste d'un aminoacide choisi parmi l'asparagine, la valine, l'arginine, l'acide aspartique, la glutamine, l'acide glutamique, le tryptophane, la β-alanine, la lysine, la proline, la glycine, le γ-aminobutyrate, la Nε-acétyl-lysine, la Nδ-acétylornithine, le Nγ-acétyldiaminobutyrate, le Nα-acétyldiaminobutyrate, l'histidine, l'isoleucine, la leucine, la méthionine, la phénylalanine, la sérine, la cystéine, la thréonine, l'alanine et la tyrosine,
3) le groupe trifluorométhylsulfonyle,
4) $-O-P(O)(OH)_2$,
5) $-O-P(O)(OH)_2$, une ou deux fois estérifié par un groupe phényle ou alkyle en $C_1$-$C_6$, ou
6) un radical de formule IV

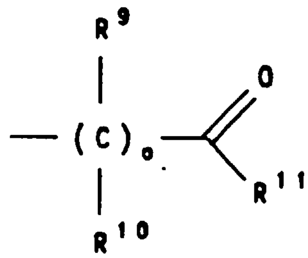 . (IV)

o étant le nombre zéro ou 1,

$R^9$ et $R^{10}$ ayant, indépendamment l'un de l'autre, la signification suivante:

1) un atome d'hydrogène ou
2) un groupe alkyle en $C_1$-$C_4$ et

$R^{11}$ étant

1) OH,
2) un groupe -O-alkyle($C_1$-$C_4$),
3) un groupe alkyle en $C_1$-$C_{20}$,
4) un groupe alkyle en $C_1$-$C_{20}$ une ou plusieurs fois substitué par, indépendamment les uns des autres,

    4.1 -COOH,
    4.2 -OH,
    4.3 le groupe O-acétyle ou
    4.4 =O,

5) -COOH,
6)

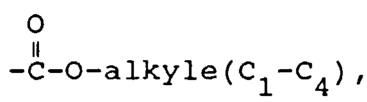

7) le groupe N-glycyle,
8) un groupe N-glycyle ester alkylique en $C_1$-$C_4$,
9) un groupe N-alkyl($C_1$-$C_4$)-hydroxylamino,
10) le groupe N-(1H-tétrazole-5-yl)-amino,
11) le groupe 5-méthylisoxazole-4-yle,
12) le groupe 1-cyano-2-hydroxy-1-propényle,
13) le groupe phényle,
14) un groupe phényle une ou plusieurs fois substitué par

    14.1 un ou des groupes alkyl

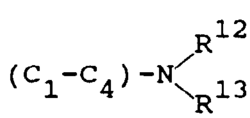

$R^{12}$ et $R^{13}$ ayant, indépendamment l'un de l'autre, la signification suivante:

1) un atome d'hydrogène,
2) un groupe alkyle en $C_1$-$C_4$,
3) un groupe phényl-alkyle($C_1$-$C_2$),
4) $R^{12}$ et $R^{13}$ forment ensemble, avec l'atome d'azote qui les relie, un hétérocycle à 5-7 chaînons, dans lequel un atome de carbone peut être remplacé par un atome de soufre, d'oxygène ou d'azote, ou
5) le reste d'un aminoacide choisi parmi l'asparagine, la valine, l'arginine, l'acide aspartique, la glutamine, l'acide glutamique, le tryptophane, la β-alanine, la lysine, la proline, la glycine, le γ-aminobutyrate, la Nε-acétyl-lysine, la Nδ-acétylornithine, le Nγ-acétyldiaminobutyrate, le Nα-acétyldiaminobutyrate, l'histidine, l'isoleucine, la leucine, la méthionine, la phénylalanine, la sérine, la cystéine, la thréonine, l'alanine et la tyrosine, ou

$R^6$ et $R^7$ sont liés l'un à l'autre et représentent ensemble 1 à 3 radicaux $CH_2$,

e) un radical de formule V

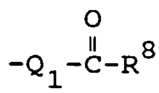

(V)

dans laquelle

$Q_1$ est

1) un groupe $-(CH_2)_m$, m étant le nombre zéro, 1, 2, 3 ou 4, ou
2) -CH=CH-, et

$R^8$ est

1) un atome d'hydrogène,
2) un groupe alkyle en $C_1$-$C_4$,
3) OH,
4) un groupe O-alkyle($C_1$-$C_4$),
5) $NH_2$,
6) le groupe N-(1H-tétrazole-5-yl)-amino ou
7) le groupe N-(3,5-diméthyl-4-hydroxybenzyl)-amino, ou

f) un radical de formule VI

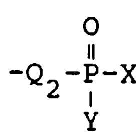

(VI)

dans laquelle

$Q_2$ est

1) un groupe $-(CH_2)_m$, m étant le nombre zéro, 1, 2, 3 ou 4, ou
2) -CH=CH-, et

X et Y représentent, indépendamment l'un de l'autre,

1) un groupe alkyle en $C_1$-$C_4$,
2) un groupe -O-alkyle($C_1$-$C_4$) ou

3) OH,

...A... est une double liaison qui est présente ou absente, avec la restriction que la double liaison et le radical $R^4$ ne sont pas présents simultanément,
$R^4$ est

1) un atome d'hydrogène ou
2) un groupe alkyle en $C_1$-$C_6$, ou bien

$R^2$ et $R^4$ forment ensemble, avec l'atome de carbone auquel ils sont fixés, un cycle aliphatique qui est constitué de 3, 4 ou 5 atomes de carbone, et
$R^3$ est

1) un atome d'hydrogène,
2) un groupe alkyle en $C_1$-$C_4$ ou
3) un groupe hydroxyalkyle en $C_1$-$C_4$.

**2.** Composé de formule I selon la revendication 1, dans lequel un radical $R^1$ ou $R^2$ correspond à la formule II et l'autre radical $R^1$ ou $R^2$ a la signification suivante:

a) le groupe 2-pyridyle,
b) le groupe 4-pyridyle,
c) le groupe thiophéne-3-yle,
d) le groupe 2-thiazolyle,
e) un radical de formule III dans lequel

n représente le nombre zéro, 1, 2, 3 ou 4,
m représente le nombre zéro ou 1,
Z est

1) -O- ou
2) -NH-,

$R^5$ et $R^6$ représentent, indépendamment l'un de l'autre,

1) un atome d'hydrogène,
2) un groupe alkyle en $C_1$-$C_2$,
3) le groupe phényle ou
6) OH, ou

$R^6$ et $R^7$ sont liés l'un à l'autre et forment ensemble un groupe méthylène, ou bien
$R^7$ est

1) un atome d'hydrogène,
2) le groupe trifluorométhylsulfonyle,
3) un aminoacide choisi parmi Gly, Phe, Ala, Lys, Tyr et Ser, ou
4) un radical de formule IV dans lequel

o est le nombre zéro ou 1,
$R^9$ et $R^{10}$ représentent, indépendamment l'un de l'autre,

1) un atome d'hydrogène ou
2) le groupe méthyle,

$R^{11}$ est

1) OH,
2) un groupe -O-alkyle($C_1$-$C_4$),

3) un groupe alkyle en $C_1$-$C_{18}$,
4) un groupe alkyle en $C_1$-$C_6$ une ou plusieurs fois substitué par, indépendamment les uns des autres,

4.1 -COOH,
4.2 -OH ou
4.3 le groupe O-acétyle,

5) -COOH,
6)

$$-\overset{\overset{\displaystyle O}{\|}}{C}-O-alkyle(C_1-C_4),$$

7) le groupe N-glycyle,
8) un groupe N-glycyle ester alkylique en $C_1$-$C_4$,
9) le groupe N-méthyl-hydroxylamino,
10) le groupe N-(1H-tétrazole-5-yl)-amino,
11) le groupe 5-méthylisoxazole-4-yle,
12) le groupe 1-cyano-2-hydroxy-1-propényle ou
13) un groupe phényle substitué une fois par

13.1 le groupe 4-(4-morpholino)méthyle,

f) un radical de formule V dans lequel

$Q_1$ est

1) un groupe $-(CH_2)_{\bar{m}}$, m étant le nombre zéro, 1 ou 2, ou
2) -CH=CH-, et

$R^8$ est

1) un atome d'hydrogène,
2) le groupe méthyle,
3) OH,
4) un groupe -O-alkyle($C_1$-$C_4$),
5) le groupe amino,
6) le groupe N-(1H-tétrazole-5-yl)-amino ou
7) le groupe N-3,5-diméthyl-4-hydroxybenzyle, ou

g) un radical de formule VI dans lequel

$Q_2$ est

1) un groupe $-(CH_2)_{\bar{m}}$, m étant le nombre zéro, 1 ou 2, ou
2) -CH=CH-,

X et Y représentent, indépendamment l'un de l'autre,

1) le groupe méthyle,
2) un groupe -O-alkyle($C_1$-$C_2$) ou
3) OH,

...A... est une double liaison qui est présente ou absente, avec la restriction que la double liaison et le radical $R^4$ ne sont pas présents simultanément,

$R^4$ est

1) un atome d'hydrogène ou
2) un groupe alkyle en $C_1$-$C_2$, ou bien

$R^2$ et $R^4$ forment ensemble, avec l'atome de carbone auquel ils sont fixés, un cycle aliphatique ayant 3, 4 ou 5 atomes de carbone, et
$R^3$ est

1) un atome d'hydrogène,
2) le groupe méthyle ou
3) le groupe hydroxyméthyle.

**3.** Composé de formule I selon la revendication 1 ou 2, dans lequel un radical $R^1$ ou $R^2$ correspond à la formule II et l'autre radical $R^1$ ou $R^2$ a la signification suivante:

a) le groupe 2-pyridyle,
b) le groupe 4-pyridyle,
c) le groupe thiophéne-3-yle,
d) le groupe 2-thiazolyle,
e) un radical de formule III dans lequel

n représente le nombre zéro, 1, 2, 3 ou 4,
m représente le nombre zéro ou 1,
Z est

1) -O- ou
2) -NH-,

$R^5$ et $R^6$ représentent, indépendamment l'un de l'autre,

1) un atome d'hydrogène,
2) un groupe alkyle en $C_1$-$C_2$,
3) le groupe phényle, ou

$R^6$ et $R^7$ sont liés l'un à l'autre et forment ensemble un groupe méthylène, ou bien
$R^7$ est

1) un atome d'hydrogène,
2) le groupe trifluorométhylsulfonyle,
3) un aminoacide choisi parmi Gly, Lys et Phe, ou
4) un radical de formule IV dans lequel

o est le nombre zéro ou 1,
$R^9$ et $R^{10}$ représentent, indépendamment l'un de l'autre,

1) un atome d'hydrogène ou
2) le groupe méthyle,

$R^{11}$ est

1) OH,
2) le groupe O-méthyle ou O-éthyle,
3) un groupe alkyle en $C_1$-$C_{18}$,
4) un groupe alkyle en $C_1$-$C_6$ une ou plusieurs fois substitué par, indépendamment les uns des autres,

4.1 -COOH,

4.2 -OH ou
4.3 le groupe O-acétyle,

5) -COOH,
6)

$$-\overset{O}{\overset{\|}{C}}-O-alkyle(C_1-C_2),$$

7) le groupe N-glycyle,
8) un groupe N-glycyle ester alkylique en $C_1$-$C_2$,
9) le groupe N-méthyl-hydroxylamino,
10) le groupe N-(1H-tétrazole-5-yl)-amino,
11) le groupe 5-méthylisoxazole-4-yle,
12) le groupe 1-cyano-2-hydroxy-1-propényle,

f) un radical de formule V dans lequel

$Q_1$ est

1) un groupe $-(CH_2)_m$, m étant le nombre zéro, 1 ou 2, ou
2) -CH=CH-, et

$R^8$ est

1) un atome d'hydrogène,
2) le groupe méthyle,
3) OH,
4) le groupe -O-méthyle,
5) le groupe amino,
6) le groupe N-(1H-tétrazole-5-yl)-amino ou
7) le groupe N-3,5-diméthyl-4-hydroxybenzyle, ou

g) un radical de formule VI dans lequel

$Q_2$ est

1) un groupe $-(CH_2)_{\bar{m}}$, m étant le nombre zéro, ou
2) -CH=CH-,

X et Y représentent, indépendamment l'un de l'autre,

1) le groupe méthyle,
2) un groupe -O-alkyle($C_1$-$C_2$) ou
3) OH,

...A... est une double liaison qui est présente ou absente, avec la restriction que la double liaison et le radical $R^4$ ne sont pas présents simultanément,
$R^4$ est

1) un atome d'hydrogène ou
2) le groupe méthyle, ou bien

$R^2$ et $R^4$ forment ensemble, avec l'atome de carbone auquel ils sont fixés, un cycle aliphatique à 3, 4 ou 5 atomes de carbone, et
$R^3$ est

1) un atome d'hydrogène ou
2) le groupe hydroxyméthyle.

**4.** Procédé pour la préparation d'une 2-isoxazoline et d'un isoxazole de formule I selon la revendication 1, et/ou d'une forme stéréoisomère du composé de formule I et/ou d'un sel physiologiquement acceptable du composé de formule I, caractérisé en ce que

a) on convertit en le nitriloxyde correspondant un composé nitro primaire, à l'aide d'isocyanates et de quantités catalytiques de triéthylamine, ou un chlorure d'acide hydroxamique, obtenu par chloration d'une aldoxime correspondante, avec une base organique ou minérale, et on fait réagir ce nitriloxyde, obtenu en tant que composé intermédiaire, sans purification, avec une oléfine convenablement substituée ou un alcyne convenablement substitué, dans le sens d'une cycloaddition 1,3-dipolaire, pour aboutir à une 2-isoxazoline ou un isoxazole de formule I, et le produit résultant est éventuellement purifié par chromatographie ou cristallisation, ou
b) on saponifie en l'acide carboxylique un ester d'acide carboxylique de formule I, préparé selon a) ou b) ou un groupe ester introduit additionnellement par le procédé h) ou k),
c) on convertit en l'amide correspondant un ester alkylique d'acide carboxylique de formule I, préparé selon le procédé a) ou o), avec une amine primaire ou secondaire convenablement substituée,
d) on saponifie en l'acide phosphinique ou en l'acide phosphonique un ester monoalkylique d'acide phosphinique ou un ester dialkylique d'acide phosphonique de formule I, préparé selon a) ou o), ou
e) on convertit d'abord un acide carboxylique obtenu selon b) en un dérivé d'acide, puis on estérifie ce dernier avec des alcools ou on le convertit en l'amide correspondant avec des amines primaires ou secondaires ou bien en l'hydroxylamide correspondant avec une hydroxylamine N-alkylée, ou
f) dans un composé obtenu en tant que composé intermédiaire selon le procédé a), b), h), e), o) ou g), on élimine un groupe protecteur de N ou O introduit ou entraîné, ou on saponifie de façon correspondante un ester d'acide carboxylique, phosphinique, phosphonique ou phosphorique introduit ou entraîné, et on obtient un composé de formule I, ou
g) on convertit un composé obtenu selon le procédé f) ou o), comportant un groupe amino libre, par une réaction avec un isocyanate, en le dérivé d'urée correspondant, ou, par une réaction avec un dérivé d'acide carboxylique activé ou avec un aminoacide protégé sur l'atome d'azote, en l'amide correspondant, ou, par une réaction avec un chlorure de sulfonyle, en le sulfonamide correspondant, ou
h) on convertit un composé obtenu selon le procédé a), o) ou f), comportant un groupe alcool libre, par une réaction avec un isocyanate, en l'uréthanne correspondant, ou, par une réaction avec un dérivé d'acide carboxylique, activé au niveau du groupe carboxyle, portant éventuellement d'autres groupes fonctionnels, ou avec un dérivé d'aminoacide correspondant, protégé sur l'atome d'azote, en l'ester correspondant, ou, avec du dicétène, en le 3-oxobutyrate correspondant, ou, par une réaction avec un composé halogéné tel qu'un acide $\alpha$-halogénocarboxylique, en l'éther correspondant, ou
i) on oxyde un composé obtenu selon le procédé a), o) ou f), comportant un groupe alcool primaire ou secondaire, en l'aldéhyde correspondant ou la cétone correspondante, ou
k) on convertit un composé carbonyle préparé selon le procédé i), par une réaction avec une base et un phosphonoacétate de dialkyle, en le dérivé d'acide crotonique correspondant, ou, par une réaction avec un méthylènediphosphonate de tétraalkyle, en le dérivé *trans*-2-(dialcoxyphosphono)vinylique correspondant, ou
l) on convertit en le 1,2-diol correspondant un groupe époxy introduit pendant la cycloaddition, ou
m) on convertit un amide préparé selon le procédé g), qui contient un cycle 3-H-isoxazole, par traitement par une base dans le sens d'une ouverture de cycle, en le 2-cyano-3-hydroxycrotonamide substitué correspondant, ou
n) on sépare en les énantiomères purs un composé de formule I, préparé selon les procédés a) - m), qui, en raison de sa structure chimique, apparaît sous des formes énantiomères, par salification avec des acides ou des bases sous forme d'anantiomères purs, chromatographie sur des phases stationnaires chirales ou transformation en dérivé au moyen de composés chiraux sous forme d'énantiomères purs, tels que des aminoacides, séaparation des diastéréoisomères ainsi obtenus et élimination du groupe auxiliaire chiral, ou
o) on sépare en les diastéréoisomères purs un mélange de diastéréoisomères de 2-isoxazoline de formule I, obtenu par cycloaddition sur des oléfines chirales ou racémiques, par chromatographie sur colonne de gel de silice, ou
p) le composé de formule I préparé selon les procédés a) - o) est soit isolé sous forme libre, soit, dans le cas de la présence de groupes acides ou basiques, éventuellement converti en sels cristallins physiologiquement acceptables, ou
q) un composé préparé selon le procédé h), qui porte un autre groupe fonctionnel tel qu'un groupe halogénométhyle, est alkylé avec un groupe amino primaire ou secondaire ou éthérifié avec un alcool.

5. Médicament, contenant une quantité efficace d'au moins un composé de formule I selon la revendication 1, 2 ou 3 ou tel qu'obtenu selon la revendication 4, et/ou un sel physiologiquement acceptable du composé de formule I et/ou une forme stéréoisomère du composé de formule I, en plus de véhicules et adjuvants physiologiquement acceptables, éventuellement d'autres additifs et/ou d'autres substances actives.

6. Utilisation d'au moins un composé de formule I selon la revendication 1, 2 ou 3, pour la fabrication de médicaments destinés à la prophylaxie et au traitement de maladies asthmatiques, d'inflammations et/ou de maladies auto-immunes.

7. Procédé pour la fabrication d'un médicament selon la revendication 5, caractérisé en ce que l'on met sous une forme d'administration appropriée au moins un composé de formule I selon la revendication 1, 2 ou 3 et/ou un sel physiologiquement acceptable du composé de formule I et/ou une forme stéréoisomère du composé de formule I et/ou un composé de formule I obtenu conformément au procédé selon la revendication 4, avec des véhicules et adjuvants physiologiquement acceptables et éventuellement d'autres additifs et/ou d'autres substances actives.